# EUROPEAN PATENT APPLICATION

(11) **EP 4 491 720 A1**
(43) Date of publication of application: **15.01.2025**
(21) Application number: 23767216.7
(22) Date of filing: 10.03.2023
(51) Int. Cl.: C12N 9/12, C12N 15/113, C12N 15/10, C12N 15/86, C12N 9/22, A61K 48/00, A61P 21/04

(54) **COMPOSITIONS AND METHODS FOR INCREASING DELETION EFFICIENCY OF NUCLEIC ACID SEGMENT BY MODULATION OF NHEJ REPAIR PATHWAY**

(30) Priority: 10.03.2022 KR 20220030000; 27.05.2022 KR 20220065600
(71) Applicant: GenKOre Inc., Daejeon 34141 (KR)
(72) Inventor: KIM, Yong Sam, Seoul 06767 (KR); KIM, Do Yon, Daejeon 34069 (KR); CHIN, Hyun Jung, Seoul 06511 (KR)
(74) Representative: Ullrich & Naumann PartG mbB
(86) International application number: PCT/KR2023/003336
(87) International publication number: WO 2023/172115

(57) **Abstract**

The present invention relates to a composition and method for increasing deletion efficiency of nucleic acid segments in a target gene by modulation of non-homologous end joining (NHEJ) repair pathway. Specifically, the present invention relates to a CRISPR/Cas system (for example, CRISPR/Cas12f1 system) for increasing deletion efficiency of nucleic acid segments in a target gene by modulation of non-homologous end joining repair pathway.

## Description

### Technical Field

This application claims priorities based on Korean Patent Application No. 2022-0030000, filed on March 10, 2022, and Korean Patent Application No. 2022-0065600, filed on May 27, 2022, the entire disclosures of which are incorporated herein by reference.

The present disclosure relates to a composition and a method for increasing deletion efficiency for a nucleic acid segment in a target gene through regulation of the non-homologous end joining (NHEJ) repair pathway. Specifically, the present disclosure relates to a CRISPR/Cas system (for example, a CRISPR/Cas12f1 system) for increasing deletion efficiency for a nucleic acid segment in a target gene by regulation of the non-homologous end joining repair pathway.

### Background Art

Gene scissors technology, represented by a CRISPR/Cas system, is a new type of gene editing technique that targets a desired gene sequence in mammalian cells and the like. Since discovery of the CRISPR/Cas system, extensive research has been conducted to improve gene-editing efficiency, such as including double-strand breaks, and to develop a CRISPR/Cas system in a form (for example, small), which can be easily delivered into cells, such as AAV.

Double-strand DNA breaks within cells can be repaired through a DNA repair mechanism called non-homologous end joining (NHEJ) or homology-directed repair (HDR). For non-homologous end joining (NHEJ), random insertion or deletion of bases occurs at double-strand break sites, which results in frameshift mutation or premature mutation in the gene where DNA double-strand breaks have occurred, thereby knocking out the gene. On the other hand, homology-directed repair (HDR) requires a donor DNA (homologous template) to repair double-strand breaks, and a new sequence with the sequence of this donor DNA as a template is introduced into a break site. Gene editing (for example, knock-out of a specific gene or introduction of a new gene caused by indel) is mainly accomplished by utilizing the NHEJ or HDR pathway in cells.

Meanwhile, it is known that some genetic diseases can be treated by deletion or removal of a specific gene segment (for example, specific exon) which has undergone a genetic mutation or becomes problematic due to the genetic mutation. For example, Duchenne muscular dystrophy, which is caused by a mutation in the dystrophin gene, can be treated by deleting the entire exon 51 (exon skipping), which has undergone a frameshift mutation or premature-termination mutation, thereby allowing for production of a protein having a near-normal function. However, despite the existence of such a definitive therapeutic strategy, effective deletion of a specific gene segment with a relatively long sequence remains challenging.

Despite recent advances in gene editing techniques, there is still a need for a gene editing technique that can enhance removal (large deletion) of a gene segment or deletion efficiency therefor.

### Disclosure of Invention

### Technical Problem

The object of the present disclosure is to solve the above-mentioned problems of the prior art.

In addition, an object of the present disclosure is to provide a composition and a method for increasing deletion efficiency for a nucleic acid segment.

In addition, another object of the present disclosure is to provide a gene editing system with increased deletion efficiency of a nucleic acid segment.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above purpose are as follows.

According to an aspect of the present disclosure, there is provided a composition for increasing deletion of a nucleic acid segment, comprising a molecule that inhibits expression of a gene involved in non-homologous end joining (NHEJ) pathway, or a nucleic acid construct encoding the molecule, or a use of a composition for increasing deletion of a nucleic acid segment, the composition comprising a molecule that inhibits expression of a gene involved in non-homologous end joining (NHEJ) pathway, or a nucleic acid construct encoding themolecule.

In an embodiment, the gene involved in non-homologous end joining pathway may comprise at least one selected from the group consisting of ATM1, XRCC4, XLF, XRCC6, LIG4, and DCLRE1C.

In an embodiment, the gene involved in non-homologous end joining pathway may comprise at least one selected from the group consisting of XRCC6 and DCLRE1C.

In an embodiment, the inhibitory molecule may be shRNA, dsRNA, siRNA, miRNA, or an antisense oligonucleotide.

In an embodiment, the shRNA molecule may comprise at least one selected from the group consisting of shXRCC6 and shDCLRE1C.

In an embodiment, the shRNA molecule may comprise a nucleotide sequence selected from the group consisting of SEQ ID NOS: 360 to 389, SEQ ID NOS: 400 to 430, SEQ ID NOS: 433 to 445, and SEQ ID NO: 473.

In an embodiment, the shRNA molecule may comprise a nucleotide sequence selected from the group consisting of SEQ ID NOS: 375 to 379, SEQ ID NOS: 385 to 389, SEQ ID NOS: 410 to 414, SEQ ID NOS: 420 to 424, and SEQ ID NOS: 433 to 445.

In an embodiment, the composition may comprise at least two of the molecules that inhibit expression of a gene involved in non-homologous end joining (NHEJ) pathway or nucleic acid constructs encoding the molecules.

In an embodiment, the at least two inhibitory molecules may each inhibit expression of the same or different genes.

In an embodiment, the composition may further comprise (i) a Cas endonuclease and (ii) a first guide RNA and a second guide RNA that hybridize to a first target sequence and a second target sequence, respectively; the nucleic acid segment may exist between the first target sequence and the second target sequence; and the Cas endonuclease may form a complex with the first guide RNA to induce first cleavage, and the Cas endonuclease may form a complex with the second guide RNA to induce second cleavage.

In an embodiment, the composition may further comprise at least one nucleic acid construct comprising (i) a nucleic acid encoding a Cas endonuclease, (ii) a nucleic acid encoding a first guide RNA that hybridizes to a first target sequence, and (iii) a nucleic acid encoding a second guide RNA that hybridizes to a second target sequence; the nucleic acid segment may exist between the first target sequence and the second target sequence; and the Cas endonuclease may form a first complex with the first guide RNA to induce first cleavage, and the Cas endonuclease may form a second complex with the second guide RNA to induce second cleavage.

In an embodiment, the first cleavage and the second cleavage may be each independently a single-strand DNA break or a double-strand DNA break.

In an embodiment, the composition or nucleic acid construct may comprise an additional guide RNA that hybridizes to an additional target sequence or a nucleic acid encoding the additional guide RNA.

In an embodiment, the Cas endonuclease may be an endonuclease comprising a Cas12f1 protein.

In an embodiment, the Cas12f1 protein may have at least 70% sequence identity to the amino acid sequence of SEQ ID NO: 1 or 5.

In an embodiment, the first or second guide RNA may be an engineered guide RNA.

In an embodiment, the engineered guide RNA may comprise a U-rich tail sequence linked to the 3'-end of its guide sequence, in which the U-rich tail is represented by 5'-(UₘV)ₙUₒ-3', wherein V is each independently A, C, or G, m and o are integers between 1 to 20, and n is an integer between 0 to 5.

In an embodiment, the engineered guide RNA may comprise a nucleotide sequence having at least 50% sequence identity to a scaffold region of a wild-type Cas12f1 guide RNA sequence that sequentially comprises, from the 5'-end, a first stem-loop region, a second stem-loop region, a third stem-loop region, a fourth stem-loop region, and a tracrRNA-crRNA complementarity region, and the engineered guide RNA may comprise at least one modification selected from the group consisting of the following (1) to (5) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region;
(4) replacement of one or more uracil (U) residues with A, G, or C in three or more consecutive U residues when the consecutive U residues are present in the tracrRNA-crRNA complementarity region; and
(5) addition of a U-rich tail to the 3'-end of its crRNA sequence (a sequence of the U-rich tail is represented by 5'-(UₘV)ₙUₒ-3', wherein V is each independently A, C, or G, m and o are integers between 1 to 20, and n is an integer between 0 to 5).

In an embodiment, the wild-type Cas12f1 guide RNA may comprise tracrRNA comprising the nucleotide sequence of SEQ ID NO: 11 and crRNA comprising the nucleotide sequence of SEQ ID NO: 12.

In an embodiment, the engineered guide RNA may consist of a sequence represented by Formula (I) or has at least 80% sequence identity thereto: 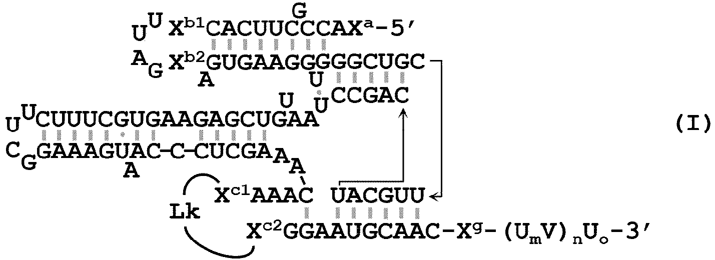
in Formula (I),
X^{a}, X^{b1}, X^{b2}, X^{c1}, and X^{c2} each independently consist of 0 to 35 (poly)nucleotides,
X^{g} is a first or second guide sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

In an embodiment, X^{a} may comprise the nucleotide sequence of SEQ ID NO: 14 or a nucleotide sequence having the sequence of SEQ ID NO: 14 from which 1 to 20 nucleotides are deleted.

In an embodiment, X^{b1} may comprise the nucleotide sequence of SEQ ID NO: 25 or a nucleotide sequence having the sequence of SEQ ID NO: 25 from which 1 to 13 nucleotides are deleted.

In an embodiment, X^{b2} comprises the nucleotide sequence of SEQ ID NO: 29 or a nucleotide sequence having the sequence of SEQ ID NO: 29 from which 1 to 14 nucleotides are deleted.

In an embodiment, the sequence 5'-X^{b1}UUAGX^{b2}-3' in Formula (I) may be a nucleotide sequence selected from the group consisting of SEQ ID NOS: 34 to 38.

In an embodiment, X^{c1} comprises the nucleotide sequence of SEQ ID NO: 39 or a nucleotide sequence having the sequence of SEQ ID NO: 39 from which 1 to 28 nucleotides are deleted.

In an embodiment, in a case where three or more consecutive uracil (U) residues are present in a sequence of X^{c1}, the sequence of X^{c1} comprises a modification in which at least one U residue thereof is replaced with A, G, or C.

In an embodiment, X^{c2} comprises the nucleotide sequence of SEQ ID NO: 58 or a nucleotide sequence having the sequence of SEQ ID NO: 58 from which 1 to 27 nucleotides are deleted.

In an embodiment, in a case where the sequence 5'-ACGAA-3' is present in X^{c2}, the sequence may be replaced with 5'-NGNNN-3', and N may be each independently A, C, G, or U.

In an embodiment, the sequence 5'-X^{c1}-Lk-X^{c2}-3' in Formula (I) may be a nucleotide sequence selected from the group consisting of SEQ ID NOS: 80 to 86.

In an embodiment, Lk may comprise a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

In an embodiment, the engineered guide RNA may comprise an engineered tracrRNA having a nucleotide sequence selected from the group consisting of SEQ ID NOS: 87 to 132.

In an embodiment, the engineered guide RNA may comprise an engineered crRNA sequence having a nucleotide sequence selected from the group consisting of SEQ ID NOS: 133 to 148.

In an embodiment, the engineered guide RNA may be a dual guide RNA or a single guide RNA.

In an embodiment, the engineered single guide RNA may comprise a nucleotide sequence selected from the group consisting of SEQ ID NOS: 149 to 186.

In an embodiment, the nucleic acid construct may be or be contained in an adeno-associated virus vector.

In an embodiment, the composition may not comprise a donor sequence required for homology-directed repair (HDR) pathway or a nucleic acid construct encoding the donor sequence.

According to another aspect of the present disclosure, there is provided a method for increasing deletion of a nucleic acid segment in a target gene of a cell, comprising bringing, into contact with the cell, the above-described composition.

In an embodiment, the nucleic acid segment may comprise a gene fragment that needs to be removed for gene correction.

In an embodiment, the nucleic acid segment may comprise a gene fragment that needs to be removed for treating a genetic disease.

In an embodiment, the nucleic acid segment may comprise a mutated sequence.

In an embodiment, the nucleic acid segment may comprise a mutated sequence that causes a genetic disease.

According to yet another aspect of the present disclosure, there is provided a kit or system for gene editing, comprising: a composition for increasing deletion of a nucleic acid segment, comprising a molecule that inhibits expression of a gene involved in non-homologous end joining (NHEJ) pathway, or a nucleic acid construct encoding the molecule; and a gene editing composition for deletion of a nucleic acid segment, comprising (i) a Cas endonuclease and a first guide RNA and a second guide RNA that hybridize to a first target sequence and a second target sequence, respectively, or (ii) one or more nucleic acid constructs comprising a nucleic acid encoding a Cas endonuclease, a nucleic acid encoding a first guide RNA that hybridizes to a first target sequence, and a nucleic acid encoding a second guide RNA that hybridizes to a second target sequence, wherein the nucleic acid segment exists between the first target sequence and the second target sequence, and the Cas endonuclease forms a complex with the first guide RNA to induce first cleavage, and the Cas endonuclease forms a complex with the second guide RNA to induce second cleavage.

Regarding the respective components of the kit and system for gene editing, it is clear that reference may be made to the detailed description disclosed herein, and reference may also be made to all embodiments or combinations thereof described for the compositions or methods disclosed herein.

### Advantageous Effects of Invention

The present inventors have identified that in a case of inducing deletion or removal of a nucleic acid segment with a CRISPR/Cas12f1 system, efficiency of the deletion or removal actually increases when expression or activity of a factor involved in the NHEJ repair pathway is inhibited, thereby completing the present disclosure. According to an embodiment of the present disclosure, it has been shown that inhibition of NHEJ pathway factors by shRNA can increase efficiency of large-scale deletion induced by UnCas12f1. In particular, interference of DCLRE1C gene, which encodes the Artemis protein, and XCCR6 gene, which encodes the Ku70 protein, has been found to most effectively increase deletion efficiency. In addition, in an embodiment, it has been identified that deletion efficiency for a nucleic acid segment is increased in a case of using a gene-editing system, which is more efficient and has broader applications, together with shRNA that inhibits expression of DCLRE1C or XCCR6, wherein the system comprises a Cas12f1 protein (for example, UnCas12f1, CWCas12f1, or a variant protein thereof), which is a hypercompact nucleic acid cleavage protein, and an engineered guide RNA in which certain regions have been modified to exhibit excellent indel efficiency when used with the protein. Accordingly, the present disclosure can be utilized in gene therapy, gene correction, or the like that requires effective deletion of a relatively long nucleic acid segment, such as exon skipping.

### Brief Description of Drawings

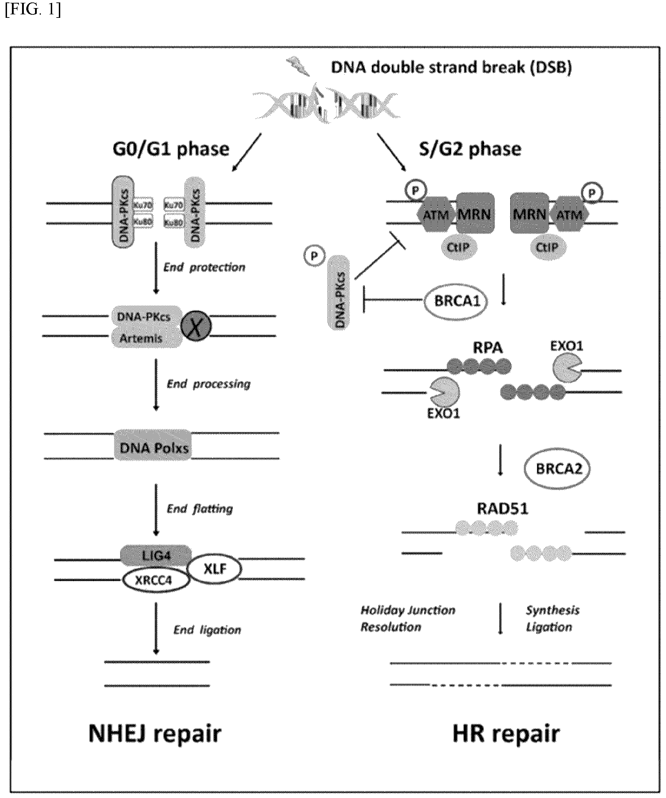

FIG. 1 illustrates a schematic diagram showing canonical DSB repair pathways including NHEJ and HDR.
FIG. 2 illustrates a schematic diagram showing how a patient with Duchenne muscular dystrophy is treated by deletion of a nucleic acid segment comprising exon 51 in the dystrophin gene.
FIG. 3 illustrates modification sites in the engineered guide RNA.
FIG. 4 illustrates a human codon-optimized amino acid sequence of the Cas12f1 protein (FIGS. 4A and 4C) and a human codon-optimized nucleotide sequence encoding the protein (FIGS. 4D to 4H).
FIG. 5 illustrates results obtained by comparing the effects of RNA PolIII-dependent promoters on inhibition of DCLRE1C expression.
FIGS. 6A to 6C illustrate results obtained by performing qPCR analysis on inhibited expression of respective NHEJ component genes caused by shRNA (mean ± standard error, n = 2 independent experiments).
FIGS. 7A to 7C illustrate results obtained by performing Western blot analysis on inhibited expression of respective NHEJ component genes caused by shRNA.
FIG. 8 illustrates that increased deletion efficiency for E51 is achieved by UnCas12f1 in a case of being treated with shRNA.
FIG. 9 illustrates deletion efficiency for E51 depending on treatment with various shRNAs (mean ± standard error, n = 3 independent experiments. * p < 0.5, ** p < 0.01).
FIG. 10A illustrates results obtained by identifying changes in deletion caused by shDCLRE1C using qPCR on day 3 after treatment of HEK293T cells according to Example 6.
FIG. 10B illustrates results obtained by measuring DCLRE1C mRNA levels using qPCR on day 3 after treatment of HEK293T cells according to Example 6.
FIG. 11 illustrates results obtained by performing whole genome sequencing (WGS) analysis to measure deletion efficiency for E51. The section indicated by 800 bp represents the analyzed 800 bp region between the two gRNAs.
FIG. 12 illustrates results obtained by identifying the effect of shDCLRE1C on increased deletion efficiency through complementation of DCLRE1C expression. Results from two independent experiments on HEK293T and AC16 cells are presented on agarose gels.
FIG. 13 illustrates results obtained by identifying deletion efficiency for E51 in DCLRE1C-KO cell line.
FIG. 14 illustrates results obtained by measuring murine Dclreic mRNAlevels depending on treatment with shRNAs for various mouse Dclre1c genes.
FIG. 15 illustrates a graph showing mRNA expression levels of ATM1 and XRCC4 measured in cells transfected with shRNAs according to an embodiment.
FIG. 16 illustrates a graph showing mRNA expression levels of XLF-1 and XRCC6 measured in cells transfected with shRNAs according to an embodiment.
FIG. 17 illustrates a graph showing mRNA expression levels of LIG4 and DCLRE1C measured in cells transfected with shRNAs according to an embodiment.
FIG. 18 illustrates a graph obtained by identifying, with qRT-PCR, deletion efficiency for exon 51 achieved by inhibited expression of NHEJ-related genes in HEK293 cells. The experiments using UnCas12f1 protein are indicated by "Cas12f1," and the experiments using CWCas12f1 protein are indicated by "TaRGET."
FIG. 19 illustrates a graph obtained by identifying, with qRT-PCR, deletion efficiency for exon 51 achieved by inhibited expression of NHEJ-related genes in AC16 cells. The experiments using UnCas12f1 protein are indicated by "Cas12f1," and the experiments using CWCas12f1 protein are indicated by "TaRGET."
FIG. 20 illustrates a graph obtained by identifying, with qRT-PCR, deletion efficiency for exon 51 achieved using a single type of shRNA or a combination of two or more types of shRNA in HEK293 cells.
FIG. 21 illustrates a graph obtained by identifying, with qRT-PCR, deletion efficiency for exon 51 achieved using a single type of shRNA or a combination of two or more types of shRNA in AC 16 cells.
FIG. 22 illustrates a graph obtained by identifying, with qRT-PCR, deletion efficiency for exon 51 depending on the number of days post transfection using systems comprising shRNA according to an embodiment. The results are indicated by SaCas9, Cas12f1 (UnCas12f1), and TaRGET depending on the CRIPSR protein used in the system.
FIG. 23 illustrates results obtained by analyzing deletion efficiency for exon 51 depending on treatment with shRNADCLRE1C and shXRCC6 (mean ± standard error, n = 2 independent experiments).

### Modes for Carrying out Invention

The detailed description of the present disclosure set forth below will be described with reference to specific drawings (only if there are drawings) with respect to specific embodiments in which the present disclosure may be practiced; however, the present disclosure is not limited thereto and is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply and whenever appropriate, terms used in the singular will also include the plural and vice versa.

Hereinafter, in order to enable those skilled in the art to easily practice the present disclosure, various preferred embodiments/examples of the present disclosure will be described in detail with reference to the attached drawings (only if there are drawings).

### I. Definition

As used herein, "nucleic acid," "nucleotide," "nucleoside," and "base" have the meanings commonly understood by those skilled in the art. Specifically, "nucleic acid" is a biological molecule composed of nucleotides, and is used interchangeably with polynucleotide. The nucleic acid comprises both DNA and RNA, which is double-stranded or single-stranded. "Nucleotide" is a unit composed of phosphoric acid, a pentose sugar, and a base (or nucleobase). In RNA (ribonucleic acid), the pentose sugar is ribose; and in DNA (deoxyribonucleic acid), the pentose sugar is deoxyribose. The nucleotide has one selected from adenine (A), guanine (G), cytosine (C), thymine (T), and uracil (U) as a nucleobase. Adenine, guanine, and cytosine exist both in RNA and DNA, thymine exists only in DNA, and uracil exists only in RNA. In addition, the pentose sugar and nucleobase constituting the nucleotide may be referred to as "nucleoside." The nucleoside is classified into adenosine, thymidine, cytidine, guanosine, and uridine depending on the type of nucleobase. The abbreviations for base, nucleoside, and nucleotide may be identical and may be appropriately interpreted depending on the context. For example, the sequence 5'-UUUUU-3' may be a sequence of five consecutive bases (uracil residues), a sequence of five consecutive nucleosides (uridine residues), and/or a sequence of five consecutive nucleotides (uridine monophosphate). In addition, when describing a nucleic acid, RNA, and DNA, nucleotides constituting the same are abbreviated as uridine, adenosine, thymidine, cytidine, and guanosine according to the type of nucleoside. The above abbreviation may be appropriately interpreted depending on the context. For example, RNA comprising a sequence of four consecutive uridine residues may be interpreted as RNA comprising four consecutive uridine monophosphate nucleotides. In addition, the terms nucleic acid, nucleotide, nucleoside, and base as used herein may include modified nucleic acids, nucleotides, nucleosides, and bases known in the art for improving, for example, safety or immunogenicity thereof.

As used herein, "target nucleic acid" or "target gene" refers to a nucleic acid or gene that is a subject of gene editing (for example, double-strand break or deletion of a gene segment) or targeted by a gene editing system (for example, Cas12f1 system or TaRGET system). These terms may be used interchangeably and refer to the same subject. Unless otherwise defined, the target gene may be a unique gene or nucleic acid possessed by a target cell (for example, a prokaryotic cell, a eukaryotic cell, an animal cell, a mammalian cell, or a plant cell), a gene or nucleic acid of external origin, or an artificially synthesized nucleic acid or gene, and may mean single-stranded or double-stranded DNA or RNA. The target gene or target nucleic acid may be a mutated gene involved in a genetic disease. In an embodiment, the target gene or target nucleic acid may be a gene having a mutation. In an embodiment, the target gene or target nucleic acid may be a mutated human gene.

As used herein, "target region" means a region of a target gene to which a guide RNA is designed to bind and in which cleavage occurs. The target region may comprise a target sequence. The target region may comprise a target sequence. In addition, in double-stranded nucleic acids, the target region may refer to a region that comprises a target sequence (included in a target strand) and a sequence complementary thereto (included in a non-target strand).

As used herein, "target sequence" refers to a sequence located in a target nucleic acid or a target gene, which is recognized by a guide RNA, or a sequence to be modified by a gene editing system such as CRISPR/Cas12f1 system. Specifically, the target sequence refers to a sequence complementary to a guide sequence included in a guide RNA or a sequence that binds complementarily to the guide sequence. The strand including the target sequence is referred to as a "target strand." When the target nucleic acid or the target gene is single-stranded, the strand may be a target strand. When the target nucleic acid or the target gene is double-stranded, one of the double strands may be a target strand, and a strand complementary to the target strand may exist. The strand complementary to the target strand is referred to as a "non-target strand." The "non-target strand" comprises a PAM (Protospacer Adjacent Motif) sequence and a protospacer sequence. The PAM sequence is a sequence recognized by Cas endonucleases such as Cas12f1 protein. The protospacer sequence, which is located at the 5'- end or the 3'-end of the PAM sequence, is a sequence having complementarity to a target sequence or a sequence that forms a complementary bond with a target sequence. Correlation between the protospacer sequence and the target sequence is similar to correlation between the target sequence and the guide sequence. Due to these characteristics, in general, a guide sequence may be designed using a protospacer sequence. That is, a guide sequence which binds complementarily to a target sequence may be designed as a nucleotide sequence having the same nucleotide sequence as the protospacer sequence, and the guide sequence is designed by replacing T in the protospacer sequence with U.

As used herein, "stem" refers to a nucleic acid region having a secondary structure that comprises a nucleotide region capable of forming a double strand. A configuration in which a double strand is connected primarily by a region of single-stranded nucleotides (a loop region) is referred to as a "stem-loop." The terms "stem" and "stem-loop" may be used interchangeably and should be interpreted appropriately depending on the context.

The term "non-homologous end joining (NHEJ)" refers to a mechanism that repairs a double-strand break in a nucleotide sequence by direct ligation of the broken ends without the requirement for a homologous template (as opposed to homology-directed repair, which requires a homologous sequence to induce healing of a double-strand break in a nucleotide sequence). NHEJ often leads to loss (deletion) of a nucleotide sequence near the double-strand break site.

The term "vector," unless otherwise specified, refers to any material capable of transporting a genetic material into a cell. For example, a vector may be a nucleic acid, typically a DNA molecule, comprising a genetic material of interest, for example, a nucleic acid encoding an effector protein (Cas protein) of a CRISPR/Cas system, and/or a nucleic acid encoding a guide RNA; however, the vector is not limited thereto.

The term "operably linked" means a functional linkage between two or more elements arranged in such a way that allows the described element to function in an intended manner. For example, when a promoter sequence is operably linked to a sequence encoding protein A, it means that the promoter is linked to the sequence encoding the protein A so as to transcribe and/or express the sequence encoding the protein A in a cell. In addition, the term includes all other meanings generally recognized by those skilled in the art and may be appropriately interpreted depending on the context.

The term "engineered" is used to distinguish a substance or molecule from one having a naturally occurring configuration, and means that the substance or molecule is obtained by application of artificial modification. For example, "engineered guide RNA" refers to a guide RNA obtained by applying an artificial modification to the configuration of a naturally occurring guide RNA.

The term "NLS (nuclear localization sequence or signal)" refers to an amino acid sequence that promotes introduction of a substance from outside the nucleus into the nucleus, for example, by nuclear transport. The term "NES (nuclear export sequence or signal)" refers to an amino acid sequence that promotes transport of a substance from inside the nucleus to the outside of the nucleus, for example, by nuclear transport. The terms NLS or NES are known in the relevant art and may be clearly understood by those skilled in the art.

The term "about" refers to an amount, level, value, number, frequency, percent, dimension, size, amount, weight or length that varies by approximately 30, 25, 20, 15, 10, 9, 8, 7, 6, 5, 4, 3, 2, or 1% with respect to a reference amount, level, value, number, frequency, percent, dimension, size, amount, weight or length. For example, the term "about" may mean x ± 5% when used in relation to a value x expressed as a number or numerical value.

The term "subject" is used interchangeably with "patient" and may be a mammal in need of prevention or treatment of a genetic disease, such as primate (for example, human), companion animal (for example, dog and cat), domestic animal (for example, cow, pig, horse, sheep, and goat), and laboratory animal (for example, rat, mouse, and guinea pig). In an embodiment of the present disclosure, the subject is a human.

The term "treatment" generally means obtaining a desired pharmacological and/or physiological effect. Such an effect has a therapeutic effect in that it partially or completely cures a disease and/or harmful effects caused by the disease. Desirable therapeutic effects include, but are not limited to, prevention of occurrence or recurrence of a disease, improvement of symptoms, reduction of any direct or indirect pathological consequences of a disease, prevention of metastasis, reduction of disease progression rate, improvement or alleviation of disease state, and remission or improved prognosis. Preferably, "treatment" may refer to medical intervention for an already manifested disease or disorder. More preferably, "treatment" may be deletion of a specific segment in a specific gene or restoration of the reading frame of the specific gene resulting therefrom.

As used herein, the term "target nucleic acid editing system," "gene editing system," or "gene restoration system" refers to a system that comprises a nucleic acid degrading enzyme, such as nucleic acid editing protein or endonuclease, and a nucleic acid-targeting molecule corresponding to the nucleic acid degrading enzyme, and this system binds to or interacts with a target nucleic acid or target gene so that a target region of the target nucleic acid or target gene can be cleaved, edited, repaired, and/or restored. Here, the nucleic acid-targeting molecule may be represented by an engineered guide RNA (gRNA), but is not limited thereto. Meanwhile, the target nucleic acid editing system may exist in any form capable of editing the target nucleic acid. For example, the system may be in a form of a composition that comprises a complex comprising a nucleic acid degrading enzyme and a nucleic acid-targeting molecule, may be in a form of a kit in which the nucleic acid degrading enzyme and the nucleic acid-targeting molecule are each included in separate compositions, or may be a vector system or composition comprising at least one vector that comprises a nucleic acid encoding the nucleic acid degrading enzyme and a nucleic acid encoding the nucleic acid-targeting molecule.

The term "hypercompact TaRGET system" refers to a gene editing system that comprises a nucleic acid degrading enzyme such as hypercompact CRISPR/Cas protein or tiny endonuclease (for example, Cas12f1 or a variant thereof) and a nucleic acid-targeting molecule corresponding to the nucleic acid degrading enzyme, and is used for differentiation from the existing gene editing system. Here, the nucleic acid-targeting molecule may be represented by an engineered guide RNA (gRNA), but is not limited thereto. The system may be any type of gene editing system capable of binding to a target nucleic acid or target gene so that a target region of the target nucleic acid or gene is cleaved, edited, repaired, and/or restored.

The term "nuclease" or "endonuclease" refers to an enzyme that possesses catalytic activity for DNA cleavage and may be used interchangeably.

The term "nuclease" or "endonuclease" refers to an enzyme that possesses catalytic activity for nucleic acid cleavage and may be used interchangeably. These terms may also be used interchangeably with "nucleic acid editing protein," "gene editing protein," or "nucleic acid degrading protein." The molecule referred to as "nucleic acid editing protein," "gene editing protein," or "nucleic acid degrading protein" refers to a (endo-) nuclease that recognizes the targeting nucleic acid, DNA or RNA, or a protospacer adjacent motif (PAM) present in a target gene, and then allows double-strand breaks (DSBs) to occur at nucleotide sequences within or outside the target nucleotide sequence. In addition, the endonuclease, the nucleic acid editing protein, or the like is also referred to as an effector protein that constitutes a nucleic acid construct for a nucleic acid editing system. Here, the effector protein may be a nucleic acid degrading protein capable of binding to a guide RNA (gRNA) or engineered gRNA, or may be a peptide fragment capable of binding to a target nucleic acid or target gene.

The term "guide RNA (gRNA)" refers to RNA that is capable of forming a complex with a molecule referred to as an endonuclease, a gene editing protein, a nucleic acid degrading protein, or the like, and interacting with (for example, hybridizing to, forming a complementary bond(s) with, or forming a hydrogen bond(s) with) a target nucleotide sequence, and comprises a guide sequence having sufficient complementarity with the target nucleotide sequence to cause sequence-specific binding of the complex to the target nucleotide sequence. In the present disclosure, a guide RNA and a guide molecule may be used interchangeably.

The terms "tracrRNA (trans-activating crRNA)" and "crRNA (CRISPR RNA)" include the meanings commonly understood by those skilled in the art. These terms may be used to refer to respective molecules of a dual guide RNA found in nature, and may also be used to refer to respective portions of a single guide RNA (sgRNA) in which the tracrRNA and the crRNA are connected by a linker. Unless otherwise stated, the description tracrRNA and crRNA means tracrRNA and crRNA that constitute a guide RNA.

The term "scaffold region" refers collectively to a portion of a guide RNA (gRNA) which can interact with a molecule called endonuclease, gene editing protein, nucleic acid degrading protein, or the like, and may be used to refer to the remaining portion of a guide RNA found in nature, excluding a spacer.

The terms "guide sequence," "spacer," or "spacer sequence" may be used interchangeably, and refer to a polynucleotide within the CRISPR/Cas system which is capable of interacting with (for example, hybridizing to, forming a complementary bond(s) with, or forming a hydrogen bond(s) with) a target sequence portion. For example, the guide sequence or spacer sequence refers to 10 to 50 consecutive nucleotides linked directly or indirectly through a linker or the like to or near the 3'-end of crRNA, which constitutes a guide RNA, in a target nucleic acid editing system.

The term "wild type" has the meaning commonly understood by those skilled in the art and means a typical form of an organism, strain, gene, or characteristic as it occurs in nature to the extent that it is distinguishable from mutant or variant forms.

The term "variant" should be understood to mean expression of qualities having a pattern that deviates from what occurs in nature. For example, the variant protein may mean a variant of (wild-type) Cas12f1.

The term "nucleic acid construct" refers to a nucleic acid molecule that comprises one or more distinct segments and/or elements. The term includes linear nucleic acid constructs (for example, λ-phage constructs, PCR products) as well as circular nucleic acid constructs such as plasmid constructs, viral vector constructs, cosmid vectors, and the like. In an embodiment, the nucleic acid construct is a single-stranded or double-stranded nucleic acid molecule, which may be modified to contain a nucleic acid segment. In an embodiment, the nucleic acid construct may be such that specific nucleic acids are operably linked. In another embodiment, the nucleic acid construct of the present disclosure is an expression vector, which comprises sequences that render it suitable for replication and integration in prokaryotes. In yet another embodiment, the expression vector is suitable for expression in eukaryotes. In still yet another embodiment, the expression vector is suitable for expression in both prokaryotes and eukaryotes (for example, a shuttle vector). The expression vector also comprises an expression signal such as a promoter and/or an enhancer. Nucleotide sequences required for expression in prokaryotes often include promoters, operators (non-essential), and ribosomal binding sites along with other sequences. Eukaryotic cells are known to utilize promoters, enhancers, and termination and polyadenylation signals.

The terms "protein," "polypeptide," and "peptide" may be used interchangeably and refer to a polymeric form of amino acids of any length which may comprise genetically coded and non-genetically coded amino acids, chemically or biochemically modified or derivatized amino acids, and polypeptides having modified peptide backbones. The terms include fusion proteins, including, but not limited to, fusion proteins with a heterologous amino acid sequence, fusions with heterologous and homologous leader sequences, with or without N-terminal methionine residues; immunologically tagged proteins; and the like.

The term "hybridizable" means, for example, that a guide sequence has at least 60%, for example at least 70%, at least 80%, at least 90%, at least 95%, at least 98%, at least 99%, or 100% sequence complementarity to a target sequence or has less than 5 mismatches and binds complementarily to the target sequence.

The term "microRNA" or "miRNA" is used interchangeably and has the meaning commonly understood by those skilled in the art. miRNA is a small, non-coding, single-stranded RNA of approximately 22 nucleotides in length (typically between 19 and 25 nucleotides in length). miRNA typically targets more than one gene. Targeting with miRNA allows mismatches and inhibition of mRNA translation is mediated by incomplete complementarity, whereas siRNA and shRNA are specific to their targets due to perfect sequence complementarity.

The term "siRNA" refers to a small interfering or short interfering RNA and has the meaning commonly understood by those skilled in the art. siRNA refers to an RNA duplex of nucleotides that can target a desired gene and inhibit expression of a gene that shares homology therewith. siRNA is formed from a long double strand RNA (dsRNA) or shRNA.

The term "shRNA" refers to a small hairpin RNA or a short hairpin RNA, and has the meaning commonly understood by those skilled in the art. shRNA refers to an RNA duplex in which the siRNA portion is part of a hairpin structure. shRNA may be processed into functional siRNA in cells.

The term "antisense oligonucleotide" refers to a nucleotide sequence that can interact and/or hybridize with a target sequence in pre-mRNA or mRNA molecule having a complementary nucleotide sequence, thereby modifying gene expression, the meaning of which will be clearly understood by those skilled in the art.

All technical terms used in the present disclosure, unless otherwise defined, have meanings commonly understood by those skilled in the relevant technical field and may be interpreted appropriately depending on the context.

### II. Increased deletion efficiency for nucleic acid segment caused by inhibited expression of genes involved in NHEJ pathway

In mammalian cells, the "canonical" or "classical" NHEJ pathway (C-NHEJ) requires several factors, including DNA-PK, Ku70-80, Artemis, ligase IV (Lig4), XRCC4, CLF, and Pol µ, to repair double-strand breaks (Kasparek & Humphrey Seminars in Cell & Dev. Biol. 22:886-897, 2011). In a case of artificially inducing a double-strand break in a target gene using a gene-editing system such as Cas12f1, random base insertion and deletion (Indel) occur at the double-strand break site through the NHEJ pathway in a cell. The present disclosure is based, in part, on the finding that, when it is desired to induce large-scale deletion of a relatively long nucleic acid segment, that is, a nucleotide sequence, in a target gene, increased deletion efficiency for the nucleic acid segment is achieved by inhibiting expression or activity of factors (in particular, XRCC6 and DCLRE1C) involved in the NHEJ repair pathway.

According to an aspect of the present disclosure, there is provided a composition for increasing deletion of a nucleic acid segment in a target gene, comprising a molecule that inhibits expression of a gene involved in non-homologous end joining pathway, or a nucleic acid construct encoding the molecule.

In an embodiment, the gene involved in non-homologous end joining pathway may comprise at least one selected from the group consisting of ATM1, XRCC4, XLF, XRCC6, LIG4, and DCLRE1C. The ATM1 gene encodes ATM1 protein. The XRCC4 gene encodes XRCC4 protein. The XLF gene encodes XLF protein. The XRCC6 gene encodes Ku70 protein. The LIG4 gene encodes DNA ligase IV protein. The DCLRE1C gene encodes ARTEMIS protein. The proteins expressed by these six genes are all known to be involved in NHEJ. Specifically, KU70 is a DNA recognition protein that binds to the ends of double-strand breaks to assist in DNA repair; ARTEMIS participates in end processing during NHEJ repair; and XLF-XRCC4-DNA ligase IV complex provides a scaffold for joining double-strand breaks, ensuring accurate and efficient ligation and repair.

In another embodiment, the gene involved in non-homologous end joining pathway may comprise at least one selected from the group consisting of XRCC6 and DCLRE1C.

In an embodiment, the inhibitory molecule may be a small molecule or an inhibitory nucleic acid.

In an embodiment, the inhibitory molecule may be shRNA, dsRNA, siRNA, miRNA, or an antisense oligonucleotide. Preferably, the inhibitory molecule may be shRNA, siRNA, or an antisense oligonucleotide. More preferably, the inhibitory molecule may be shRNA or siRNA.

In another embodiment, the inhibitory molecule may be targeted to enzymes involved in NHEJ, HDR, or upstream regulation thereof, by post translational modification, for example, through phosphorylation, ubiquitination, and/or sumoylation.

In another embodiment, the composition may comprise an shRNA molecule, a dsRNA molecule, a siRNA molecule, a miRNA molecule, or an antisense oligonucleotide molecule that inhibits expression of an XRCC6 gene; an shRNA molecule, a dsRNA molecule, a siRNA molecule, a miRNA molecule, or an antisense oligonucleotide molecule that inhibits expression of a DCLRE1C gene; or a combination thereof.

In yet another embodiment, the shRNA molecule may comprise at least one selected from the group consisting of shXRCC6 and shDCLRE1C.

In still yet another embodiment, the inhibitory molecule or shRNA molecule may comprise a nucleotide sequence selected from the group consisting of SEQ ID NOS: 360 to 389, SEQ ID NOS: 400 to 430, SEQ ID NOS: 433 to 445, and SEQ ID NO: 473.

In still yet another embodiment, the inhibitory molecule or shRNA molecule may comprise a nucleotide sequence selected from the group consisting of SEQ ID NOS: 375 to 379, SEQ ID NOS: 385 to 389, SEQ ID NOS: 410 to 414, SEQ ID NOS: 420 to 424, and SEQ ID NOS: 433 to 445.

In an embodiment, the composition may comprise at least one nucleic acid construct encoding a molecule that inhibits expression of a gene involved in non-homologous end joining pathway.

In an embodiment, the composition may comprise at least two of the above-described nucleic acid constructs encoding molecules that inhibit expression of a gene involved in non-homologous end joining pathway. Specifically, the at least two of the nucleic acid constructs may each encode a molecule that inhibits expression of the same gene, or may encode molecules that inhibit expression of different genes. For example, the composition may comprise at least two of the nucleic acid constructs encoding molecules (for example, shXRCC6) that inhibit XRCC6. For example, the composition may comprise at least two of the nucleic acid constructs encoding molecules (for example, shDCLRE1C) that inhibit expression of DCLRE1C. In addition, the composition may comprise a nucleic acid construct encoding a molecule (for example, shXRCC6) that inhibits XRCC6 and a nucleic acid construct encoding a molecule (for example, shDCLRE1C) that inhibits expression of DCLRE1C.

In an embodiment, the nucleic acid construct may be or comprise the above-described nucleotide sequence encoding a molecule that inhibits expression of a gene involved in non-homologous end joining pathway.

In an embodiment, the nucleic acid construct may comprise, in an operably-linked form, the above-described nucleotide sequence encoding a molecule that inhibits expression of a gene involved in non-homologous end joining pathway. For example, the nucleic acid construct may comprise the above-described nucleotide sequence encoding a molecule that inhibits expression of a gene involved in non-homologous end joining pathway, in a form of being operably linked to another element, such as a promoter, that enables the nucleotide sequence to be expressed in a cell.

In an embodiment, the nucleic acid construct may be a vector. In another embodiment, at least two of the nucleic acid constructs according to an embodiment of the present disclosure may be contained in a single vector. Specifically, the at least two of the nucleic acid constructs may each encode the above-described molecule that inhibits expression of the same gene involved in non-homologous end joining pathway, and may also encode the above-described molecules that inhibit expression of different genes. For example, at least two of the nucleic acid constructs encoding shXRCC6 may be contained in a single vector. For example, the vector may comprise at least one nucleic acid construct encoding shXRCC6 and at least one nucleic acid construct encoding shDCLRE1C. Details on vectors or vector systems are given in a separate section below.

In another aspect, the composition may be a gene editing composition for deleting a nucleic acid segment (for example, a CRISPR/Cas system such as a CRISPR/Cas12f1 system). In an embodiment, the composition may be a gene editing composition for deleting a nucleic acid segment in a gene of a cell. In another embodiment, the composition may be a gene editing composition or an adjuvant composition for gene editing to increase deletion efficiency for a nucleic acid segment in a gene of a cell. Specifically, the composition may further comprise (i) a Cas endonuclease and (ii) a first guide RNA and a second guide RNA that hybridize to a first target sequence and a second target sequence, respectively, in a target gene. The nucleic acid segment may exist between the first target sequence and the second target sequence, and the Cas endonuclease may form a complex with the first guide RNA to induce first cleavage, and the Cas endonuclease may form a complex with the second guide RNA to induce second cleavage.

In yet another aspect, the composition may further comprise at least one nucleic acid construct comprising (i) a nucleic acid encoding a Cas endonuclease, (ii) a nucleic acid encoding a first guide RNA that hybridizes to a first target sequence in a target gene, and (iii) a nucleic acid encoding a second guide RNA that hybridizes to a second target sequence in the target gene. The nucleic acid segment exists between the first target sequence and the second target sequence. Once the nucleic acid constructs are expressed in a cell, the Cas endonuclease may form a first complex with the first guide RNA to induce first cleavage, and the Cas endonuclease may form a second complex with the second guide RNA to induce second cleavage.

The endonuclease, together with the guide RNAs, may be referred to as a gene editing system, a CRISPR/Cas system, or a CRISPR/Cas12f1 system.

In an embodiment, the first cleavage and the second cleavage may be each independently a single-strand DNA break or a double-strand DNA break.

In another embodiment, the composition or nucleic acid construct may comprise an additional guide RNA that hybridizes to an additional target sequence in the target gene or a nucleic acid encoding the same. The target sequences may overlap with each other.

In another embodiment, the guide RNA may target a region adjacent to a nucleic acid segment to be deleted in a target gene to generate cleavage (for example, a single-strand break or a double-strand break).

In yet another embodiment, two guide RNAs may target the upstream and downstream regions, respectively, of a nucleic acid segment to be deleted in a target gene, thereby generating at least one cleavage (for example, two single-strand breaks or two double-strand breaks).

In still yet another embodiment, at least two guide RNAs may be used to generate at least two sets of cleavage (for example, two double-strand breaks, one double-strand break, and one single-strand break; or two pairs of single-strand breaks).

Without wishing to be bound by any particular theory, it is thought that the composition of the present disclosure, which comprises a molecule that inhibits expression of a gene involved in non-homologous end joining pathway or a nucleic acid construct encoding the molecule, blocks repair of a double-strand break site by the NHEJ pathway when the double-strand break is formed by a gene editing system, thereby maintaining the double-strand break state for a long time, which increases likelihood that a double break can occur on both sides of a nucleic acid segment to be deleted so that the nucleic acid segment is removed.

Details on the gene editing system, CRISPR/Cas system, and CRISPR/Cas12f1 system included in the composition of the present disclosure will be given in a separate section below.

In an embodiment, the endonuclease comprising the Cas protein and the guide RNA may be included in the form of a ribonucleoprotein particle (RNP).

In an aspect, the composition of the present disclosure may not comprise a donor sequence required for homology-directed repair (HDR) pathway or a nucleic acid construct encoding the donor sequence. Without wishing to be bound by any particular theory, the composition of the present disclosure is intended to remove a specific nucleic acid segment by a double-strand break.

According to another aspect of the present disclosure, there is provided a method for increasing deletion of a nucleic acid segment in a target gene of a cell, comprising bringing, into contact with the cell, the composition according to the above-described embodiment.

In an embodiment, the nucleic acid segment may be a gene fragment that needs to be removed or deleted for various purposes, such as gene therapy or improvement of breed. For example, the nucleic acid segment may be a gene fragment that needs to be removed for gene correction. In addition, the nucleic acid segment may comprise a gene fragment that needs to be removed for treatment of a genetic disease.

In an embodiment, the nucleic acid segment may have a length of 1 bp to 1000 kbp, 100 bp to 1000 kbp, 200 bp to 1000 kbp, 300 bp to 1000 kbp, 400 bp to 1000 kbp, 500 bp to 1000 kbp, 600 bp to 1000 kbp, 700 bp to 1000 kbp, 800 bp to 1000 kbp, 900 bp to 1000 kbp, 1 kbp to 1000 kbp, 100 kbp to 1000 kbp, 200 kbp to 1000 kbp, 300 kbp to 1000 kbp, 400 kbp to 1000 kbp, 500 kbp to 1000 kbp, 600 kbp to 1000 kbp, 700 kbp to 1000 kbp, 800 kbp to 1000 kbp, or 900 kbp to 1000 kbp. In addition, the length of the nucleic acid segment may have a length within any range of the specified numerical ranges, which can be readily determined and understood by those skilled in the art.

In an embodiment, the nucleic acid segment may comprise a (undesirable) mutated sequence. In another embodiment, the nucleic acid segment may comprise a sequence altered by a genetic mutation. The mutation may include, but is not limited to, frameshift mutations, premature termination mutations, point mutations, mutations caused by expanded repeat sequences, and mutations caused by repeat sequences.

In an embodiment, the nucleic acid segment may comprise a mutated sequence that causes a genetic disease. For example, the mutated sequence that causes a genetic disease may be, but is not limited to, a frameshift mutation or a premature termination mutation in dystrophin exon 51 which causes Duchenne dystrophy, or a point mutation where adenine in intron 26 of the CEP290 gene is replaced with guanine which causes Leber congenital amaurosis type 10 (LCA10).

In another embodiment, the cell may be a prokaryotic cell, a eukaryotic cell, an animal cell, a mammalian cell, or a plant cell. Specifically, the cell may be an animal cell, a mammalian cell, or a human cell. In addition, the cell may be a plant cell.

In an embodiment, the bringing into contact with the cell may be delivery or introduction of a composition comprising a nucleic acid construct into the cell. Specifically, the nucleic acid construct (for example, a vector) may be delivered or introduced into a cell, for example, by *in vivo* electroporation, liposomes, nanoparticles, or DNA injection or DNA vaccination, with or without a recombinant vector.

In addition, the composition comprising a nucleic acid construct of the present disclosure may be delivered or introduced by a virus, such as a retrovirus, a lentivirus, an adenovirus, an adeno-associated virus, a vaccinia virus, a poxvirus, a herpes simplex virus or a phage. Specifically, the composition may be contained in a packaging virus and delivered into a cell in the form of a virus produced by the packaging virus.

Specifically, the bringing-into-contact, delivery, or introduction may be made by electroporation, gene gun, sonoporation, magnetofection, nanoparticles, and/or transient cell compression or squeezing method. When the cell is a eukaryotic cell, cationic liposome method, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep 13. pii: S0169-409X(12)00283-9) may be used.

In another embodiment, the bringing-into-contact, delivery, or introduction may be performed *in vitro*, *in vivo*, or *ex vivo.*

According to yet another aspect of the present disclosure, there is provided a kit or system for gene editing, comprising: a composition for increasing deletion of a nucleic acid segment, comprising a molecule that inhibits expression of a gene involved in non-homologous end joining (NHEJ) pathway, or a nucleic acid construct encoding the molecule; and a gene editing composition for deletion of a nucleic acid segment, comprising (i) a Cas endonuclease and a first guide RNA and a second guide RNA that hybridize to a first target sequence and a second target sequence, respectively, or (ii) at least one nucleic acid construct comprising a nucleic acid encoding a Cas endonuclease, a nucleic acid encoding a first guide RNA that hybridizes to a first target sequence, and a nucleic acid encoding a second guide RNA that hybridizes to a second target sequence, wherein the nucleic acid segment exists between the first target sequence and the second target sequence, and the Cas endonuclease forms a complex with the first guide RNA to induce first cleavage, and the Cas endonuclease forms a complex with the second guide RNA to induce second cleavage.

For respective components of the kit and system for gene editing, it is clear that reference may be made to the detailed description disclosed herein, and reference may also be made to all embodiments or combinations thereof described for the compositions or methods disclosed herein.

### III. CRISPR/Cas system

As disclosed herein, the CRISPR/Cas12f1 system is provided as a CRISPR/Cas system for editing or modifying a target gene; however, the CRISPR/Cas system is not limited thereto. The CRISPR/Cas system may be a CRISPR/Cas9 system, a CRISPR/Cascpf1 system, a CRISPR/Cas12f1 system, or a CRISPR/TnpB system. Recent studies have shown that transposon-associated transposase B (TnpB) can be used as an endonuclease for the CRISPR/Cas system (Altae-Tran H, Kannan S, Demircioglu FE, Oshiro R, Nety SP, McKay LJ, Dlakic M, Inskeep WP, Makarova KS, Macrae RK, Koonin EV, Zhang F. The widespread IS200/IS605 transposon family encodes diverse programmable RNA-guided endonucleases. Science. 2021 Oct;374(6563):57-65. doi: 10.1126/science.abj6856. Epub 2021 Sep 9. PMID: 34591643; PMCID: PMC8929163).

The disclosed system comprises (i) an endonuclease comprising at least one Cas protein or a nucleic acid construct encoding the endonuclease and (ii) at least one (for example, two) guide RNA molecule or a nucleic acid construct encoding the molecule.

In an embodiment, the Cas protein may be a Cas 9 protein, a Cpf1 protein, a Cas12f1 protein, or a TnpB protein.

In another embodiment, the Cas protein may be a Cas12f1 protein. The Cas12f1 protein may be in a wild-type, variant, or engineered form.

The present inventors have confirmed that TnpB (Transposon-associated transposase B) protein derived from *Candidatus Woesearchaeota* archaeon has an amino acid sequence similar to the UnCas12f1 protein (and thus, TnpB having an amino acid sequence similar to the UnCas12f1 protein is also named CWCas12f1; CWCas12f1 may be collectively referred to as Cas12f1 protein together with UnCas12f1, and may belong to a variant of Cas12f1 in its relationship with UnCas12f1), has a molecular weight that is about 1/3 smaller than that of an existing nucleic acid degrading protein including the Cas9 protein, which has been studied the most to date, and has significantly higher nucleic acid cleavage efficiency for a target nucleic acid or target gene. In addition, the present inventors have confirmed that engineered guide RNAs having a small size obtained by modifying the wild-type Cas12f1 guide RNA may induce excellent nucleic acid cleavage efficiency (for example, a double-strand break) together with the Cas12f1 protein such as CwCas12f1 or UnCas12f1. The hypercompact gene editing system comprising an engineered guide RNA and Cas12f1 or a variant thereof, such as CWCas12f1 or UnCas12f1, disclosed herein may be referred to as "CRISPR/Cas12f1 system" or "TaRGET system," and these terms may be used interchangeably. (However, for convenience, in the examples, the system using the UnCas12f1 protein is referred to as Cas12f1 system, and the system using the CWCas12f1 protein is referred to as TaRGET system). Hereinafter, respective components of the Cas12f1 gene editing system are described.

### 1. Endonuclease comprising Cas12f1 protein

The gene editing system based on CRISPR/Cas12f1 comprises an endonuclease comprising Cas12f1 or a nucleic acid encoding the endonuclease. The Cas12f1 protein is a (small) endonuclease characterized by exhibiting excellent activity in cleaving a target site of a target nucleic acid and being significantly smaller in size by about 1/3 compared to the existing CRISPR/Cas9 system.

Cas 12f1 is one of the effector proteins named Cas14 in a previous study (see Harrington et al., Science, 362, 839-842, 2018), and is also called Cas14a1 protein. The Cas12f1 protein disclosed herein may be a wild-type Cas12f1 protein existing in nature. In addition, the Cas12f1 protein may be a variant of the wild-type Cas12f1 protein. A variant of Cas12f1 is referred to as a "Cas12f1 variant." The Cas12f1 variant may be a variant having the same or equivalent function as the wild-type Cas12f1 protein, a variant of which some or all functions are modified, and/or a variant in which additional functions are added.

It has been reported that the Cas12f1 protein forms a complex with a guide RNA such that two Cas12f1 protein molecules bind to a guide RNA in the form of a dimer, and that all or part of the domain of the Cas12f1 protein recognizes a specific part of the scaffold region of the guide RNA to form a CRISPR/Cas12f1 complex (see Takeda et al., Structure of the miniature type V-F CRISPR-Cas effector enzyme, Molecular Cell 81, 1-13, 2021; and Xiao et al., Structural basis for the dimerization-dependent CRISPR-Cas12f nuclease, bioRxiv, 2020). Cas12f1 may generate a double-strand or single-strand break in a target nucleic acid or a target gene. Deletion of a desired gene segment may be induced by such a double-strand or single-strand break.

The Cas12f1 protein may recognize a protospacer adjacent motif (PAM) sequence present in a target nucleic acid or target gene. The PAM sequence is a unique sequence determined depending on the CRIPSR protein. The PAM sequence recognized by Cas12f1 may be a T-rich sequence. The PAM sequence recognized by Cas12f1 may be a sequence of 5'-TTTR-3', wherein R may be T, A, C, or G. Preferably, the PAM sequence may be 5'-TTTA-3', 5'-TTTT-3', 5'-TTTC-3' or 5'-TTTG-3'. More preferably, the PAM sequence may be 5'-TTTA-3' or 5'-TTTG-3'.

In an embodiment, the Cas12f1 protein may be derived from a Cas14 family (see Harrington et al., Science 362, 839-842 (2018); US 2020/0172886 A1).

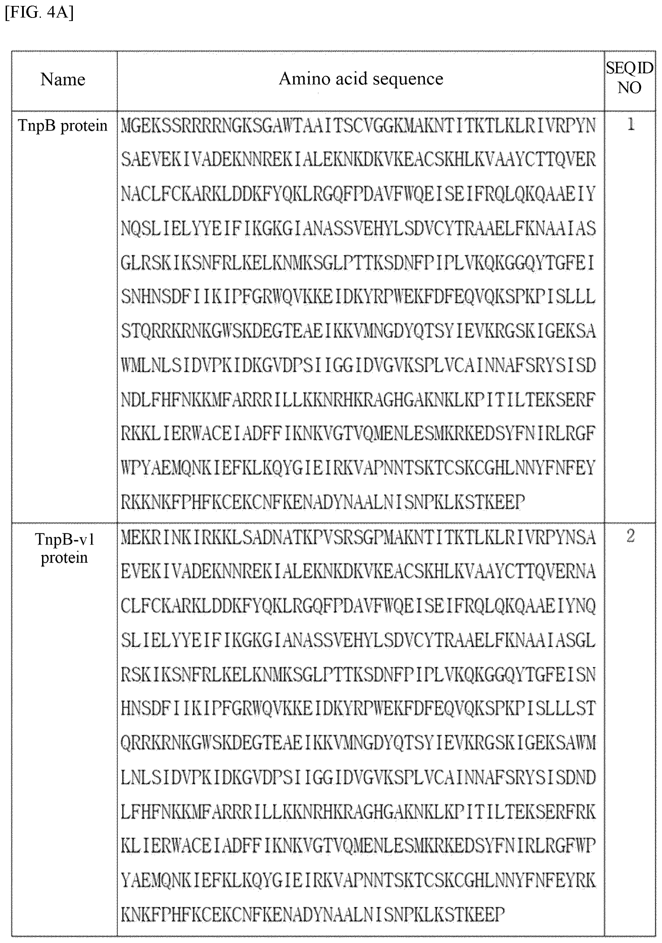

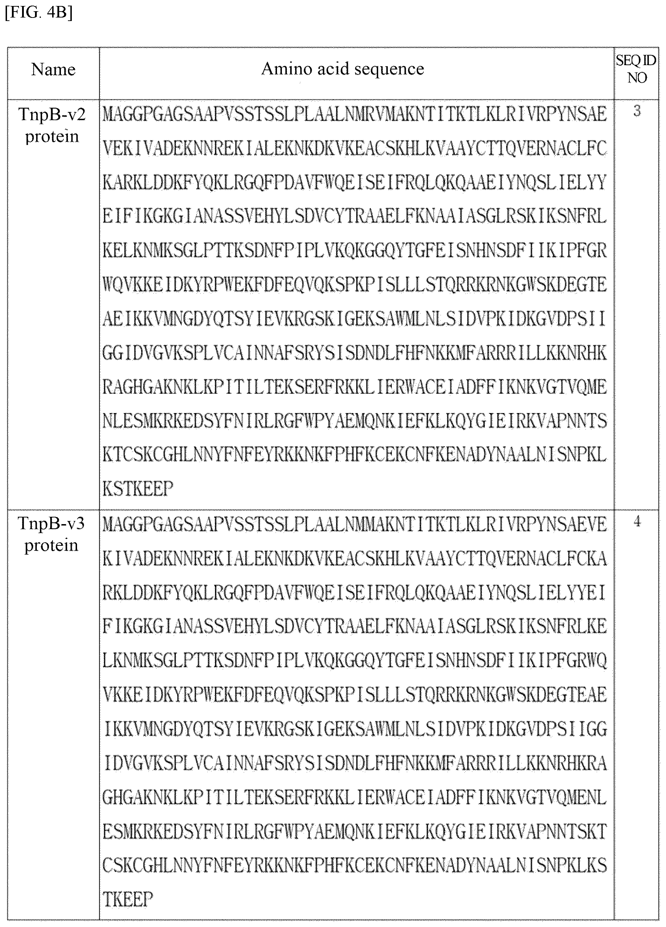

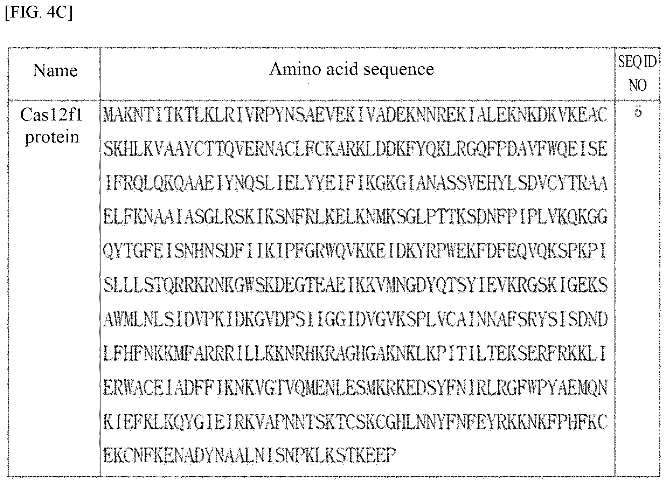

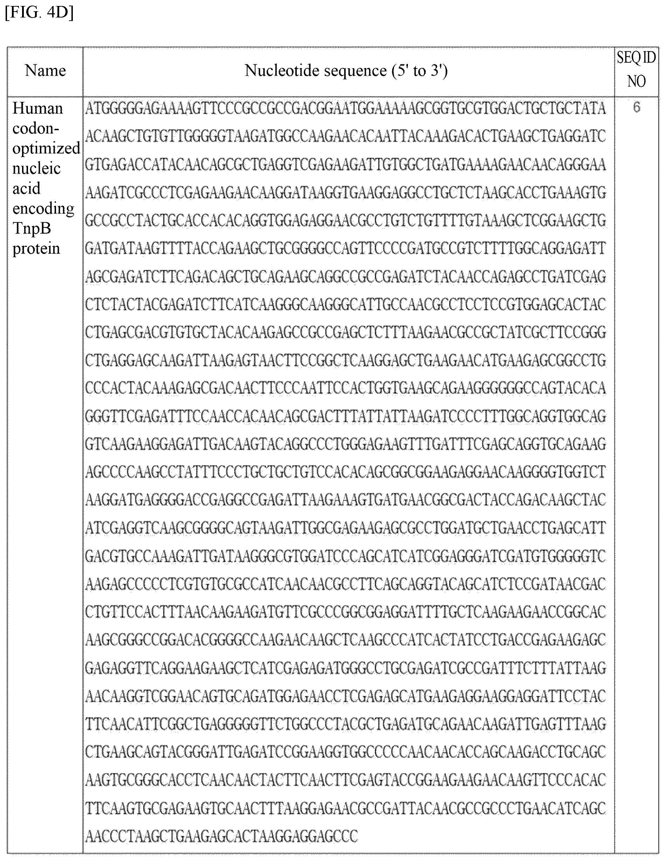

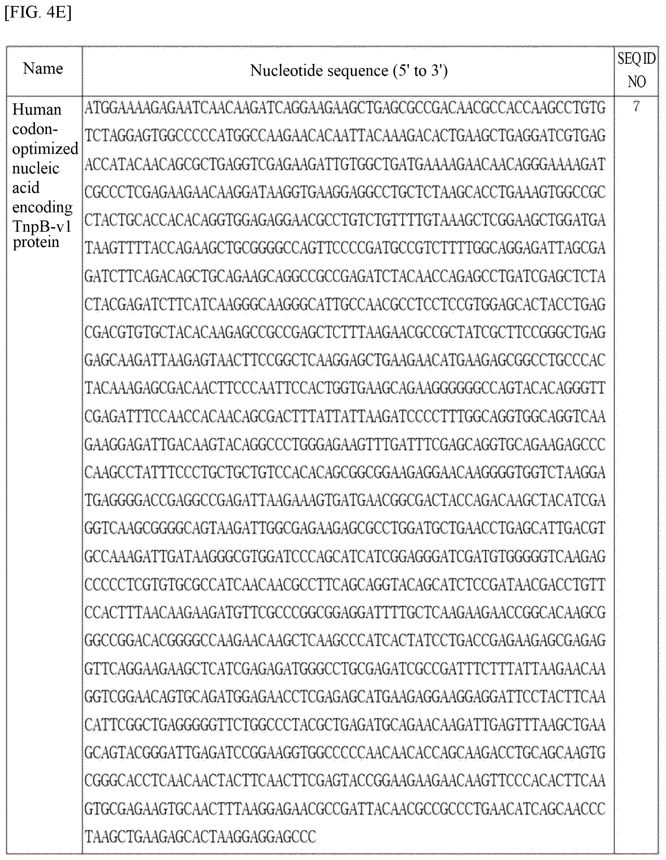

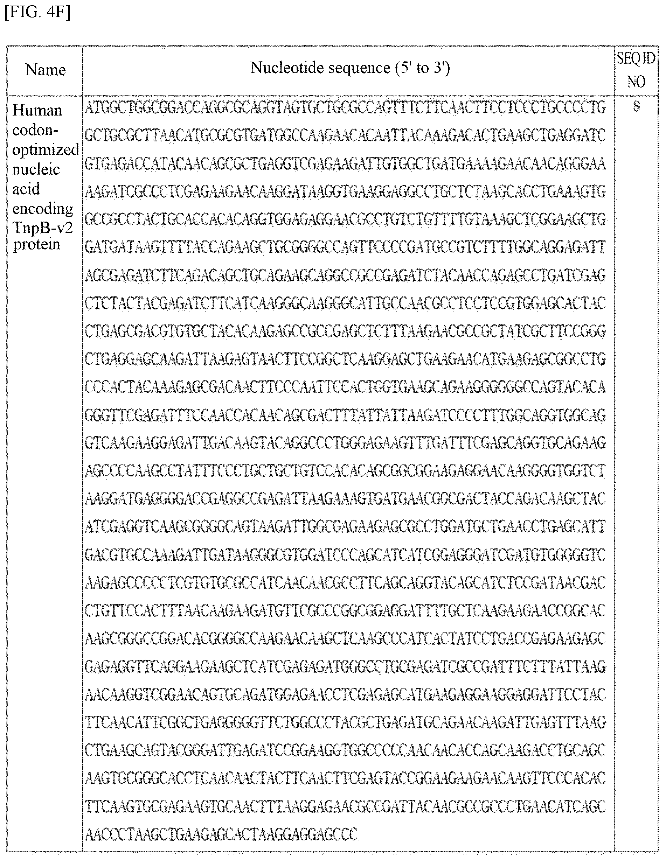

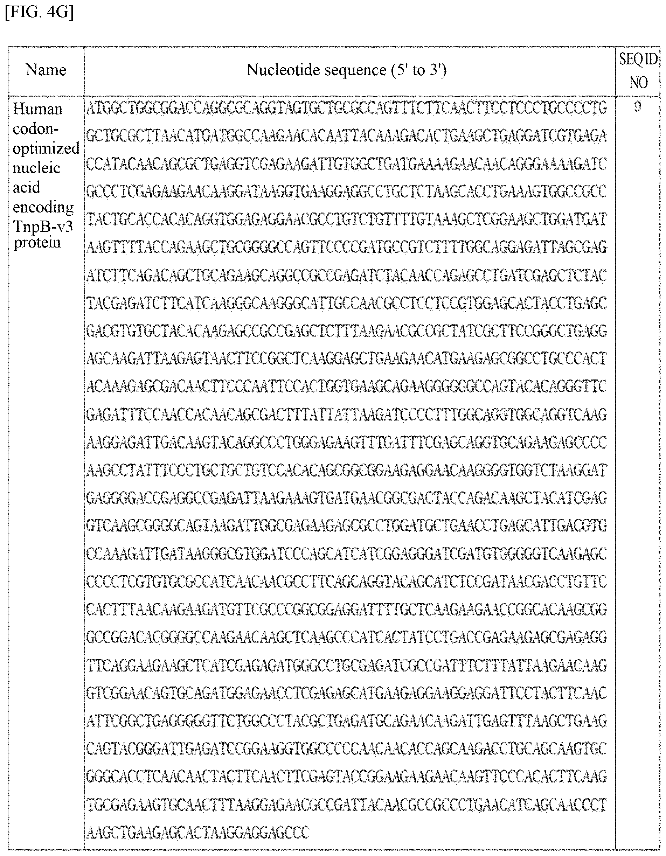

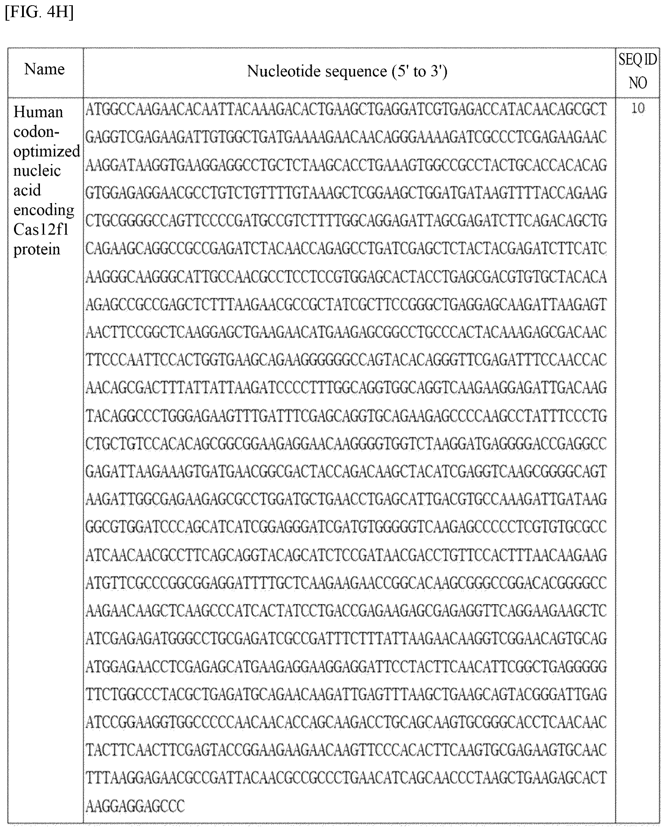

In another embodiment, the Cas12f1 protein may be a Cas14a1 (UnCas12f1) protein derived from a uncultured archaeon (Harrington et al., Science 362, 839-842 (2018); US 2020/0172886 A1). For example, the UnCas12f1 protein may comprise or consist of the amino acid sequence of SEQ ID NO: 5 (see FIG. 4).

In another embodiment, the Cas12f1 protein may be TnpB (transposon-associated transposase B) protein derived from the *Candidatus Woesearchaeota* archaeon. The TnpB protein is a protein conventionally known as a transposase. To date, the TnpB protein has been known only as a transposon-encoded nuclease, and it is not known whether the TnpB protein has Cas endonuclease activity. In addition, a guide RNA for the TnpB protein has also not been known. The present inventors have confirmed for the first time that TnpB variant or engineered TnpB, which is based on the TnpB protein sequence, has excellent endonuclease activity of targeting a target nucleic acid or a target gene and cleaving a double-stranded DNA of the target site while having a similar size to a Cas12f1 protein, which belongs to the group with the smallest molecular weight among nucleic acid degrading proteins, and have constructed an engineered guide RNA that exhibits excellent gene editing activity when used together with TnpB or a variant protein thereof. This TnpB protein is named CWCas12f1 protein. For example, the CWCas12f1 protein may comprise or consist of the amino acid sequence of SEQ ID NO: 1 (see FIG. 4).

In an embodiment, the Cas12f1 protein may be a Cas12f1 variant. The Cas12f1 variant may comprise a modification of at least one amino acid, such as deletion, substitution, insertion, or addition, compared to the amino acid sequence of the wild-type Cas12f1 protein.

In another embodiment, the Cas12f1 variant may comprise deletion of at least one amino acid or substitution with another amino acid sequence compared to the amino acid sequence of the wild-type Cas12f1 protein (for example, the amino acid sequence of RuvC domain or PAM recognition domain).

In another embodiment, the Cas12f1 variant may be a variant having at least one amino acid residue added to the N-terminus and/or C-terminus of the amino acid sequence of wild-type Cas12f1 (for example, UnCas12f1 or CWCas12f1) or a variant protein thereof. The present inventors have confirmed that among the variants having amino acids added to the N-terminus and/or C-terminus of the wild-type Cas12f1 protein, there are variants having a function equivalent to the wild-type Cas12f1. For this purpose, reference may be made to Korean Patent Application No. 10-2021-0181875, the entire disclosure of which should be deemed to be incorporated herein. Preferably, the Cas12f1 variant may be such that it has 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids added to the N-terminus and/or C-terminus of wild-type Cas12f1 or a variant protein thereof. In an embodiment, Cas12f1 (for example, UnCas12f1 or CWCas12f1) or a variant protein thereof may comprise an amino acid sequence having the amino acid sequence of SEQ ID NO: 1 in which 1 to 28 amino acids at the N-terminus are removed or substituted. For example, the Cas12f1 variant may comprise or consist of TnpB-v1 protein (SEQ ID NO: 2), which further comprises 26 amino acids derived from the N-terminus of CasX at the N-terminus of the UnCas12f1 protein, TnpB-v2 protein (SEQ ID NO: 3), which further comprises 28 random amino acid sequences at the N-terminus of the UnCas12f1 protein, or TnpB-v3 protein (SEQ ID NO: 4), which further comprises 26 random amino acid sequences at the N-terminus of the UnCas12f1 protein (see FIG. 4).

In an embodiment, the Cas12f1 variant may be such that it is engineered to recognize a PAM sequence other than 5'-TTTA-3' or 5'-TTTG-3'. In an embodiment, the Cas12f1 variant may comprise substitution of at least one amino acid residue selected from the group consisting of amino acids at position 170 (serine), position 174 (tyrosine), position 184 (alanine), position 188 (serine), position 191 (arginine), position 225 (glutamine), position 230 (tyrosine), position 271 (valine), and position 272 (glutamine) with respect to the wild-type sequence of CWCas12f1 (TnpB) (for example, amino acid sequence of SEQ ID NO: 1). Preferably, the Cas12f1 variant may comprise substitution of at least one amino acid residue selected from the group consisting of amino acids at position 170 (serine, S), position 188 (serine, S), position 191 (arginine, R), position 225 (glutamine, Q), and position 272 (glutamine, Q). More preferably, the Cas12f1 variant may comprise one or more selected from the following substitutions with respect to the wild-type sequence (for example, the amino acid sequence of SEQ ID NO: 1): S170T, S188Q, S188H, S188K, R191K, Q225T, Q225F, and Q272K (wherein T is threonine, Q is glutamine, H is histidine, K is lysine, and F is phenylalanine). In addition, the Cas12f1 variant may comprise an amino acid sequence selected from the group consisting of SEQ ID NOS: 392 to 399. These Cas12 variants may further recognize 5'-TNTN-3', 5'-TTTN-3', 5'-TGTA-3', 5'-TCTG-3', 5'-TGTG-3', or 5'-TTTC-3' as a PAM sequence, wherein N is A, T, C, or G.

In another embodiment, the Cas12f1 variant may be a fusion protein. The fusion protein may comprise two or more heterologous polypeptide domains, wherein one polypeptide domain comprises a Cas12f1 protein or a variant protein thereof, and the other domain comprises a (poly)peptide having another function or activity. For example, the (poly)peptide having another function or activity may have methylase activity, demethylase activity, transcription activation activity, transcription repression activity, transcription release factor activity, histone modification activity, RNA cleavage activity, or nucleic acid binding activity. In addition, the (poly)peptide, which has a different function or activity, may be a tag or reporter protein for separation and/or purification. For example, the tag or reporter protein includes, but is not limited to, a tag protein such as a histidine (His) tag, a V5 tag, a FLAG tag, an influenza hemagglutinin (HA) tag, a Myc tag, a VSV-G tag, and a thioredoxin (Trx) tag; a fluorescent protein such as green fluorescent protein (GFP), yellow fluorescent protein (YFP), cyan fluorescent protein (CFP), blue fluorescent protein (BFP), HcRED, and DsRed; and a reporter protein (enzyme) such as glutathione-S-transferase (GST), horseradish peroxidase (HRP), chloramphenicol acetyltransferase (CAT), β-galactosidase, β-glucuronidase, and luciferase.

In addition, the (poly)peptide having another function or activity may be, but is not limited to, a reverse transcriptase, a deaminase or another proteolytic enzyme.

In another embodiment, the Cas12f1 protein may comprise an amino acid sequence having at least 70%, at least 75%, at least 80%, at least 85%, at least 90%, or at least 95% sequence identity to an amino acid sequence selected from the group consisting of SEQ ID NOS: 1 to 5.

In an embodiment, the Cas12f1 protein may comprise one selected from the following sequences: (i) the amino acid sequence of SEQ ID NO: 5; (ii) the amino acid sequence of SEQ ID NO: 1; (iii) an amino acid sequence having the amino acid sequence of SEQ ID NO: 1 in which 1 to 28 amino acids at the N-terminus have been removed or substituted; or (iv) an amino acid sequence having the amino acid sequence of SEQ ID NO: 1 in which 1 to 600 amino acids have been added to the N-terminus or C-terminus.

In another embodiment, the Cas12f1 variant protein may be a protein comprising or consisting of one selected from the amino acid sequences having the amino acid sequence of SEQ ID NO: 1 in which 1 to 600 amino acids have been added to the N-terminus or C-terminus. Here, there is no limitation on the added sequence of 1 to 600 amino acids. For example, the added 1 to 600 amino acids may be the amino acid sequence of SEQ ID NO: 390 or SEQ ID NO: 391. An NLS or NES sequence may further be included between the added sequence and the Cas12f1 variant protein.

In an embodiment, since the target nucleic acid editing system cleaves a nucleic acid at a target site of a target nucleic acid or target gene, the target site may be located in the nucleus of a cell. The Cas12f1 protein may comprise one or more nuclear localization signal (NLS) sequences that localize the molecule into the nucleus. For example, one or more nuclear localization signal sequences may have a sufficient amount or activity to induce the Cas12f1 protein to be targeted to the nucleus of a eukaryotic cell (for example, a mammalian cell) in a detectable amount. For example, differences in the strength of activity may result from the number of NLSs included in the Cas12f1 protein, the type of specific NLS(s) used, or a combination of these factors. For example, the NLS may be, but is not limited to, an NLS sequence derived from NLS of SV40 virus large T-antigen, NLS from nucleoplasmin, c-myc NLS; hRNPA1 M9 NLS, the sequence of IBB domain from importin-alpha, the sequence of myoma T protein, the sequence of human p53, the sequence of mouse c-abl IV, the sequence of influenza virus NS1, the sequence of hepatitis virus delta antigen, the sequence of mouse Mx1 protein, the sequence of human poly(ADP-ribose) polymerase, or the sequence of steroid hormone receptor (human) glucocorticoid.

In another embodiment, the Cas12f1 protein may comprise a nuclear export sequence (NES).

In another embodiment, the Cas12f1 protein may be a fusion of various enzymes that may be involved in a gene expression process within cells. Here, the Cas12f1 protein to which the enzymes are fused may cause various quantitative and/or qualitative changes in gene expression in cells. For example, the various enzymes to be additionally bound may be DNMT, TET, KRAB, DHAC, LSD, p300, Moloney Murine Leukemia Virus (M-MLV) reverse transcriptase, or variants thereof. The Cas12f1 protein to which the reverse transcriptase is fused may also function as a prime editor.

In an embodiment, there is provided a nucleic acid encoding the Cas12f1 protein. The nucleic acid encoding Cas12f1 (including a variant thereof) may be codon optimized for a subject (for example, a human) to which the Cas12f1 protein is to be introduced. For example, the human codon optimized nucleotide sequence encoding Cas12f1 may be, for example, at least one selected from SEQ ID NOS: 6 to 10.

### 2. Guide RNA

As disclosed herein, the CRISPR/Cas12f1 system comprises at least one guide RNA or a nucleic acid construct encoding the guide RNA. Cas12f1 guide RNA provides targeting for CRISPR/Cas12f1. The guide RNA of the CRISPR/Cas12f1 system may be a Cas12f1 guide RNA found in nature or an engineered Cas12f1 guide RNA. The Cas12f1 guide RNA found in nature or engineered Cas12f1 guide RNA comprises a scaffold region and a spacer region. The scaffold region of the Cas12f1 guide RNA is a region that comprises parts of tracrRNA (trans-activating CRISPR RNA) and crRNA (CRISPR RNA) and functions to interact with the Cas12f1 protein. The spacer region of the Cas12f1 guide RNA comprises a guide sequence.

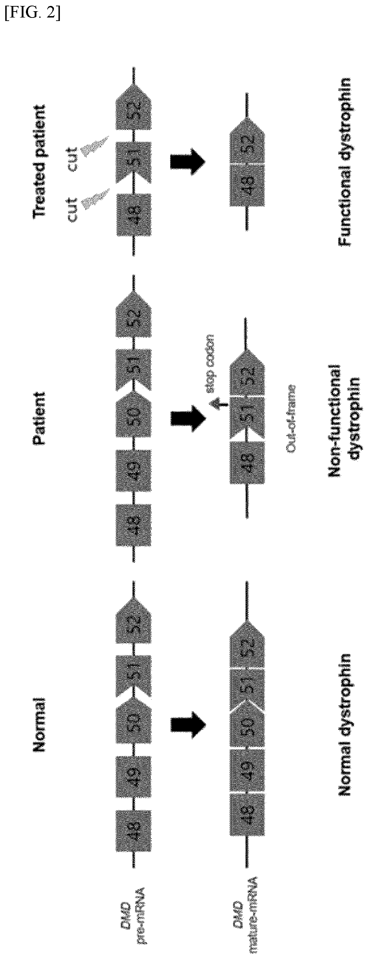

The wild-type gRNA includes two structures in which a part of tracrRNA (tracrRNA anti-repeat) and a part of crRNA repeat (crRNA repeat) are complementarily bound to form a duplex, which are conveniently referred to as R:AR1 and R:AR2. The wild-type guide RNA may comprise (i) at least one stem region, (ii) a tracrRNA-crRNA complementarity region, and optionally (iii) a region comprising three or more consecutive uracil (U) residues. Specifically, the wild-type guide RNA may sequentially comprise, from the 5'-end, a first stem region, a second stem region, a third stem region, a fourth stem region, and a fifth stem region (tracrRNA-crRNA complementarity region). For example, referring to FIG. 2, the scaffold region of the wild-type guide RNA comprises five stem regions, that is, a first stem region (stem 1), a second stem region (stem 2), a third stem region (stem 3), a fourth stem region (stem 4), and a fifth stem region (stem 5 (R:AR2)), from the 5'-end. The region comprising stem 5 (R:AR2) is also referred to as a tracrRNA-crRNA complementarity region.

More specifically, the wild-type gRNA may comprise a wild-type tracrRNA having the nucleotide sequence of SEQ ID NO: 11, or a wild-type crRNA having the nucleotide sequence of SEQ ID NO: 12. In addition, the wild-type gRNA may be fused in the form of a single guide RNA to become a single guide RNA (sgRNA) having the nucleotide sequence of SEQ ID NO: 13.

**[Table 1]**

| **Name** | **Nucleotide sequence** | **SEQ ID NO** |
|---|---|---|
| Wild-type tracrRNA | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAUUUUUCCUCUCCAAUUCUG CACAA | 11 |
| Wild-type crRNA | GUUGCAGAACCCGAAUAGACGAAUGAAGGAAUGCAAC | 12 |
| Canonical sgRNA | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAUUUUUCCUCUCCAAUUCUG CACAAgaaaGUUGCAGAACCCGAAUAGacgaaUGAAGGAAUG CAACNNNNNNNNNNNNNNNNNNNN | 13 |

### 2.1. Guide sequence

The guide RNA may comprise at least one guide sequence that hybridizes with a target sequence in a target gene. Since a protospacer sequence complementary to the target sequence is located at the 5'- or 3'-end of the PAM sequence recognized by the Cas12f1 protein, the guide sequence may be designed using the protospacer sequence. A guide sequence that binds complementarily to a target sequence may be designed as a nucleotide sequence having the same nucleotide sequence as the protospacer sequence. When the protospacer sequence is a DNA sequence, the guide sequence may be such that T in the protospacer sequence is replaced with U.

In an embodiment, the guide sequence may be hybridizable with or complementary to a target sequence of contiguous 15 to 30 bp in length, wherein the target sequence is located in a region 5000 bp, 4000 bp, 3000 bp, 2000 bp, or 1000 bp upstream of a nucleic acid segment to be deleted in a target gene, or a region 5000 bp, 4000 bp, 3000 bp, 2000 bp, or 1000 bp downstream of the nucleic acid segment, and is adjacent to the 5'-end or 3'-end of a PAM sequence recognized by the Cas12f1 protein.

In an embodiment, the guide sequence of the guide RNA may bind complementarily to the target sequence. Complementary binding between the guide sequence and the target sequence may include at least one mismatch bond. For example, complementary binding between the guide sequence and the target sequence may include 0 to 5 mismatches. The guide sequence may be a sequence having at least 70% sequence complementarity to the target sequence. Unless stated otherwise, "complementary" may mean including 0 to 5 mismatches or having at least 70% complementarity, and should be interpreted appropriately depending on the context. When the target sequence is DNA, for an adenosine (A) present in the target sequence, the guide sequence may comprise a uridine (U) residue that can form a complementary bond with A.

In an embodiment, the target sequence may be a sequence of 15 to 40 nucleotides. For example, the target sequence may be a sequence of 15 to 20, 15 to 25, 15 to 30, 15 to 35, or 15 to 40 nucleotides. The target sequence may be a sequence of 20 to 25, 20 to 30, 20 to 35, or 20 to 40 nucleotides. In addition, the target sequence may be a sequence of 25 to 30, 25 to 35, or 25 to 40 nucleotides. In addition, the target sequence may be a sequence of 30 to 35 or 30 to 40 nucleotides. In addition, the target sequence may be a sequence of 35 to 40 nucleotides. In addition, the target sequence may be a sequence of 15, 16, 17, 18, 19, 20, 21, 22, 23, 24, 25, 26, 27, 28, 29, 30, 31, 32, 33, 34, 35, 36, 37, 38, 39, or 40 nucleotides.

In an embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% complementary to the target sequence. Specifically, the guide sequence may be a sequence that is at least 80% to 85%, at least 80% to 90%, at least 80% to 95%, at least 80% to 100%, at least 85% to 90%, at least 85% to 95% or at least 85% to 100% complementary to the target sequence. More specifically, the guide sequence may be a sequence that is at least 90% to 95%, at least 90% to 100%, or at least 95% to 100% complementary to the target sequence. More specifically, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% complementary to the target sequence.

In another embodiment, the guide sequence may be identical to or similar to the protospacer sequence. The guide sequence may have at least 70% sequence identity to the protospacer sequence. For thymine (T) present in the protospacer sequence, the guide sequence may comprise uracil (U) instead of thymine (T).

In an embodiment, the guide sequence may have at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% sequence identity or similarity to the protospacer sequence. Specifically, the guide sequence may have at least 80% to 85%, at least 80% to 90%, at least 80% to 95%, at least 80% to 100%, at least 85% to 90%, at least 85% to 95%, or at least 85% to 100% sequence identity or similarity to the protospacer sequence. More specifically, the guide sequence may have at least 90% to 95%, at least 90% to 100%, or at least 95% to 100% identity or similarity to the protospacer sequence. Even more specifically, the guide sequence may have at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% identity or similarity to the protospacer sequence.

In an embodiment, the guide sequence may be a sequence that is hybridizable with or complementary to a nucleotide sequence present in an upstream region and/or a downstream region of a nucleic acid segment to be deleted. For example, a guide sequence of a first guide RNA may target a nucleotide sequence present in an upstream region of a nucleic acid segment to be deleted, and a guide sequence of a second guide RNA may target a nucleotide sequence present in a downstream region of the nucleic acid segment to be deleted.

In an embodiment, the guide sequence may be present at the 5'-end of the crRNA. In another embodiment, a U-rich tail may be added to the 5'-end of the guide sequence. The U-rich tail will be described later.

### 2.2. Engineered guide RNA

Since no naturally occurring gRNA has been found for CWCas12f1 according to an embodiment of the present disclosure, it was desired to produce an optimal gRNA exhibiting highly efficient targeting and editing activity not only for the engineered UnCas12f1 protein but also for the engineered CWCas12f1 protein. From this perspective, the gRNA may be a wild-type gRNA found in nature for wild-type UnCas12f1, which is similar in size to the CWCas12f1 protein. That is, in the present disclosure, the "wild-type" gRNA for the engineered Cas12f1 protein was used to mean "basic" or "canonical" gRNA.

In an embodiment, the guide RNA for the engineered Cas12f1 protein is characterized in that it is an engineered guide RNA in which a new configuration is added to a wild-type guide RNA found in nature, or the existing structure is removed and/or substituted, or whose structure is partially modified.

In an embodiment, the engineered gRNA is an engineered gRNA comprising a sequence having the wild-type gRNA sequence in which at least one nucleotide has been substituted, deleted, inserted, or added, wherein the sequence excluding the guide sequence has at least 50%, 55%, 60%, 65%, 70%, 75%, 80%, 85%, 90%, 91%, 92%, 93%, 94%, 95%, 96%, 97%, 98%, 99%, or 100% sequence identity to the wild-type Cas12f1 gRNA. In the context of RNA, nucleic acids, or polypeptides, the term "sequence identity" refers to a value determined by comparing two sequences that are optimally aligned over a comparison window, in which a sequence portion of RNA, nucleic acid, and the like within the comparison window may comprise insertions or deletions (that is, gaps) relative to the reference sequence to achieve optimal alignment.

Hereinafter, the structures of wild-type and engineered gRNAs and modifications thereof will be described in detail for each of the five modification sites. The modification site is abbreviated as "MS" throughout this specification, and the numbers following "modification site" or "MS" are sequentially assigned depending on engineering flow of each modification site according to an embodiment. However, this does not mean that engineering (modification) at a modification site with a later number necessarily includes engineering (modification) at a modification site with an earlier number. FIG. 2 illustrates modification sites MS1 to MS5 included in the engineered guide RNA according to an embodiment of the present disclosure on the wild-type guide RNA sequence.

The modifications applied to the engineered guide RNA (gRNA) of the present disclosure are ultimately intended to achieve high gene editing efficiency while deriving a gRNA that is shorter in length. That is, the modifications disclosed in the present disclosure are intended to produce an engineered gRNA of a shorter length having equal or improved recognition/cleavage efficiency for a target nucleic acid compared to the wild-type gRNA of a longer length, thereby allowing more space to be allocated to other components (for example, additional guide RNAs, shRNAs for inhibiting specific gene expression) for various purposes or uses within the packaging limit (about 4.7 kb) of a delivery vehicle such as adeno-associated virus (AAV). This provides a highly efficient gene editing effect that could not be achieved with the existing CRISPR/Cas system.

Therefore, the engineered gRNA provided in the present disclosure basically comprises a sequence having the wild-type Cas12f1 gRNA sequence in which one or more nucleotides are substituted, deleted, inserted, or added. Here, for the engineered gRNA, a portion thereof excluding the guide sequence may have sequence identity of 50% or more, 60% or more, 70% or more, 80% or more, 85% or more, 90% or more, or 95% or more to the wild-type Cas12f1 gRNA.

In an embodiment, compared to a wild-type Cas12f1 gRNA comprising (i) at least one stem region, (ii) a tracrRNA-crRNA complementarity region and optionally (iii) a region comprising three or more consecutive uracil (U) residues, the engineered gRNA of the present disclosure may comprise at least one modification selected from the group consisting of (a) deletion of at least a part of the at least one stem region; (b) deletion of at least a part of the tracrRNA-crRNA complementarity region; (c) replacement of one or more of uracil (U) residues when three or more consecutive uracil (U) residues are present; and (d) addition of one or more uridine residues to the 3'-end of the crRNA sequence.

In another embodiment, the engineered guide RNA may comprise at least one modification selected from the group consisting of (a1) deletion of at least a part of the first stem region; (a2) deletion of at least a part of the second stem region; (b) deletion of at least a part of the tracrRNA-crRNA complementarity region; (c) replacement of one or more U residues with A, G or C in three or more consecutive uracil (U) residues when the consecutive U residues are present in the tracrRNA-crRNA complementarity region; and (d1) addition of a U-rich tail to the 3'-end of the crRNA sequence, wherein a sequence of the U-rich tail is represented by 5'-(UₘV)ₙUₒ-3', wherein V is each independently A, C, or G, m and o are integers between 1 to 20, and n is an integer between 0 to 5.

In another embodiment, the engineered guide RNA may be represented by Formula (I).

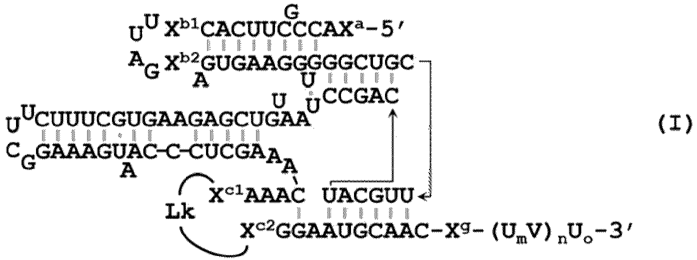

In Formula (I),
X^{a}, X^{b1}, X^{b2}, X^{c1}, and X^{c2} each independently consist of 0 to 35 (poly)nucleotides,
X^{g} is a guide sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

[In Formula (I), the black solid line refers to a chemical bond (for example, phosphodiester bond) between nucleotides or specific molecules, and the gray thick line refers to a complementary bond between nucleotides].

In an embodiment, X^{a} may be absent or a (poly)nucleotide having a stem-loop conformation.

In an embodiment, X^{b1} and X^{b2} may be (poly)nucleotides capable of complementary binding to each other.

In an embodiment, X^{c1} and X^{c2} may be (poly)nucleotides capable of complementary binding to each other.

In another embodiment, the engineered guide RNA may have at least 70%, at least 80%, at least 85%, at least 90%, at least 95%, or at least 98% sequence identity to the sequence represented by Formula (I). Here, the sequence identity with Formula (I) is based on the sequence excluding the regions indicated by the symbols.

When referring to the scaffold region of the wild-type guide RNA, the first stem region of the scaffold sequence may be a region corresponding to X^{a} in Formula (I). The second stem region of the scaffold sequence may be a region corresponding to X^{b1} and X^{b2} in Formula (I). The third stem region of the scaffold sequence may be a region corresponding to the sequence 5'-GGCUGCUUGCAUCAGCC-3' in Formula (I). The fourth stem region of the scaffold sequence may be a region corresponding to the sequence 5'-UCGAGAAGUGCUUUCUUCGGAAAGUAACCCUCGA-3' in Formula (I). In addition, the tracrRNA-crRNA complementarity region (the fifth stem region) of the scaffold sequence may be a region corresponding to X^{c1} and X^{c2} in Formula (I).

Hereinafter, modifications at respective modification sites in the engineered gRNA will be described in detail.

### (1) Modification at modification site 1 (MS1)

This section describes a modification at MS1. In an embodiment, wild-type tracrRNA (for example, SEQ ID NO: 11), which may be a guide RNA (gRNA) existing in nature, may have a sequence comprising five consecutive uracil (U) residues therein. This poses a problem in that, in a case of attempting to express the wild-type tracrRNA in a cell using a vector or the like, such a sequence acts as a transcription termination signal under certain conditions, thereby causing unintended early termination of transcription. That is, in a case where the sequence containing five consecutive U residues acts as a transcription termination signal, normal or complete expression of the tracrRNA is inhibited, and formation of normal or complete gRNA is also inhibited, which consequently decreases efficiency of cleavage or homology-directed repair of the target nucleic acid editing system of the present disclosure.

Therefore, in order to solve the above-mentioned problem, the engineered gRNAmay be such that at least one uracil (U) of three or more, four or more, or five or more consecutive U residues, preferably four or five U residues, which are contained in the wild-type tracrRNA (for example, SEQ ID NO: 11), is artificially modified into another nucleotide such as A, C, T, or G.

In an embodiment, the engineered gRNA is provided which comprises a modification in which at least one of three or more consecutive U residues is substituted with a different type of nucleotide in a region containing three or more consecutive U residues, referred to as MS1. For example, the three or more consecutive U residues may be present in the tracrRNA-crRNA complementarity region of the tracrRNA, wherein a modification may be made by substituting at least one of the three or more U residues with A, G, or C such that no sequence with three or more consecutive U residues exists.

Here, it is preferable that the sequence within the tracrRNA-crRNA complementarity region of crRNA, which corresponds to the sequence to be modified, is also modified together. In an embodiment, when there is the sequence 5'-ACGAA-3' within the tracrRNA-crRNA complementarity region of crRNA, which forms a partial complementary bond with the sequence 5'-UUUUU-3' within the tracrRNA-crRNA complementarity region of tracrRNA, this sequence may be replaced with 5'-NGNNN-3'. Here, N is each independently A, C, G, or U.

In another embodiment, MS1 may be present in the polynucleotides indicated by X^{c1} and X^{c2} in Formula (I).

In an embodiment, in the engineered gRNA of Formula (I), when three or more consecutive uracil (U) residues are present in the X^{c1} sequence, the sequence may comprise a modification in which one or more of U residues thereof are replaced with A, G, or C. For example, when the sequence 5'-UUUUU-3' is present in the X^{c1} sequence, the sequence may be replaced with 5'-NNNCN-3'. Here, N is each independently A, C, G, or U. As a more specific example, the sequence 5'-UUUUU-3' in the X^{c1} sequence may be replaced with any one nucleotide sequence selected from the group consisting of the following sequences; however, the replacing sequence is not limited to the following sequences as long as it prevents appearance of a sequence containing three or more consecutive U residues: 5'-UUUCU-3', 5'-GUUCU-3', 5'-UCUCU-3', 5'-UUGCU-3', 5'-UUUCC-3', 5'-GCUCU-3', 5'-GUUCC-3', 5'-UCGCU-3', 5'-UCUCC-3', 5'-UUGCC-3', 5'-GCGCU-3', 5'-GCUCC-3', 5'-GUGCC-3', 5'-UCGCC-3', 5'-GCGCC-3', and 5'-GUGCU-3'.

In another embodiment, in the engineered gRNA of Formula (I), the X^{c2} sequence comprises a region in which at least a part of the sequence forms a complementary bond with the X^{c1} sequence (also referred to as a tracrRNA-crRNA complementarity region), wherein a corresponding sequence in the X^{c2} sequence, which forms at least one complementary bond with 3 or more consecutive U residues present in the X^{c1} sequence, may also be modified. For example, when the sequence 5'-ACGAA-3' is present in the X^{c2} sequence of Formula (I), the sequence may be replaced with 5'-NGNNN-3'. Here, N is each independently A, C, G, or U. As a more specific example, the sequence 5'-ACGAA-3' in the X^{c1} sequence of Formula (I) may be replaced with any one nucleotide sequence selected from the group consisting of the following sequences; however, the replacing sequence is not limited to the following sequences: 5'-AGGAA-3', 5'-AGCAA-3', 5'-AGAAA-3', 5'-AGCAU-3', 5'-AGCAG-3', 5'-AGCAC-3', 5'-AGCUA-3', 5'-AGCGA-3', 5'-AGCCA-3', 5'-UGCAA-3', 5'-UGCUA-3', 5'-UGCGA-3', 5'-UGCCA-3', 5'-GGCAA-3', 5'-GGCUA-3', 5'-GGCGA-3', 5'-GGCCA-3', 5'-CGCAA-3', 5'-CGCUA-3', 5'-CGCGA-3', and 5'-CGCCA-3'.

In another embodiment, when a sequence containing 3 or more consecutive U residues in the X^{c1} sequence of Formula (I) is modified into another sequence, it is preferred that the corresponding nucleotides in the X^{c2} sequence (that is, at least some of which forms a complementary bond therewith) are modified so that they can form a complementary bond with the modified nucleotides. For example, when the sequence 5'-UUUUU-3' in the X^{c1} sequence is modified into 5'-GUGCU-3', it is preferred that the sequence 5'-ACGAA-3' in the X^{c2} sequence is modified into 5'-AGCAA-3'; however, complementary bonding is not necessarily required.

### (2) Modification at modification site 2 (MS2)

This section describes a modification at MS2. In an embodiment, the engineered guide RNA (gRNA) may be obtained by adding a new configuration to the gRNA found in nature, and may be such that one or more uridine residues are added to the 3'-end of the crRNA sequence. Here, the 3'-end of the crRNA sequence may be the 3'-end of the guide sequence (spacer). In the present disclosure, the one or more uridine residues added to the 3'-end are also referred to herein as a U-rich tail. The engineered gRNA comprising one or more uridine residues or a U-rich tail added to the 3'-end serves to increase nucleic acid cleavage or indel efficiency of the hypercompact CRISPR/Cas12 system for a target gene or target nucleic acid.

The term "U-rich tail" as used herein may refer not only to an RNA sequence itself that is rich in uridine (U), but also a DNA sequence encoding the same, and this may be appropriately interpreted depending on the context. The present inventors have experimentally elucidated the structure and effects of the U-rich tail sequence in detail, and the U-rich tail sequence will be described in more detail below with specific embodiments.

In an embodiment, the U-rich tail sequence may be represented by Ux, wherein x may be 1, 2, 3, 4, 5, 6, 7, 8, 9, 10, 11, 12, 13, 14, 15, 16, 17, 18, 19, or 20. For example, x may be an integer within a range of two numerical values selected from the numerical values listed above. For example, x may be an integer between 1 and 6. As another example, x may be an integer between 1 and 20. In an embodiment, x may be an integer of 20 or higher.

In another embodiment, the U-rich tail sequence is represented by 5'-(UₘV)ₙUₒ-3', wherein V may be each independently A, C or G, m and o may be integers from 1 to 20, and n may be an integer from 0 to 5. As an example, n may be 0, 1, or 2. As an example, m and o may be each independently 1, 2, 3, 4, 5, 6, 7, 8, 9, or 10.

In another embodiment, the engineered gRNA may be gRNA consisting of a sequence represented by Formula (I) or having at least 80%, at least 85%, at least 90%, or at least 95% sequence identity thereto. Here, MS2 is a region corresponding to (UₘV)ₙUₒ in Formula (I), wherein U is uridine, and V, m, o, and n are as defined above.

Preferably, in the engineered gRNA represented by Formula (I), (UₘV)ₙUₒ may be a U-rich tail in which (i) n is 0, o is an integer between 1 and 6, or (ii) V is A or G, m and o are each independently an integer between 3 and 6, and n is an integer between 1 and 3. In a specific example, (UₘV)ₙUₒ in Formula (I) may a U-rich tail consisting of any one sequence selected from the group consisting of 5'-U-3', 5'-UU-3', 5'-UUU-3', 5'-UUUU-3', 5'-UUUUU-3', 5'-UUUUUU-3', 5'-UUURUUU-3', 5'-UUURUUURUUU-3', 5'-UUUURU-3', 5'-UUUURUU-3', 5'-UUUURUUU-3', 5'-UUUURUUUU-3', 5'-UUUURUUUUU-3', and 5'-UUUURUUUUUU-3', wherein R is A or G.

In yet another embodiment, the U-rich tail sequence may comprise a modified uridine repeat sequence that contains a non-uridine ribonucleoside (A, C, or G) for every 1 to 5 repetitions of uridine. The modified uridine repeat sequence is particularly useful in a case of designing a vector that expresses an engineered crRNA. In an embodiment, the U-rich tail sequence may comprise a sequence in which UV, UUV, UUUV, UUUUV, and/or UUUUUV are repeated one or more times. Here, V is one of A, C or G.

In addition, the U-rich tail sequence may be a combination of the sequence represented by Ux and the sequence represented by (UaV)n. In an embodiment, the U-rich tail sequence may be represented by (U)n1-V1-(U)n2-V2-Ux. Here, V1 and V2 are each one of adenine (A), cytidine (C), and guanine (G). Here, n1 and n2 may each be an integer between 1 and 4. Here, x may be an integer between 1 and 20. In addition, the U-rich tail sequence may have a length of 1 nt, 2 nts, 3 nts, 4 nts, 5 nts, 6 nts, 7 nts, 8 nts, 9 nts, 10 nts, 11 nts, 12 nts, 13 nts, 14 nts, 15 nts, 16 nts, 17 nts, 18 nts, 19 nts, or 20 nts. In an embodiment, the U-rich tail sequence may have a length of 20 nts or longer.

In another embodiment, when the engineered gRNAis expressed in a cell, the U-rich tail may exist in a plurality of forms due to premature termination of transcription. For example, according to an embodiment, when a gRNA intended to contain a U-rich tail of the sequence 5'-UUUUAUUUUUU-3' is transcribed in a cell, four or more or five or more T residues may act as a termination sequence, and thus gRNAs containing a U-rich tail such as 5'-UUUUAUUUU-3', 5'-UUUUAUUUUU-3', or 5'-UUUUAUUUUUU-3' may be produced simultaneously. Therefore, in the present disclosure, a U-rich tail containing four or more U residues may be understood to also include a U-rich tail sequence having a shorter length than the intended length.

In yet another embodiment, the U-rich tail sequence may comprise additional nucleotides other than uridine, depending on the environment where the CRISPR/Cas12 system is actually used and expression environment, such as the internal environment of a eukaryotic cell or a prokaryotic cell.

### (3) Modification at modification site 3 (MS3)

This section describes a modification at MS3. As described above, MS3 refers to a region (which may be referred to as the first stem region) that comprises at least a part of the nucleotides forming a stem structure within a complex of the gRNA with an effector protein. The MS3 may comprise a region that does not interact with the effector protein when the gRNA and effector protein form a complex. The modification at MS3 involves removal of at least a part of the first stem region near the 5'-end of tracrRNA.

In an embodiment, the engineered gRNA comprises a modification in which at least a part of the first stem region (for example, the sequence of SEQ ID NO: 14) is deleted.

In another embodiment, the engineered gRNA comprises a modification in which at least a part of the first stem region on tracrRNA is deleted, wherein at least a part of the first stem region to be deleted may consist of 1 to 20 nucleotides. Specifically, at least a part of the first stem region may consist of 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, 19, or 20 nucleotides.

In yet another embodiment, the MS3 or the first stem region is a portion corresponding to the polynucleotide indicated by X^{a} of Formula (I), wherein due to a modification in which at least a part of the first stem region is deleted, X^{a} may consist of 0 to 35 (poly)nucleotides, preferably 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1 or 0 (poly)nucleotides.

In an embodiment, in the engineered gRNA of Formula (I), X^{a} may comprise the nucleotide sequence of SEQ ID NO: 14 or may comprise a nucleotide sequence having at least a part thereof, preferably a nucleotide sequence having the sequence of SEQ ID NO: 14 from which 1 to 20 nucleotides are deleted. For example, the nucleotide deletion may involve random deletion of at least 1, 2, 3, 5, 6, 7, 8, 9, 10, 11, 12, 13, 14 15, 16, 17, 18, 19, or 20 nucleotides from the sequence of SEQ ID NO: 14. As a preferred example, the nucleotide deletion may involve sequential deletion of at least 1, 2, 3, 5, 6, 7, 8, 9, 10, 11, 12, 13, 14 15, 16, 17, 18, 19, or 20 nucleotides from the 5'-end of the sequence of SEQ ID NO: 14. More specifically, X^{a} may comprise or consist of CUUCACUGAUAAAGUGGAGA-3' (SEQ ID NO: 14), 5'-UUCACUGAUAAAGUGGAGA-3' (SEQ ID NO: 15), 5'-UCACUGAUAAAGUGGAGA-3' (SEQ ID NO: 16), 5'-CACUGAUAAAGUGGAGA-3' (SEQ ID NO: 17), 5'-ACUGAUAAAGUGGAGA-3' (SEQ ID NO: 18), 5'-CUGAUAAAGUGGAGA-3' (SEQ ID NO: 19), 5'-UGAUAAAGUGGAGA-3' (SEQ ID NO: 20), 5'-GAUAAAGUGGAGA-3' (SEQ ID NO: 21), 5'-AUAAAGUGGAGA-3' (SEQ ID NO: 22), 5'-UAAAGUGGAGA-3' (SEQ ID NO: 23), 5'-AAAGUGGAGA-3' (SEQ ID NO: 24), 5'-AAGUGGAGA-3', 5'-AGUGGAGA-3', 5'-GUGGAGA-3', 5'-UGGAGA-3', 5'-GGAGA-3', 5'-GAGA-3', 5'-AGA-3', 5'-GA-3', or 5'-A-3', or X^{a} may be absent.

### (4) Modification at modification site 4 (MS4)

This section describes a modification at MS4. MS4 refers to a region spanning the 3'-end of tracrRNA and the 5'-end of crRNA, or, in a case of a single guide RNA form, a region where the sequence corresponding to tracrRNA and the sequence corresponding to crRNA form at least partial complementary bonding. MS4 may comprise at least a part of the sequence referred to as the tracrRNA-crRNA complementarity region (which may also be referred to as the fifth stem region). In the present disclosure, the tracrRNA-crRNA complementarity region may comprise both modification site 1 (MS1) and modification site 4 (MS4). The modification at MS4 comprises deletion of at least a part of the tracrRNA-crRNA complementarity region. The tracrRNA-crRNA complementarity region may comprise a part of tracrRNA and a part of crRNA. In this regard, the tracrRNA-crRNA complementarity region may comprise nucleotides such that partial nucleotides contained in tracrRNA can form complementary bonds with partial nucleotides contained in crRNA within a complex of gRNA with the nucleic acid degrading protein, and may comprise nucleotides adjacent thereto. The tracrRNA-crRNA complementarity region of tracrRNA may comprise a region that does not interact with the nucleic acid degrading protein within a complex of gRNA with the nucleic acid degrading protein.

In some embodiments, the engineered gRNA comprises deletion of at least a part of the tracrRNA-crRNA complementarity region in tracrRNA, deletion of at least a part of the tracrRNA-crRNA complementarity region in crRNA, or deletion of at least a part of the tracrRNA-crRNA complementarity region in both the tracrRNA and the crRNA.

In another embodiment, the engineered gRNA comprises a modification in which a part of the tracrRNA-crRNA complementarity region is deleted, wherein the part of the complementary region to be deleted may consist of 1 to 54 nucleotides.

In yet another embodiment, the engineered gRNA comprises a modification in which the entire tracrRNA-crRNA complementarity region is deleted, wherein the entire complementary region to be deleted may consist of 55 nucleotides.

In an embodiment, the tracrRNA-crRNA complementarity region may comprise the nucleotide sequence of SEQ ID NO: 39 and/or the nucleotide sequence of SEQ ID NO: 58.

In another embodiment, the tracrRNA-crRNA complementarity region may further comprise a linker sequence.

Specifically, at least a part of the tracrRNA-crRNA complementarity region may consist of 3 to 55, 5 to 55, 7 to 55, 9 to 55, 11 to 55, 13 to 55, 15 to 55, 17 to 55, 19 to 55, 21 to 55, 23 to 55, 25 to 55, 27 to 55, 29 to 55, 31 to 55, 33 to 55, 35 to 55, 37 to 55, 39 to 55, or 41 to 55 nucleotides, preferably 42 to 55, 43 to 55, 44 to 55, 45 to 55, 46 to 55, 47 to 55, 48 to 55, 49 to 55, 50 to 55, 51 to 55, 52 to 55, 53 to 55, or 54, or 55 nucleotides.

In yet another embodiment, MS4 or the tracrRNA-crRNA complementarity region is a region corresponding to the polynucleotide indicated by X^{c1} and X^{c2} in Formula (I), in which due to the modification where at least a part of the tracrRNA-crRNA complementarity region is deleted, X^{c1} and X^{c2} may each independently consist of 0 to 35 (poly)nucleotides.

Preferably, X^{c1} may consist of 0 to 28, 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides. In addition, preferably, X^{c2} may consist of 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides.

In an embodiment, in the engineered gRNA of Formula (I), X^{c1} may comprise the nucleotide sequence of SEQ ID NO: 39 or a nucleotide sequence having the sequence of SEQ ID NO: 39 from which 1 to 28 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, 27, or 28 nucleotides from the 5'-end of the sequence of SEQ ID NO: 39. More specifically, X^{c1} may comprise or consist of 5'-UUCAUUUUUCCUCUCCAAUUCUGCACAA-3' (SEQ ID NO: 39), 5'-UUCAUUUUUCCUCUCCAAUUCUGCACA-3' (SEQ ID NO: 40), 5'-UUCAUUUUUCCUCUCCAAUUCUGCAC-3' (SEQ ID NO: 41), 5'-UUCAUUUUUCCUCUCCAAUUCUGCA-3' (SEQ ID NO: 42), 5'-UUCAUUUUUCCUCUCCAAUUCUGC-3' (SEQ ID NO: 43), 5'-UUCAUUUUUCCUCUCCAAUUCUG-3' (SEQ ID NO: 44), 5'-UUCAUUUUUCCUCUCCAAUUCU-3' (SEQ ID NO: 45), 5'-UUCAUUUUUCCUCUCCAAUUC-3' (SEQ ID NO: 46), 5'-UUCAUUUUUCCUCUCCAAUU-3' (SEQ ID NO: 47), 5'-UUCAUUUUUCCUCUCCAAU-3' (SEQ ID NO: 48), 5'-UUCAUUUUUCCUCUCCAA-3' (SEQ ID NO: 49), 5'-UUCAUUUUUCCUCUCCA-3' (SEQ ID NO: 50), 5'-UUCAUUUUUCCUCUCC-3' (SEQ ID NO: 51), 5'-UUCAUUUUUCCUCUC-3' (SEQ ID NO: 52), 5'-UUCAUUUUUCCUCU-3' (SEQ ID NO: 53), 5'-UUCAUUUUUCCUC-3' (SEQ ID NO: 54), 5'-UUCAUUUUUCCU-3' (SEQ ID NO: 55), 5'-UUCAUUUUUCC-3' (SEQ ID NO: 56), 5'-UUCAUUUUUC-3' (SEQ ID NO: 57), 5'-UUCAUUUUU-3', 5'-UUCAUUUU-3', 5'-UUCAUUU-3', 5'-UUCAUU-3', 5'-UUCAU-3', 5'-UUCA-3', 5'-UUC-3', 5'-UU-3', or 5'-U-3', or X^{c1} may be absent.

Here, in a case where there is a region containing 3, 4, or 5 or more uracil (U) residues in the sequence of X^{c1} from which some nucleotides have been removed, the modification at MS1 as described above may also apply. For details about MS1, see the section "(1) Modification at modification site 1 (MS1)."

In another embodiment, in the engineered gRNA of Formula (I), X^{c2} may comprise the nucleotide sequence of SEQ ID NO: 58 or a nucleotide sequence having the sequence of SEQ ID NO: 58 from which 1 to 27 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, or 27 nucleotides from the 5'-end of the sequence of SEQ ID NO: 58. More specifically, X^{c2} may comprise or consist of 5'-GUUGCAGAACCCGAAUAGACGAAUGAA-3' (SEQ ID NO: 58), 5'-UUGCAGAACCCGAAUAGACGAAUGAA-3' (SEQ ID NO: 59), 5'-UGCAGAACCCGAAUAGACGAAUGAA-3' (SEQ ID NO: 60), 5'-GCAGAACCCGAAUAGACGAAUGAA-3' (SEQ ID NO: 61), 5'-CAGAACCCGAAUAGACGAAUGAA-3' (SEQ ID NO: 62), 5'-AGAACCCGAAUAGACGAAUGAA-3' (SEQ ID NO: 63), 5'-GAACCCGAAUAGACGAAUGAA-3' (SEQ ID NO: 64), 5'-AACCCGAAUAGACGAAUGAA-3' (SEQ ID NO: 65), 5'-ACCCGAAUAGACGAAUGAA-3' (SEQ ID NO: 66), 5'-CCCGAAUAGACGAAUGAA-3' (SEQ ID NO: 67), 5'-CCGAAUAGACGAAUGAA-3' (SEQ ID NO: 68), 5'-CGAAUAGACGAAUGAA-3' (SEQ ID NO: 69), 5'-GAAUAGACGAAUGAA-3' (SEQ ID NO: 70), 5'-AAUAGACGAAUGAA-3' (SEQ ID NO: 71), 5'-AUAGACGAAUGAA-3' (SEQ ID NO: 72), 5'-UAGACGAAUGAA-3' (SEQ ID NO: 73), 5'-AGACGAAUGAA-3' (SEQ ID NO: 74), 5'-GACGAAUGAA-3' (SEQ ID NO: 75), 5'-ACGAAUGAA-3', 5'-CGAAUGAA-3', 5'-GAAUGAA-3', 5'-AAUGAA-3', 5'-AUGAA-3', 5'-UGAA-3', 5'-GAA-3', 5'-AA-3', or 5'-A-3', or X^{c2} may be absent.

Here, in a case where there is a sequence corresponding a sequence containing 3 or more, or 3, 4, or 5 or more uracil (U) residues in the sequence of X^{c2} from which some nucleotides have been removed, the modification at MS1 as described above may also apply. For details regarding MS1, see the section "(1) Modification at modification site 1 (MS1)."

In the engineered gRNA of Formula (I), the regions corresponding to X^{c1} and X^{c2} may each independently undergo the above-described modification. However, MS4 or the tracrRNA-crRNA complementarity region is a region where tracrRNA and crRNA form complementary bonds. For the tracrRNA and the crRNA to function as a dual guide RNA, it is preferable that the position and number of nucleotides to be deleted in each of X^{c1} and X^{c2} be identical with or similar to each other. That is, in order to preserve complementarity, in a case of sequentially deleting nucleotides from the 3'-end of tracrRNA in MS4 (tracrRNA-crRNA complementarity region), it is preferable to sequentially delete nucleotides from the 5'-end of crRNA.

In some embodiments, the 3'-end of X^{c1} and the 5'-end of X^{c2} in the engineered gRNA of Formula (I) may be linked by a linker (Lk) so that the gRNAis modified into a single guide RNA (sgRNA) form. The linker Lk is a sequence that physically or chemically connects tracrRNA and crRNA, and may be a polynucleotide sequence having a length of 1 to 30 nucleotides. In an embodiment, Lk may be a sequence of 1 to 5, 5 to 10, 10 to 15, 2 to 20, 15 to 20, 20 to 25, or 25 to 30 nucleotides. For example, Lk may be, but is not limited to, 5'-GAAA-3'. As another example, Lk may be a linker comprising or consisting of 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), or 5'-UUCAUUGAAAAAUGAA-3' (SEQ ID NO: 79).

Meanwhile, while it is possible to use a linker (Lk) to make a single guide RNA (sgRNA), it is also possible to directly connect the 3'-end of tracrRNA, of which a partial sequence has been removed, to the 5'-end of crRNA of which a partial sequence has been removed.

In another embodiment, a case where X^{c1} and X^{c2} in the engineered gRNA of Formula (I) are linked by a linker may be indicated by 5'-X^{c1}-Lk-X^{c2}-3' as in Formula (I), and the 5'-X^{c1}-Lk-X^{c2}-3' may be any one nucleotide sequence selected from the group consisting of SEQ ID NOS: 80 to 86, but is not limited thereto.

### (5) Modification at modification site 5 (MS5)

This section describes a modification at MS5. As described above, MS5 corresponds to a region located toward the 3'-end of tracrRNA, which is referred to as the second stem region. The second stem region may comprise nucleotides that form a stem structure within a complex of the guide RNA (gRNA) with nucleic acid editing protein, and may comprise nucleotides adjacent thereto. Here, the stem structure is distinct from the stem included in the above-described first stem region.

In an embodiment, the engineered gRNA comprises a modification in which at least a part of the second stem region is deleted.

In another embodiment, the engineered gRNA comprises deletion of at least a part of the second stem region, wherein at least a part of the second stem region to be deleted may consist of 1 to 27 nucleotides. Specifically, the at least a part of the second stem region may consist of 2 to 27, 3 to 27, 4 to 27, 5 to 27, 6 to 27, 7 to 27, 8 to 27, 9 to 27, 10 to 27, 11 to 27, 12 to 27, 13 to 27, 14 to 27, 15 to 27, 16 to 27, 17 to 27, 18 to 27, 19 to 27, 20 to 27, 21 to 27, 22 to 27, 23 to 27, 24 to 27, 25 to 27, 26, or 27 nucleotides.

In an embodiment, the second stem region may comprise or consist of the nucleotide sequence of SEQ ID NO: 25 and/or the nucleotide sequence of SEQ ID NO: 29.

In another embodiment, MS5 or the second stem region is a region comprising a (poly)nucleotide (comprising a loop of 5'-UUAG-3') that is adjacent to the polynucleotide indicated by X^{b1} and X^{b2} in Formula (I), in which due to the modification where at least the part of the second stem region is deleted, X^{b1} and X^{b2} may each independently consist of 0 to 35 (poly)nucleotides.

Preferably, X^{b1} in Formula (I) may consist of 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides. In addition, preferably, X^{b2} may consist of 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides.

In an embodiment, in the engineered gRNA of Formula (I), X^{b1} may comprise the nucleotide sequence of SEQ ID NO: 25 or a nucleotide sequence having the sequence of SEQ ID NO: 25 from which 1 to 13 nucleotides are deleted. Preferably, the nucleotide deletion may involve sequential removal of at least 1, 2, 3, 5, 6, 7, 8, 9, 10, 11, 12, or 13 nucleotide from the 5'-end of the sequence of SEQ ID NO: 25. More specifically, X^{b1} may comprise or consist of 5'-CAAAAGCUGUCCC-3' (SEQ ID NO: 25), 5'-CAAAAGCUGUCC-3' (SEQ ID NO: 26), 5'-CAAAAGCUGUC-3' (SEQ ID NO: 27), 5'-CAAAAGCUGU-3' (SEQ ID NO: 28), 5'-CAAAAGCUG-3', 5'-CAAAAGCU-3', 5'-CAAAAGC-3', 5'-CAAAAG-3', 5'-CAAAA-3', 5'-CAAA-3', 5'-CAA-3', 5'-CA-3', or 5'-C-3', or X^{b1} may be absent.

In another embodiment, in the engineered gRNA of Formula (I), X^{b2} may comprise the nucleotide sequence of SEQ ID NO: 29 or a nucleotide sequence having the sequence of SEQ ID NO: 29 from which 1 to 14 nucleotides are deleted. Preferably, the nucleotide deletion may involve sequential removal of at least 1, 2, 3, 5, 6, 7, 8, 9, 10, 11, 12, 13, or 14 nucleotides from the 5'-end of the sequence of SEQ ID NO: 29. More specifically, X^{b2} may comprise or consist of 5'-GGGAUUAGAACUUG-3' (SEQ ID NO: 29), 5'-GGAUUAGAACUUG-3' (SEQ ID NO: 30), 5'-GAUUAGAACUUG-3' (SEQ ID NO: 31), 5'-AUUAGAACUUG-3' (SEQ ID NO: 32), 5'-UUAGAACUUG-3' (SEQ ID NO: 33), 5'-UAGAACUUG-3', 5'-AGAACUUG-3', 5'-GAACUUG-3', 5'-AACUUG-3', 5'-ACUUG-3', 5'-CUUG-3', 5'-UUG-3', 5'-UG-3', or 5'-G-3', or X^{b2} may be absent.

In the engineered gRNA of Formula (I), the regions corresponding to X^{b1} and X^{b2} may be each independently modified. However, for normal preservation of the stem-loop structure, it is preferable that the position and number of nucleotides to be deleted in each of X^{b1} and X^{b2} be identical with or similar to each other. For example, in a case of sequentially deleting nucleotides from the 5'-end direction in X^{b1}, it is preferable to sequentially delete nucleotides from the 3'-end direction in X^{b2}.

In another embodiment, a sequence of the loop portion connecting X^{b1} and X^{b2} in the engineered gRNA of Formula (I) is indicated by 5'-UUAG-3', and this may be replaced with another sequence such as 5'-NNNN-3' and '5-NNN-3', if necessary. Here, N is each independently A, C, G, or U. For example, the 5'-NNNN-3' may be 5'-GAAA-3', and the '5-NNN-3' may be 5'-CGA-3'.

For example, in the engineered gRNA of Formula (I), a sequence of the loop portion connecting X^{b1} and X^{b2} is 5'-UUAG-3', and the sequence 5'-X^{b1}UUAG X^{b2}-3' in Formula (I) may comprise or consist of any one nucleotide sequence selected from the group consisting of SEQ ID NOS: 34 to 38.

### (6) Examples of gRNAs to which modifications at modification sites 1 to 5 have been applied

The engineered guide RNA (gRNA) included in the target nucleic acid editing system of the present disclosure may comprise modifications at two or more of the above-mentioned modification sites 1 (MS1) to 5 (MS5).

In some embodiments, the engineered guide RNA may comprise one or more modifications selected from the group consisting of (a1) deletion of at least a part of the first stem region; (a2) deletion at least a part of the second stem region; (b) deletion of at least a part of the tracrRNA-crRNA complementarity region; (c) replacement of one or more uracil (U) residues with A, G, or C in three or more consecutive U residues when the consecutive U residues are present in the tracrRNA-crRNA complementarity region; and (d1) addition of a U-rich tail to the 3'-end of the crRNA sequence. The U-rich tail sequence may be represented by 5'-(UₘV)ₙUₒ-3', wherein V is each independently A, C, or G, m and o are integers between 1 to 20, and n is an integer between 0 to 5.

For example, the engineered guide RNA may comprise (d1) addition of a U-rich tail to the 3'-end of the crRNA sequence and (c) replacement of one or more uracil (U) residues with A, G, or C in three or more consecutive U residues when the consecutive U residues are present in the tracrRNA-crRNA complementarity region.

As another example, the engineered guide RNA may comprise (d1) addition of a U-rich tail to the 3'-end of the crRNA sequence, (c) replacement of one or more U residues with A, G or C in three or more consecutive U residues when the consecutive U residues are present in the tracrRNA-crRNA complementarity region, and (a1) deletion of at least a part of the first stem region.

As yet another example, the engineered guide RNA may comprise (d1) addition of a U-rich tail to the 3'-end of the crRNA sequence, (c) replacement of one or more U residues with A, G or C in three or more consecutive U residues when the consecutive U residues are present in the tracrRNA-crRNA complementarity region, and (a1) deletion of at least a part of the first stem region.

As still yet another example, the engineered guide RNA may comprise (d1) addition of a U-rich tail to the 3'-end of the crRNA sequence, (a1) deletion of at least a part of the first stem region, and (b) deletion of at least a part of the tracrRNA-crRNA complementarity region, wherein the engineered guide RNA may further comprise replacement of one or more U residues with A, G or C in three or more consecutive U residues when the consecutive U residues are present in the tracrRNA-crRNA complementarity region containing partial deletion.

As still yet another example, the engineered guide RNA may comprise (d1) addition of a U-rich tail to the 3'-end of the crRNA sequence, (a1) deletion of at least a part of the first stem region, (b) deletion of at least a part of the tracrRNA-crRNA complementarity region, and (a2) deletion of at least a part of the second stem region, wherein the engineered guide RNA may further comprise replacement of one or more U residues with A, G or C in three or more consecutive U residues when the consecutive U residues are present in the tracrRNA-crRNA complementarity region containing partial deletion.

As an example of tracrRNA to which modifications at the plurality of modification sites (MS) as described above have been applied, there is provided an engineered tracrRNA comprising the nucleotide sequence of any one of SEQ ID NOS: 87 to 132.

Specifically, the engineered tracrRNA of the present disclosure may comprise or consist of the nucleotide sequence of SEQ ID NO: 87 (MS1), SEQ ID NO: 88 (MS1/MS3-1), SEQ ID NO: 89 (MS1/MS3-2), SEQ ID NO: 90 (MS1/MS3-3), SEQ ID NO: 91 (MS1/MS4*-1), SEQ ID NO: 92 (MS1/MS4*-2), SEQ ID NO: 93 (MS1/MS4*-3), SEQ ID NO: 94 (MS1/MS5-1), SEQ ID NO: 95 (MS1/MS5-2), SEQ ID NO: 96 (MS1/MS5-3), SEQ ID NO: 97 (MS1/MS3-3/MS4*-1), SEQ ID NO: 98 (MS1/MS3-3/MS4*-2), SEQ ID NO: 99 (MS1/MS3-3/MS4*-3), SEQ ID NO: 100 (MS1/MS4*-2/MS5-1), SEQ ID NO: 101 (MS1/MS4*-2/MS5-2), SEQ ID NO: 102 (MS1/MS4*-2/MS5-3), SEQ ID NO: 103 (MS1/MS3-3/MS5-1), SEQ ID NO: 104 (MS1/MS3-3/MS5-2), SEQ ID NO: 105 (MS1/MS3-3/MS5-3), SEQ ID NO: 106 (MS1/MS3-3/MS4*-2/MS5-3), SEQ ID NO: 107 (mature form, MF), SEQ ID NO: 108 (MF/MS3-1), SEQ ID NO: 109 (MF/MS3-2), SEQ ID NO: 110 (MF/MS3-3), SEQ ID NO: 111 (MF/MS4-1), SEQ ID NO: 112 (MF/MS4-2), SEQ ID NO: 113 (MF/MS4-3), SEQ ID NO: 114 (MF/MS5-1), SEQ ID NO: 115 (MF/MS5-2), SEQ ID NO: 116 (MF/MS5-3), SEQ ID NO: 117 (MF/MS5), SEQ ID NO: 118 (MF/MS3-3/MS4-1), SEQ ID NO: 119 (MF/MS3-3/MS4-2), SEQ ID NO: 120 (MF/MS3-3/MS4-3), SEQ ID NO: 121 (MF/MS4-3/MS5-1), SEQ ID NO: 122 (MF/MS4-3/MS5-2), SEQ ID NO: 123 (MF/MS4-3/MS5-3), SEQ ID NO: 124 (MF/MS4-3/MS5), SEQ ID NO: 125 (MF/MS3-3/MS5-1), SEQ ID NO: 126 (MF/MS3-3/MS5-2), SEQ ID NO: 127 (MF/MS3-3/MS5-3), SEQ ID NO: 128 (MF/MS3-3/MS5), SEQ ID NO: 129 (MF/MS3-3/MS4-3/MS5-3), SEQ ID NO: 130 (MF/MS3-3/MS4-1/MS5), SEQ ID NO: 131 (MF/MS3-3/MS4-2/MS5), or SEQ ID NO: 132 (MF/MS3-3/MS4-3/MS5).

In some embodiments, exemplary sequences of the engineered tracrRNA, which has one or more modifications at any one or more of the modification sites selected from MS1, MS3, MS4, and MS5, are provided in Table 2.

**[Table 2]**

| **tracrRNA** | **Nucleotide sequence** | **SEQ ID NO** |
|---|---|---|
| MS1 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCUCCUCU CCAAUUCUGCACAA | 87 |
| MS1/MS3-1 | GAUAAAGUGGAGAACCGCUUCACCAAAAGCUGUCCCU UAGGGGAUUAGAACUUGAGUGAAGGUGGGCUGCUUG CAUCAGCCUAAUGUCGAGAAGUGCUUUCUUCGGAAAG | 88 |
| | UAACCCUCGAAACAAAUUCAGUGCUCCUCUCCAAUUC UGCACAA | |
| MS1/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC GAAACAAAUUCAGUGCUCCUCUCCAAUUCUGCACAA | 89 |
| MS1/MS3-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUCUGCACAA | 90 |
| MS1/MS4*-1 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCUCCUCU CCAAUUC | 91 |
| MS 1/MS4*-2 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCUCCUCU C | 92 |
| MS1/MS4*-3 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCU | 93 |
| MS1/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAGUGCUCCUCUCCAAUU CUGCACAA | 94 |
| MS1/MS5-2 | CUUCACUGAUAAAGUGGAGAACCGCUUCACCAAAAGC UUAGGAACUUGAGUGAAGGUGGGCUGCUUGCAUCAG CCUAAUGUCGAGAAGUGCUUUCUUCGGAAAGUAACCC UCGAAACAAAUUCAGUGCUCCUCUCCAAUUCUGCACA A | 95 |
| MS1/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAGUGCUCCUCUCCAAUUCUGCACAA | 96 |
| MS1/MS3-3/MS4*-1 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUC | 97 |
| MS1/MS3-3/MS4*-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUC | 98 |
| MS1/MS3-3/MS4*-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCU | 99 |
| MS1/MS4*-2/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAGUGCUCCUCUC | 100 |
| MS1/MS4*-2/MS5-2 | CUUCACUGAUAAAGUGGAGAACCGCUUCACCAAAAGC UUAGGAACUUGAGUGAAGGUGGGCUGCUUGCAUCAG CCUAAUGUCGAGAAGUGCUUUCUUCGGAAAGUAACCC UCGAAACAAAUUCAGUGCUCCUCUC | 101 |
| MS1/MS4*- 2/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAGUGCUCCUCUC | 102 |
| MS1/MS3- 3/MS5-1 | ACCGCUUCACCAAAAGCUGUUUAGAUUAGAACUUGAG UGAAGGUGGGCUGCUUGCAUCAGCCUAAUGUCGAGAA GUGCUUUCUUCGGAAAGUAACCCUCGAAACAAAUUCA GUGCUCCUCUCCAAUUCUGCACAA | 103 |
| MS1/MS3- 3/MS5-2 | ACCGCUUCACCAAAAGCUUAGGAACUUGAGUGAAGGU GGGCUGCUUGCAUCAGCCUAAUGUCGAGAAGUGCUUU CUUCGGAAAGUAACCCUCGAAACAAAUUCAGUGCUCC UCUCCAAUUCUGCACAA | 104 |
| MS1/MS3- 3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAGUGCUCCUCUCCAAU UCUGCACAA | 105 |
| MS1/MS3- 3/MS4*-2/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAGUGCUCCUCUC | 106 |
| Mature Form(MF) | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAUUU | 107 |
| MF/MS3-1 | GAUAAAGUGGAGAACCGCUUCACCAAAAGCUGUCCCU UAGGGGAUUAGAACUUGAGUGAAGGUGGGCUGCUUG CAUCAGCCUAAUGUCGAGAAGUGCUUUCUUCGGAAAG UAACCCUCGAAACAAAUUCAUUU | 108 |
| MF/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC | 109 |
| | GAAACAAAUUCAUUU | |
| MF/MS3-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAUUU | 110 |
| MF/MS4-1 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAU | 111 |
| MF/MS4-2 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUC | 112 |
| MF/MS4-3 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAA | 113 |
| MF/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAUUU | 114 |
| MF/MS5-2 | CUUCACUGAUAAAGUGGAGAACCGCUUCACCAAAAGC UUUAGAGAACUUGAGUGAAGGUGGGCUGCUUGCAUC AGCCUAAUGUCGAGAAGUGCUUUCUUCGGAAAGUAAC CCUCGAAACAAAUUCAUUU | 115 |
| MF/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAUUU | 116 |
| MF/MS5 | CUUCACUGAUAAAGUGGAGAACCGCUUCACUUAGAGU GAAGGUGGGCUGCUUGCAUCAGCCUAAUGUCGAGAAG UGCUUUCUUCGGAAAGUAACCCUCGAAACAAAUUCAU UU | 117 |
| MF/MS3-3/MS4-1 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAU | 118 |
| MF/MS3-3/MS4-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUC | 119 |
| MF/MS3-3/MS4-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AA | 120 |
| MF/MS4-3/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAA | 121 |
| MF/MS4-3/MS5-2 | CUUCACUGAUAAAGUGGAGAACCGCUUCACCAAAAGC UUUAGAGAACUUGAGUGAAGGUGGGCUGCUUGCAUC AGCCUAAUGUCGAGAAGUGCUUUCUUCGGAAAGUAAC CCUCGAAACAAA | 122 |
| MF/MS4-3/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA A | 123 |
| MF/MS4-3/MS5 | CUUCACUGAUAAAGUGGAGAACCGCUUCACUUAGAGU GAAGGUGGGCUGCUUGCAUCAGCCUAAUGUCGAGAAG | 124 |
| | UGCUUUCUUCGGAAAGUAACCCUCGAAACAAA | |
| MF/MS3-3/MS5-1 | ACCGCUUCACCAAAAGCUGUUUAGAUUAGAACUUGAG UGAAGGUGGGCUGCUUGCAUCAGCCUAAUGUCGAGAA GUGCUUUCUUCGGAAAGUAACCCUCGAAACAAAUUCA UUU | 125 |
| MF/MS3-3/MS5-2 | ACCGCUUCACCAAAAGCUUUAGAGAACUUGAGUGAAG GUGGGCUGCUUGCAUCAGCCUAAUGUCGAGAAGUGCU UUCUUCGGAAAGUAACCCUCGAAACAAAUUCAUUU | 126 |
| MF/MS3-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAUUU | 127 |
| MF/MS3-3/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUCAUUU | 128 |
| MF/MS3-3/MS4-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAA | 129 |
| MF/MS3-3/MS4-1/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUCAU | 130 |
| MF/MS3-3/MS4-2/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUC | 131 |
| MF/MS3-3/MS4-3/MS5 | ACCGCUUCACUUAGAGUGAAGGUGGGCUGCUUGCAUC AGCCUAAUGUCGAGAAGUGCUUUCUUCGGAAAGUAAC CCUCGAAACAAA | 132 |

In addition, as an example of crRNA to which modifications at the plurality of modification sites (MS) as described above have been applied, there is provided an engineered crRNA comprising the nucleotide sequence of any one of SEQ ID NOS: 133 to 148. Specifically, the engineered crRNA of the present disclosure may comprise or consist of the nucleotide of SEQ ID NO: 133 (MS1), SEQ ID NO: 134 (MS1/MS4*-1), SEQ ID NO: 135 (MS1/MS4*-2), SEQ ID NO: 136 (MS1/MS4*-3), SEQ ID NO: 137 (mature form; MF), SEQ ID NO: 138 (MF/MS4-1), SEQ ID NO: 139 (MF/MS4-2), SEQ ID NO: 140 (MF/MS4-3), SEQ ID NO: 141 (MS1/MS2), SEQ ID NO: 142 (MS1/MS2/MS4*-1), SEQ ID NO: 143 (MS1/MS2/MS4*-2), SEQ ID NO: 144 (MS1/MS2/MS4*-3), SEQ ID NO: 145 (MF/MS2), SEQ ID NO: 146 (MF/MS2/MS4-1), SEQ ID NO: 147 (MF/MS2/MS4-2), or SEQ ID NO: 148 (MF/MS2/MS4-3). In some embodiments, exemplary sequences of the engineered crRNA, which has one or more modifications at any one or more modification sites selected from MS1, MS2, and MS4 are provided in Table 3.

**[Table 3]**

| **crRNA** | **Nucleotide sequence** | **SEQ ID NO** |
|---|---|---|
| MS1 | GUUGCAGAACCCGAAUAGAGCAAUGAAGGAAUGCAAC | 133 |
| MS1/MS4*-1 | GAACCCGAAUAGAGCAAUGAAGGAAUGCAAC | 134 |
| MS1/MS4*-2 | GAAUAGAGCAAUGAAGGAAUGCAAC | 135 |
| MS1/MS4*-3 | AGCAAUGAAGGAAUGCAAC | 136 |
| MF | GAAUGAAGGAAUGCAAC | 137 |
| MF/MS4-1 | AUGAAGGAAUGCAAC | 138 |
| MF/MS4-2 | GAAGGAAUGCAAC | 139 |
| MF/MS4-3 | GGAAUGCAAC | 140 |
| MS1/MS2 | GUUGCAGAACCCGAAUAGAGCAAUGAAGGAAUGCAAC NNNNNNNNNNNNNNNNNNNNUUUUAUUUUUU | 141 |
| MS1/MS2/MS4* -1 | GAACCCGAAUAGAGCAAUGAAGGAAUGCAACNNNNN NNNNNNNNNNNNNNNUUUUAUUUUUU | 142 |
| MS1/MS2/MS4* -2 | GAAUAGAGCAAUGAAGGAAUGCAACNNNNNNNNNNN NNNNNNNNNUUUUAUUUUUU | 143 |
| MS1/MS2/MS4* -3 | AGCAAUGAAGGAAUGCAACNNNNNNNNNNNNNNNNN NNNUUUUAUUUUUU | 144 |
| MF/MS2 | GAAUGAAGGAAUGCAACNNNNNNNNNNNNNNNNNNN NUUUUAUUUUUU | 145 |
| MF/MS2/MS4-1 | AUGAAGGAAUGCAACNNNNNNNNNNNNNNNNNNNNU UUUAUUUUU | 146 |
| MF/MS2/MS4-2 | GAAGGAAUGCAACNNNNNNNNNNNNNNNNNNNNNNUUU UAUUUUUU | 147 |
| MF/MS2/MS4-3 | GGAAUGCAACNNNNNNNNNNNNNNNNNNNNUUUUAU UUUUU | 148 |

In Table 3, indication of a guide sequence (spacer) is omitted from all crRNA sequences unless necessary, and the sequence indicated by 'NNNNNNNNNNNNNNNNNNNN' indicates any guide sequence (spacer) that can hybridize with a target sequence in a target gene. The guide sequence may be appropriately designed by those skilled in the art depending on a desired target gene and/or a target sequence in the target gene as described above, and therefore is not limited to a specific sequence of a particular length. In another embodiment, the the engineered gRNA may comprise tracrRNA comprising or consisting of any one nucleotide sequence selected from the group consisting of SEQ ID NOS: 87 to 132; and crRNA comprising or consisting of any one nucleotide sequence selected from the group consisting of SEQ ID NOS: 133 to 148. In another embodiment, when the engineered gRNA of the present disclosure is in the form of a single guide RNA (sgRNA), the engineered sgRNA may sgRNA comprising or consisting of any one nucleotide sequence selected from the group consisting of SEQ ID NOS: 149 to 186.

Specifically, the engineered sgRNA may be sgRNA of SEQ ID NO: 149 comprising a modification at MS1, sgRNA of SEQ ID NO: 150 comprising modifications at MS1/MS2, sgRNA of SEQ ID NO: 151 comprising modifications at MS1/MS2/MS3, sgRNA of SEQ ID NO: 152 comprising modifications at MS2/MS3/MS4, or sgRNA of SEQ ID NO: 153 comprising modifications at MS2/MS3/MS4/MS5.

In another specific example, the engineered sgRNA may be sgRNA comprising or consisting of the nucleotide sequence of SEQ ID NO: 154 (MS1/MS3-1), SEQ ID NO: 155 (MS1/MS3-2), SEQ ID NO: 156 (MS1/MS3-3), SEQ ID NO: 157 (MS1/MS4*-1), SEQ ID NO: 158 (MS1/MS4*-2), SEQ ID NO: 159 (MS1/MS4*-3), SEQ ID NO: 160 (MS1/MS5-1), SEQ ID NO: 161 (MS1/MS5-2), SEQ ID NO: 162 (MS1/MS5-3), SEQ ID NO: 163 (MS1/MS2/MS4*-2), SEQ ID NO: 164 (MS1/MS3-3/MS4*-2), SEQ ID NO: 165 (MS1/MS2/MS5-3), SEQ ID NO: 166 (MS1/MS3-3/MS5-3), SEQ ID NO: 167 (MS1/MS4*-2/MS5-3), SEQ ID NO: 168 (MS1/MS2/MS3-3/MS4*-2), SEQ ID NO: 169 (MS1/MS2/MS3-3/MS5-3), SEQ ID NO: 170 (MS1/MS2/MS4*-2/MS5-3), SEQ ID NO: 171 (MS1/MS3-3/MS4*-2/MS5-3), or SEQ ID NO: 172 (MS1/MS2/MS3-3/MS4*-2/MSS-3).

In addition, the sgRNA may be sgRNA comprising or consisting of the nucleotide sequence of SEQ ID NO: 173, which is a mature form (abbreviated as MF) of sgRNA.

In another embodiment, there is provided an exemplary sgRNA which comprises partial modification of the nucleotide sequence of the MF sgRNA. Specifically, the MF sgRNA may be an sgRNA comprising or consisting of the nucleotide sequence of SEQ ID NO: 174 (MS3-1), SEQ ID NO: 175 (MS3-2), SEQ ID NO: 176 (MS3-3), SEQ ID NO: 177 (MS4-1), SEQ ID NO: 178 (MS4-2), SEQ ID NO: 179 (MS4-3), SEQ ID NO: 180 (MS5-1), SEQ ID NO: 181 (MS5-2), SEQ ID NO: 182 (MS5-3), SEQ ID NO: 183 (MS3-3/MS4-3), SEQ ID NO: 184 (MS3-3/MS5-3), SEQ ID NO: 185 (MS4-3/MS5-3), or SEQ ID NO: 186 (MS3-3/MS4-3/MS5-3).

In a preferred embodiment, the engineered sgRNA may consist of the nucleotide sequence of SEQ ID NO: 151 (Cas12f_ge3.0), SEQ ID NO: 152 (Cas12f_ge4.0), or SEQ ID NO: 153 (Cas12f_ge4.1).

### (7) Additional sequence

The engineered tracrRNA of the present disclosure may optionally further comprise an additional sequence. The additional sequence may be located at the 3'-end of the engineered tracrRNA. In addition, the additional sequence may be located at the 5'-end of the engineered tracrRNA. For example, the additional sequence may be located at the 5'-end of the first stem region.

The additional sequence may consist of 1 to 40 nucleotides. In an embodiment, the additional sequence may be any nucleotide sequence or a randomly arranged nucleotide sequence. For example, the additional sequence may be 5'-AUAAAGGUGA-3' (SEQ ID NO: 187).

In addition, the additional sequence may be a known nucleotide sequence. For example, the additional sequence may be a hammerhead ribozyme nucleotide sequence. Here, the hammerhead ribozyme nucleotide sequence may be 5'-CUGAUGAGUCCGUGAGGACGAAACGAGUAAGCUCGUC-3' (SEQ ID NO: 188) or 5'-CUGCUCGAAUGAGCAAAGCAGGAGUGCCUGAGUAGUC-3' (SEQ ID NO: 189). The sequences listed above are merely examples, and the additional sequence is not limited thereto.

### (8) Chemical modification

In some embodiments, the engineered tracrRNA or engineered crRNA included in the engineered gRNA may have chemical modification in at least one or more nucleotides, if necessary. Here, the chemical modification may be a modification in various covalent bonds that may occur in a nucleotide base and/or sugar portion.

For example, the chemical modification may be methylation, halogenation, acetylation, phosphorylation, phosphorothioate (PS) linkage, locked nucleic acid (LNA), 2'-O-methyl 3'phosphorothioate (MS) or 2'-O-methyl 3'thioPACE (MSP). The above example is a simple example and the modification is not limited thereto.

In a case of using the hypercompact gene editing system comprising a complex of the engineered gRNA with engineered Cas12f1 (CWCas12f1 or UnCas12f1) of the present disclosure, indel efficiency for a target gene or target nucleic acid in a cell is significantly improved compared to a case of using the guide RNA or Cas12f1 found in nature.

Above all, the engineered gRNA may involve optimized length for high efficiency and resulting cost reduction in gRNA synthesis, creation of additional space or capacity in a case of being inserted into a viral vector, normal expression of tracrRNA, increased expression of operable gRNA, increased gRNA stability, increased stability of complex of gRNA with nucleic acid editing protein, induction of formation of complex of gRNA with nucleic acid editing protein at high efficiency, increased cleavage efficiency of target nucleic acid by hypercompact nucleic acid editing system comprising complex of gRNA with nucleic acid editing protein, and increased homology-directed repair efficiency for target nucleic acid caused by such a system. Accordingly, in a case of using the above-described engineered gRNA for Cas12f1 or an engineered Cas12f1 protein, it is possible to overcome the limitations of the above-mentioned prior art, thereby cleaving or editing a gene with high efficiency in a cell.

In addition, the engineered gRNA has a short length compared to gRNA found in nature, and thus has high applicability in the field of gene editing technology. Using the engineered gRNA, the hypercompact gene editing system comprising a complex of the gRNA with nucleic acid editing protein has advantages of being very small in size and having excellent editing efficiency, which allows the system to be utilized in various gene editing technologies.

### 3. Nucleic acid construct encoding each component of Cas12f1 system

Since each component of the gene editing system provided in the present disclosure is intended to be expressed in a cell, according to an embodiment, there is provided a nucleic acid construct encoding each component of the gene editing system. The nucleic acid construct may comprise a synthetic nucleotide sequence.

In an embodiment, the nucleic acid may be DNA or RNA (for example, mRNA). The nucleic acid or polynucleotide encoding each component of the gene editing system are disclosed herein as a representative example, or the nucleotide sequence thereof may be readily determined by those skilled in the art by referring to the specific sequence of each component.

In an embodiment, the nucleic acid construct may comprise a human codon-optimized nucleotide sequence encoding a Cas12f1 protein. The term "codon optimization" refers to a process of modifying a native nucleic acid sequence for enhanced expression in a cell of interest by replacing at least one codon in the native sequence with a codon, which is used more frequently or most frequently in a gene of the target cell, while maintaining its native amino acid sequence. Different species have specific biases for specific codons for specific amino acids, and codon bias (differences in codon usage between organisms) is often correlated with translation efficiency of an mRNA, which is considered to be dependent on the nature of codons being translated and availability of specific tRNA molecules. Predominance of tRNA selected in a cell generally reflects the most frequently used codon in peptide synthesis. Thus, genes may be tailored for optimal gene expression in a given organism based on codon optimization.

For example, the nucleic acid encoding the human codon optimized CWCas12f1 protein or a variant thereof may comprise or consist of a sequence selected from SEQ ID NOS: 6 to 9. In addition, the nucleic acid encoding the human codon optimized UnCas12f1 protein may comprise or consist of the sequence of SEQ ID NO: 10.

In another embodiment, the nucleic acid or polynucleotide may be DNA or RNA that exists in nature, or may be a modified nucleic acid in which a chemical modification has occurred in at least a part of the nucleic acid or polynucleotide. For example, the nucleic acid or polynucleotide may be one in which one or more nucleotides have been chemically modified. Here, the chemical modification may include any modification of nucleic acids known to those skilled in the art.

### IV. Vector or vector system

As disclosed herein, the nucleic acid construct may be a vector or may be contained in a vector. The components of the composition according to an embodiment of the present disclosure may be one nucleic acid construct or two or more nucleic acid constructs. In addition, the nucleic acid construct(s) may be present in one vector or may be present separately in two or more vectors. In an embodiment, when the composition comprises one or more vectors, the composition may be a vector system. Since the vector or vector system allows each component of the above-described Cas12f1 system to be expressed in a cell, the nucleic acid construct (for example, nucleotide sequence) included in the vector system comprises at least one nucleotide sequence encoding each component of an embodiment of the present disclosure. In addition, since the disclosed vector system allows each component of the present disclosure to be expressed in a cell, all effects and advantages that are achieved by the present disclosure are applied as is.

In the disclosed vector system, each nucleic acid construct is capable of expressing each component (for example, an inhibitory molecule, an endonuclease, and first and second guide RNAs) in a cell. The vector system enables removal of a nucleic acid segment to be deleted in a cell.

In the vector system disclosed herein, for the nucleotide sequence of each nucleic acid construct and the components expressed thereby, see the above-described details.

In order to use the composition disclosed herein for deletion of a nucleic acid segment, a method may be used in which one or more vectors comprising nucleotide sequences encoding respective components are introduced directly or through an appropriate delivery means or delivered through a vehicle, such as a virus, into a target cell and the respective components of the gene editing system are allowed to be expressed in the target cell. Preferably, the nucleic acid constructs, whose nucleotide sequences encoding respective components are operably linked, may be contained in a single vector.

In an embodiment, the nucleic acid construct encoding one or more components of the above-described composition may be present in two or more vectors.

In another embodiment, the nucleic acid constructs encoding one or more components of the above-described composition may be present in a single vector (for example, AAV).

In addition, the vector system of the present disclosure may comprise, in addition to the above-described components, a nucleotide sequence encoding an additional expression element that is desired to be expressed as needed by those skilled in the art. For example, the additional expression element may be a tag. Specifically, the additional expression element may be a herbicide resistance gene such as glyphosate, glufosinate ammonium, or phosphinothricin, or an antibiotic resistance gene such as ampicillin, kanamycin, G418, bleomycin, hygromycin, or chloramphenicol.

In another embodiment, the vector or vector system needs to comprise one or more regulatory and/or control components so that it is directly expressed in a cell. Specifically, the regulatory and/or control components may include, but are not limited to, a promoter, an enhancer, an intron, a polyadenylation signal, a Kozak consensus sequence, an internal ribosome entry site (IRES), a splice acceptor, a 2A sequence, and/or a replication origin. The replication origin may be, but is not limited to, an f1 origin of replication, an SV40 origin of replication, a pMB 1 origin of replication, an adeno origin of replication, an AAV origin of replication, and/or a BBV origin of replication.

In another embodiment, in order to express, in a cell, the nucleic acid construct encoding each component in a cell, a promoter sequence may need to be operably linked to the sequence encoding each component so that an RNA transcription factor can be activated in the cell. The promoter sequence may be designed differently depending on the corresponding RNA transcription factor or expression environment, and is not limited as long as it can properly express the components of the composition of the present disclosure in a cell.

For example, the promoter sequence may be a promoter that promotes transcription of RNA polymerase RNA Pol I, Pol II, or Pol III. Specifically, the promoter may be one of U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

In another embodiment, when a sequence of the vector comprises the promoter sequence, transcription of a sequence operably linked to the promoter is induced by an RNA transcription factor, and the vector may comprise a termination signal that induces termination of transcription of the RNA transcription factor. The termination signal may vary depending on the type of the promoter sequence. Specifically, when the promoter is a U6 or H1 promoter, the promoter recognizes a TTTTT (T5) or TTTTTT (T6) sequence, which is a thymidine (T) repeat sequence, as a termination signal.

The sequence of the engineered guide RNA according to an embodiment may comprise a U-rich tail sequence at its 3'-end. Accordingly, the sequence encoding the engineered guide RNA comprises a T-rich sequence corresponding to the U-rich tail sequence at its 3'-end. As described above, some promoter sequences recognize a thymidine (T) repeat sequence, for example, a sequence consisting of five or more consecutive thymidine (T) residues, as a termination signal, and therefore, in some cases, the T-rich sequence may be recognized as a termination signal. In other words, when the vector sequence provided herein comprises a sequence encoding the engineered guide RNA, a sequence encoding the U-rich tail sequence included in the engineered gRNA sequence may be used as a termination signal.

In an embodiment, when the vector sequence comprises a U6 or H1 promoter sequence and a sequence encoding the engineered guide RNA operably linked thereto, a sequence portion that encodes the U-rich tail sequence included in the guide RNA sequence may be recognized as a termination signal. Specifically, the U-rich tail sequence may comprise a sequence consisting of five or more consecutive uridine (U) residues.

In an embodiment, the vector may be a viral vector. Specifically, the viral vector may be at least one selected from the group consisting of a retrovirus vector, a lentivirus vector, an adenovirus vector, an adeno-associated virus vector, a vaccinia virus vector, a poxvirus vector, a herpes simplex virus vector, and a phagemid vector. Preferably, the viral vector may be an adeno-associated viral vector (AAV). In addition, the viral vector includes, but is not limited to, a SIN lentivirus vector, a retrovirus vector, a foamy virus vector, an adenovirus vector, an adeno-associated virus (AAV) vector, a hybrid vector and/or a plasmid transposon (for example, the Sleeping Beauty transposon system), or an integrase-based vector system.

In another embodiment, the vector may be a non-viral vector. Specifically, the non-viral vector may be at least one selected from the group consisting of, but not limited to, plasmid, naked DNA, DNA complex, mRNA (transcript), and amplicon. For example, the plasmid may be selected from the group consisting of pcDNA series, pSC101, pGV1106, pACYC177, ColE1, pKT230, pME290, pBR322, pUC8/9, pUC6, pBD9, pHC79, pU61, pLAFR1, pHV14, pGEX series, pET series, and pUC19.

The term "naked DNA" refers to DNA (for example, histone-free DNA) that encodes a protein, such as Cas12f1 of the present disclosure, cloned into a suitable expression vector (for example, plasmid) in an appropriate orientation for expression.

The term "amplicon," when used with respect to a nucleic acid, means a product of copying the nucleic acid, wherein the product has a nucleotide sequence that is identical with or complementary to at least a portion of the nucleotide sequence of the nucleic acid. For example, an amplicon may be produced by any of a variety of amplification methods that use a nucleic acid or an amplicon thereof as a template, including polymerase extension, polymerase chain reaction (PCR), rolling circle amplification (RCA), multi-displacement amplification (MDA), ligation extension, or ligation chain reaction. The amplicon may be a nucleic acid molecule having a single copy of a particular nucleotide sequence (for example, a PCR product) or multiple copies of the nucleotide sequence (for example, a concatemeric product of RCA).

The vector disclosed herein may be designed in the form of a linear or circular vector. In a case where the vector is a linear vector, RNA transcription is terminated at the 3'-end even if a sequence of the linear vector does not separately comprise a termination signal. However, in a case where the vector is a circular vector, RNA transcription is not terminated unless a sequence of the circular vector separately comprises a termination signal. Therefore, when using a circular vector, a termination signal corresponding to a transcription factor related to each promoter sequence has to be included in order for the vector to express an intended target.

In an embodiment, the viral vector or non-viral vector may be delivered by a delivery system such as liposomes, polymeric nanoparticles (for example, lipid nanoparticles), oil-in-water nanoemulsions, or combinations thereof, or in the form of a virus.

In another embodiment, the virus may be selected from the group consisting of a retrovirus, a lentivirus, an adenovirus, an adeno-associated virus, a vaccinia virus, a poxvirus, a herpes simplex virus and a phage.

In yet another embodiment, the phage may be selected from the group consisting of λgt4λB, λ-Charon, λΔz1, and M13.

In order to efficiently deliver the nucleic acid construct included in the composition of the present disclosure into a target cell or target site via a virus, in particular, an adeno-associated virus (AAV), it is important to design a size of the nucleotide sequence encoding all components of the editing system to be within 4.7 kb that is a packaging limit of AAV This has an advantage in that in a case where the Cas12f1 system of the present disclosure is used, a very small size of the hypercompact nucleic acid editing protein and two engineered gRNAs included in the system allows sufficient packaging by AAV even if an additional regulatory molecule (for example, a nucleic acid construct that expresses the inhibitory molecule of the present disclosure) is further included.

### V. Formulation

As disclosed herein, the composition may be a pharmaceutical composition.

In an embodiment, the pharmaceutical composition may be for deletion of a nucleic acid segment. In addition, the pharmaceutical composition may be for treating or delaying onset or progression of a genetic disease caused by an undesirable mutation.

In an embodiment, the pharmaceutical composition may be formulated according to the mode of administration to be used. For example, in a case where the pharmaceutical composition is an injectable pharmaceutical composition, it may be desirable to use an isotonic agent. An additive for isotonicity may generally include sodium chloride, dextrose, mannitol, sorbitol, and lactose. In an embodiment, isotonic solutions such as phosphate buffered saline are preferred. A stabilizer may include gelatin and albumin. In an embodiment, a vasoconstrictor is added to the formulation.

In another embodiment, the composition may further comprise a pharmaceutically acceptable excipient. The pharmaceutically acceptable excipient may be a functional molecule that acts as a vehicle, an adjuvant, a carrier, or a diluent. The pharmaceutically acceptable excipient may be a gene transfer enhancer (which may include a surfactant) such as an immune stimulating complex (ISCOMS), Freund's incomplete adjuvant, a LPS analogue (including monophosphoryl lipid A), a muramyl peptide, a quinone analogue, a vesicle such as squalene and squalane, hyaluronic acid, a lipid, a liposome, a calcium ion, a viral protein, a polyanion, a polycation, or a nanoparticle, or other known gene transfer facilitating agent.

In another embodiment, the composition may comprise a gene transfer enhancer. The gene transfer enhancer may be a polyanion, a polycation (including poly-L-glutamic acid (LGS)), or a lipid. The gene transfer enhancer is poly-L-glutamic acid, and more preferably, the poly-L-glutamic acid may be present in the composition for genome editing of skeletal muscle or cardiac muscle at a concentration of less than 6 mg/ml. The gene transfer enhancer may also include a surfactant, such as an immune stimulating complex (ISCOMS), Freund's incomplete adjuvant, a LPS analogue (including monophosphoryl lipid A), a muramyl peptide, a quinone analogue and a vesicle, such as squalene and squalane; and hyaluronic acid may also be used.

In an embodiment, the composition comprising one or more vectors included in the above-described vector system may comprise a gene transfer enhancer, such as a lipid, a liposome (including lecithin liposomes, or other liposomes known in the art), a DNA-liposome mixture, a calcium ion, a viral protein, a polyanion, a polycation, or a nanoparticle, or other known gene transfer enhancer. Preferably, the gene transfer enhancer is a polyanion, a polycation (for example, poly-L-glutamic acid (LGS)), or lipid 17.

An actual dosage of the (pharmaceutical) composition may vary greatly depending on various factors, such as the choice of vector, the target cell, organism, or tissue, the condition of the subject to be treated, the degree of transformation/modification sought, the route of administration, the method of administration, the form of transformation/modification sought, and the like. The administration may be performed by a route of administration selected from subretinal administration, subcutaneous administration, intradermal administration, intraocular administration, intravitreal administration, intratumoral administration, intranodal administration, intramedullary administration, intramuscular administration, intravenous administration, intralymphatic administration, and intraperitoneal administration. The pharmaceutical composition may further comprise a carrier (water, saline, ethanol, glycerol, lactose, sucrose, calcium phosphate, gelatin, dextran, agar, pectin, peanut oil, sesame oil, and the like), a diluent, a pharmaceutically acceptable carrier (for example, phosphate buffered saline), a pharmaceutically acceptable excipient, and/or other compounds known in the art.

For example, delivery for treatment of a disease may be via AAV A therapeutically effective dosage for *in vivo* delivery of AAV to a human may be a saline solution in a range of about 20 ml to about 50 ml containing about 1×10¹⁰ to about 1×10¹⁰⁰ AAV per ml of solution. The dosage may be adjusted to balance the therapeutic benefit against any adverse effects.

### Form for carrying out the disclosure

Hereinafter, the present disclosure will be described in more detail by the following examples. However, these examples are only intended to illustrate the present disclosure, and the scope of the present disclosure is not limited to these examples.

### Example 1. Experimental method

### 1. Preparation of genomic DNA (gDNA)

AC16 or HEK293T cells were transfected and harvested. Genomic DNA was prepared using a gDNA prep kit (Maxwell^{®} RSC Cultured Cells DNA, PROMEGA, AS1620). Samples were prepared according to the manufacturer's instructions.

### 2. PCR and gel purification

This experiment was performed using the GEL & PCR Purification System (GP104-200, Biofact). To the PCR product was added UB buffer in an amount equivalent to 3 times the volume of the PCR product and thorough mixing was performed. Then, isopropanol was added thereto in an amount equivalent to 2 times the volume of the PCR product and thorough mixing was performed. In a case of the gel, the gel of the corresponding band was cut and weighed. Then, UB buffer was added thereto in an amount equivalent to 3 times the weight of the gel. The gel was dissolved by incubation at 65°C for 10 minutes, and then isopropanol was added thereto in an amount equivalent to 1 time the gel volume and thorough mixing was performed. The column was prepared, 200 µl of HelpB buffer was added to the column, centrifugation was performed at 13,000 rpm for 30 seconds. Then, the filtered solution was discarded. The reaction solution was added to the column, centrifugation was performed at 7,000 rpm for 1 minute. Then, the filtered solution was discarded. 750 µl of 80% EtOH was added thereto, centrifugation was performed at 13,000 rpm for 30 seconds. Then, the filtered solution was discarded. After repeating the process twice, centrifugation was performed at 13,000 rpm for 3 minutes. The centrifuged column was placed in a 1.5 ml tube, 30 µl of EB buffer was added dropwise to the center, and the reaction was allowed to occur at room temperature for 1 minute. Centrifugation was performed at 13,000 rpm for 1 minute. The DNA collected in the 1.5 ml tube was quantified and stored at 4°C.

### 3. Collection of plasmid vector

For transfection or Sanger sequencing, the vector-transformed DH5α was used. Plasmid Mini prep kit (PM105-200, Biofact) was used according to the manufacturer's instructions. The culture medium of the vector-transformed DH5α was placed in a 1.5 ml tube, and centrifugation was performed at 13,000 rpm for 5 minutes. After centrifugation, the supernatant was discarded, and the pellet was sufficiently dispersed by vortexing. 350 µl of B1 buffer was added thereto, and the tube was shaken to ensure sufficient reaction. Next, 350 µl of A1 buffer containing RNase A was added thereto, and the tube was inverted until the blue color disappeared. Then, centrifugation was performed at 13,000 rpm for 5 minutes. The column was prepared, 200 µl of HelpB buffer was added thereto, and the solution was centrifuged at 13,000 rpm for 30 seconds. Then, the filtered solution was discarded. 750 µl of the centrifuged supernatant was added to the prepared column, centrifugation was performed at 7,000 rpm for 1 minute, and the filtered solution was discarded. 750 µl of 80% EtOH was added thereto, centrifugation was performed at 13,000 rpm for 30 seconds, and the filtered solution was discarded. This process was repeated twice. After repeating the process twice, centrifugation was performed at 13,000 rpm for 3 minutes. The centrifuged column was placed into a 1.5 ml tube, 30 µl of EB buffer was added dropwise to the center, and then the reaction was allowed to occur at room temperature for 1 minute. Centrifugation was performed at 13,000 rpm for 1 minute, and the plasmid vectors collected in the 1.5 ml tube were quantified and stored at -20°C.

### 4. Preparation of DNA cassette

To confirm indel efficiency of the spacer sequences of Cas12f1, a cassette containing the U6 promoter, scaffold sequence, guide sequence, and U-rich tail sequence (T₄AT₆) was amplified by PCR and used. The process was performed as follows.

### 1) Selection of spacer and order of oligo

The spacer was selected from the 20mer sequence followed by TTTA or TTTG, which are PAM, and spacers whose sequences end with T were excluded. In addition, to minimize off-target effects, the spacers were designed using CRISPR RGEN TOOL by classifying them with less than 2 mismatches. In addition, the reverse complement sequence comprising a DR (direct repeat) and U-rich sequence was custom-made to be used as an R primer.

### 2) PCR

The PCR was performed under the composition and condition shown in Table 4 below.

**[Table 4]**

| Reagent composition | | PCR condition | |
|---|---|---|---|
| 2x pfu PCR Master mix | 205 *µ*ℓ | Pre-denaturation | 95°C, 5 min |
| hU6 F primer (10 P) | 2.05 *µ*ℓ | Denaturation (D) | 95°C, 30 s |
| Target oligo (10 P) | 2.05 *µ*ℓ | Annealing | 60°C, 30 s |
| Template | 1 *µ*ℓ (200 ng) | Extension (E) | 72°C, 2 min |
| DW | 199.9 *µ*ℓ | D-E Cycle | 30 cycles |
| Total | 410 *µ*ℓ | Final extension | 72°C, 3 min |
| Prepared in 8 PCR tubes, each containing 50 *µ*ℓ | | Storage | 4°C, ∞ |

400 µl of the mixture was added to 8 PCR tubes, each containing 50 µl, and each sample was amplified.

### 3) Gel analysis

1% agarose gel was prepared, and the size marker and PCR products were added to the gel. Electrophoresis was performed to confirm the amplified size.

### 4) Purification and Quantification

After confirming the amplified size, the gel was purified according to Experimental Method 2 to quantify the PCR products.

### ] 5. Cell culture

For use in the experiments, AC16 and HEK 293T cells were used as human cells, and Hepa-1c17 cells were used as mouse cells. At 37°C in a 5% CO₂ incubator, AC16 and HEK 293T cells were maintained in DMEM medium supplemented with 10% fetal bovine serum (FBS) and 1% penicillin-streptomycin, and Hepa-1c17 cells were maintained in Alpha minimal medium supplemented with 10% fetal bovine serum (FBS) and 1% penicillin-streptomycin. The attached cells were washed twice with cold PBS and then immediately stored at -80°C for RNA or protein extraction.

### 6. Transfection

The day before transfection, HEK293 and AC16 cells (80% confluency) cultured in 100 mm dishes were treated with trypsin to detach from the bottom of the dish. The detached cells were placed in 50 ml of each pre-warmed medium and slowly dissolved with a pipette. 24-well plates were prepared according to the number of samples and repetitions, and 500 µl of cell suspension medium was added to each well (1/100 dilution). Then, incubation was performed overnight in a CO₂ incubator at 37°C until transfection.

When the cell confluency reached approximately 70% to 80%, 200 µl of the 500 µl medium per well was removed and the plates were placed in the incubator. 1.5 ml tubes were prepared according to the number of samples, and 200 µl of Opti-MEM was added to each tube. 1.5 µg of Cas12f1 and 0.5 µg of gRNA were added to the tube containing Opti-MEM, and vortexed for 5 seconds (nucleic acid mixture). Then, the nucleic acid mixture and FuGENE HD were added at a ratio of 1:3, and reaction was allowed to occur at room temperature for 20 minutes (that is, in a case where the nucleic acid mixture was 2 µg, 6 µl of FuGENE HD was administered). The 24-well plate was taken out from the incubator, and 200 µl of the solution containing the nucleic acid mixture and FuGENE HD was gently added along the well wall. After shaking the plate sufficiently in an S shape, it was incubated in a CO₂ incubator at 37°C for 72 hours. After 72 hours, the cells were harvested and gDNA was extracted therefrom according to Experimental Method 1.

### 7. Construction of vector

The following procedure was performed using the Cas12f1 ge4.0 dual gRNA vector (see Korean Patent Application Nos. 10-2021-0051552 and 10-2022-0043768). The restriction enzyme ends of the vector to be cloned were confirmed, and dual gRNA oligos were designed and custom-made. The custom-made oligos were diluted to a concentration of 100 pmol. 4.5 µl each of the diluted forward and reverse primers was taken and placed into a PCR tube, and then 1 µl of 10X annealing buffer was added thereto to adjust the total volume to 10 µl. Then, annealing was performed under the conditions of 95 °C for 5 minutes and -1 °C/min from 95 °C to 4 °C. The Cas12f1 ge4.0 dual gRNA vector was prepared and incubated at 500 rpm, 37°C for 2 hours under the digestion conditions in Table 5 below.

**[Table 5]**

| Reagent | Volume |
|---|---|
| NEB 10X 2.1 buffer | 5 *µ*ℓ |
| Vector | 10 *µ*g |
| BbsI | 10 *µ*ℓ |
| DW | Amount to make total volume of 50 *µ*ℓ |
| Total | 50 *µ*ℓ |

After digestion, the digested vector was obtained through electrophoresis and gel elution. Ligation was performed using the digested vector and annealed oligo (see Table 6).

**[Table 6]**

| Reagent | Volume |
|---|---|
| DNA ligation mix (TAKARA) | 2 *µ*ℓ |
| Annealed oligo | 1.5 *µ*g |
| Vector digested with BbsI | 0.5 *µ*ℓ |
| Total | 4 *µ*ℓ |

After ligation, transformation was performed on DH5α. After incubation on an LB plate, positive colonies were confirmed through colony PCR and then incubated in 3 ml LB medium. After miniprep, sequencing was performed to confirm whether the final sequences matched.

### 8. DH5α transformation

The previously-produced vector was transformed into *E. coli* to produce the vector. DH5α competent cells were taken out and thawed on ice. The ligated vector was added up to 1/10 of the amount of DH5α, and the incubation was performed on ice for 30 minutes. After heat shock at 42°C for 30 seconds, cooling was performed on ice for 2 minutes. Incubation was performed using 100 µl of LB medium or S.O.C medium at 37°C for 1 hour. The cells were spread on LB plates warmed to room temperature (containing ampicillin or kanamycin depending on the vector) and incubated at 37°C for 14 to 16 hours.

### 9. PCR of NGS sample

NGS samples for confirming indel efficiency for a target were prepared by purification of the PCR product after performing the 1st to 3rd PCRs. The preparation of NGS samples for confirming indel efficiency was conducted over a total of 3 PCRs. The first PCR produced a band of approximately 450 to 500 bp, and the second PCR was performed using this PCR product as a template. After the 2nd PCR, the sample was loaded onto a 2% agarose gel to confirm whether the band was properly displayed within 250 bp. If the band was not properly displayed, the cause was determined. Then, the process was restarted from the 1st PCR. If the correct band was confirmed, the 3rd PCR was performed using the 2nd PCR product as a template. Here, if the concentration of the 2nd PCR product was high, DW was added to adjust the concentration. After completing the 3rd PCR, the sample was loaded onto a 2% agarose gel to identify the bands. The completed PCR products were pooled in equal amounts (5 µl each) and then subjected to PCR purification.

This experiment was performed using the GEL & PCR Purification System (GP104-200, Biofact). UB buffer was added to the PCR product in an amount equivalent to 5 times the volume of the PCR product and thorough mixing was performed. The column was prepared, 200 µl of HelpB buffer was added to the column, centrifugation was performed at 13,000 rpm for 30 seconds, and then the filtered solution was discarded. The reaction solution was added to the column, centrifugation was performed at 7,000 rpm for 1 minute, and then the filtered solution was discarded. 750 µl of 80% EtOH was added thereto, centrifugation was performed at 13,000 rpm for 30 seconds, and then the filtered solution was discarded. After repeating the process twice, centrifugation was performed at 13,000 rpm for 3 minutes. The centrifuged column was placed in a 1.5 ml tube, 100 µl of EB buffer was added dropwise to the center, and the reaction was allowed to occur at room temperature for 1 minute. Centrifugation was performed at 13,000 rpm for 1 minute. The DNA collected in the 1.5 ml tube was quantified to obtain a concentration of 15 ng/µl and stored at 4°C until NGS analysis.

**[Table 7]**

| Reagent composition | | PCR condition | |
|---|---|---|---|
| KAPA HiFi PCR mix | 5 *µ*ℓ | Pre-denaturation | 95°C, 3 min |
| Forward primer (10 pmol/ul) | 0.5 *µ*ℓ | Denaturation (D) | 98°C, 20 s |
| Reverse primer (10 pmol/ul) | 0.5 *µ*ℓ | Annealing (A) | 60°C, 15 s |
| Template (gDNA) | 1 *µ*ℓ | Extension (E) | 72°C, 2 min |
| DW | 3 *µ*ℓ | D-E Cycle | 30 cycles |
| Total | 10 *µ*ℓ | Final extension | 72°C, 3 min |
| | | Storage | 4°C, ∞ |

| | | | |
|---|---|---|---|
| 10. Preparation of cell extracts and Western blot analysis | | | |

Cell extracts were prepared by scraping cells with 60 to 80 µl of RIPA Lysis and Extraction Buffer (THERMOFISHER, 89900) containing protease inhibitor cocktail (ROCHE, 11836153001) at 4°C and kept on ice for 10 minutes. The concentration of the cleared lysate was measured by Bradford assay (BIO-RAD), in which 20 to 65 µg of protein lysate, depending on the target protein, was loaded onto Mini-PROTEAN^{®} TGX^{™} Precast Protein Gels (BIO-RAD), electrophoresed, and transferred to PVDF membranes using the Trans-Blot Turbo Transfer System (BIO-RAD). The membrane was incubated at room temperature for 1 hour in a blocking solution containing 5% non-fat dry milk in TBS-T and then incubated at 4°C overnight with primary antibodies in TBS-T. After incubation with primary antibodies, the membrane was washed four times with TBS-T and then incubated at room temperature for 1 hour in a blocking solution containing anti-rabbit or anti-mouse immunoglobulin conjugated with HRP. The membrane was additionally washed four times with TBS-T and specific protein complexes were visualized with ECL prime (CYTIVA). Antibodies used were against KU70 (CST, 4588S), DNAligase IV (CST, 14649S), XLF (CST, 2854S), Artemis (CST, 13381), GAPDH (CST, 2118L), and XRCC4 (THERMO, MA5-24383).

### 11. Quantitative real-time PCR (qRT-PCR)

Purification of total RNA was performed, and reverse transcription of total 1 µg RNA was performed with Superscript IV reverse transcriptase (INVITROGEN) using random hexamers. For real-time PCR, 1/20 of the RT product was amplified with the KAPA SYBR FAST qPCR kit (KAPA BIOSYSTEM, Wilmington, MA, USA) using the real-time cycler Quantstudio (THERMO FISHER SCIENTIFIC). The relative amount of mRNA transcripts was measured by the 2ΔΔCT method. GAPDH and 18s rRNA were used as endogenous controls. The primer sequences used for qRT-PCR are shown in Table 8 below.

**[Table 8]**

| Primer name | Sequence (5'-3') | SEQ ID NO |
|---|---|---|
| hATM-F | GGACAGTGGAGGCACAAAAT | 451 |
| hATM-R | GTGTCGAAGACAGCTGGTGA | 452 |
| hKU70-F | CGGGAAACAAATGAACCAGT | 453 |
| hKU70-R | TGAAACCCATGAGCATCAAA | 454 |
| hLIG4-F | CACCTTGCGTTTTCCACGAA | 455 |
| hLIG4-R | CAGATGCCTTCCCCCTAAGTTG | 456 |
| hXRCC4-F | CTGATGGTCATTCAGCATGG | 457 |
| hXRCC4-R | TCCTGCTCCTGACAACAATG | 458 |
| hXLF-F | TCTCTGGCCTCCCCTTCTAT | 459 |
| hXLF-R | TAGCTCCCTCACTTGGCACT | 460 |
| hARTEMIS-F | AATTCCAAGTCGGGAGGAGT | 461 |
| hARTEMIS-R | GGATCTGAGTGTTGCGGTCT | 462 |
| GAPDH-F | GGAAGGACTCATGACCACAGT | 463 |
| GAPDH-R | CAGTGAGCTTCCCGTTCAG | 464 |
| 18s_rRNA-F | TCAACTTTCGATGGTAGTCGCC | 465 |
| 18s_rRNA-R | GGCCTCGAAAGAGTCCTGTATTGT | 466 |
| mDdrelc-F | GCTTCGGGTGAGAAGGAAG | 467 |
| mDclrelc-R | TGCCAGTCTGAAGTCTCCTG | 468 |

### 12. shRNA cloning

shRNA sequences for the factors involved in the NHEJ repair pathway used in this experiment were shown in Table 9 below. To clone shRNA, annealing was performed for the target oligos for each shRNA. The annealed insert was inserted into a plasmid together with a U6 or H1 promoter.

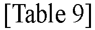

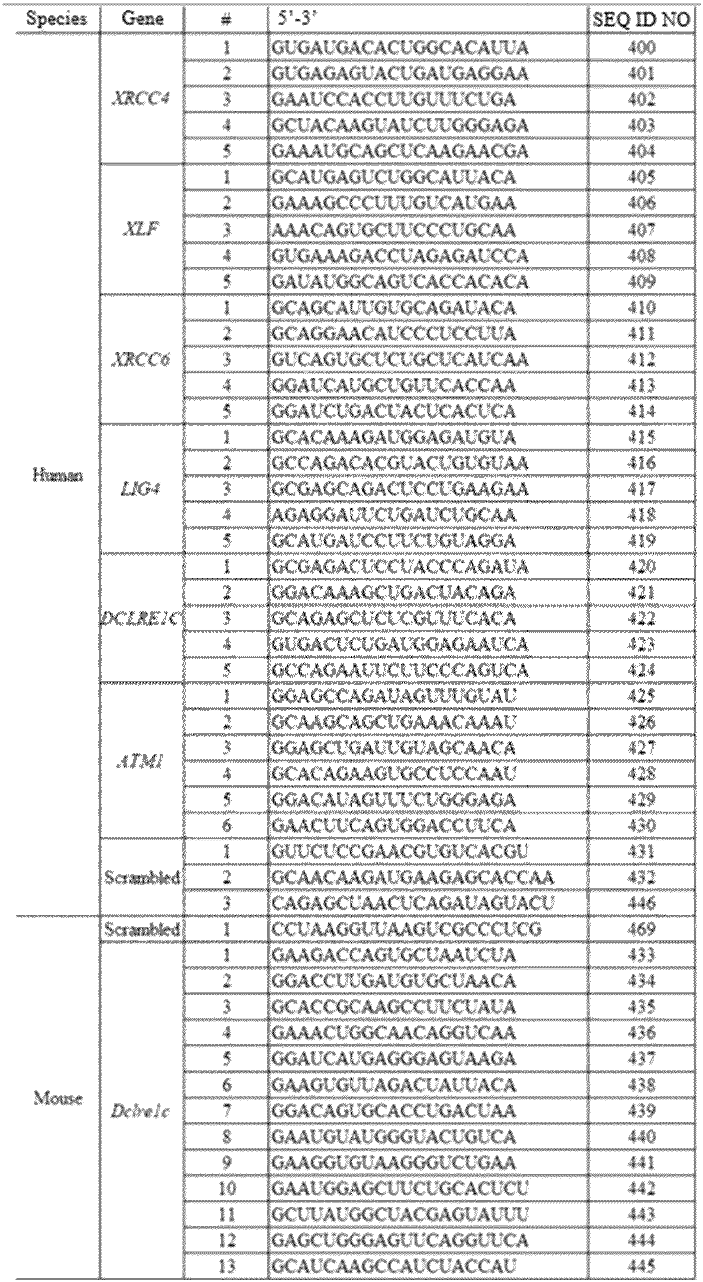

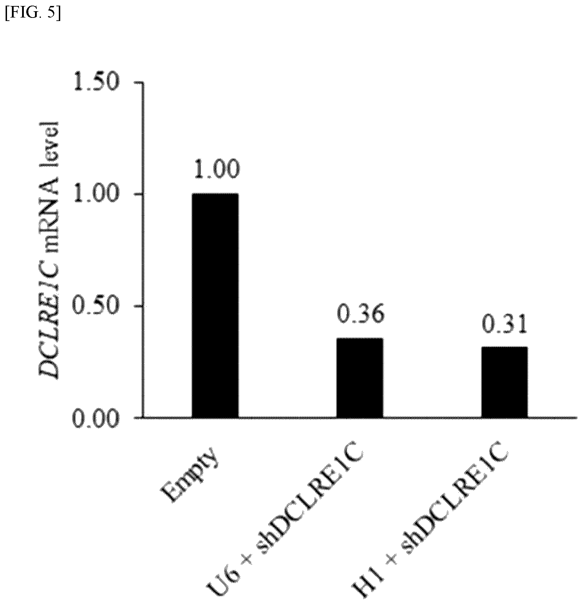

The DCLRE1C mRNA levels are shown in FIG. 5 when the U1 and H1 promoters were used together with shRNA for DCLRE1C. From the results, it can be seen that each of the U1 and H1 promoters is a suitable promoter for use with the shRNA of the present disclosure.

### 13. Quantification of large-scale deletion by qPCR (dystrophin exon 51, E51)

Quantitative real-time PCR was used to quantify genomic DNA. Primer sequences were designed for each of the E51 internal regions where large-scale deletion occurs. For qPCR, 20 ng of gDNA was amplified with the KAPA SYBR FAST qPCR kit (KAPA BIOSYSTEM, Wilmington, MA, USA) using the real-time cycler Quantstudio (THERMO FISHER SCIENTIFIC). The primer sequences used for qPCR are shown in Table 10 below.

**[Table 10]**

| Primer name | Sequence (5'-3') | SEQ ID NO |
|---|---|---|
| E51_LD_F | TGTCATGAATAAGAGTTTGGCTCA | 447 |
| E51_LD_R | AGGTTGTGTCACCAGAGTAACA | 448 |
| 18s_rRNA-F | TCAACTTTCGATGGTAGTCGCC | 449 |
| 18s_rRNA-R | GGCCTCGAAAGAGTCCTGTATTGT | 450 |

### 14. Whole genome sequencing (WGS) analysis (E51)

HEK293T cells were transfected with UnCas12f1, a pair of gRNA (F142/R52), and shRNA, and then harvested 5 days after transfection. Genomic DNA was purified using a gDNA prep kit (Maxwell^{®} RSC Cultured Cells DNA, PROMEGA, AS 1620). 400 to 500 ng of genomic DNA was fragmented, and libraries were prepared using the MGIEasy FS DNA Library Prep. Kit (MGI Tech) according to the manufacturer's instructions. The libraries were then subj ected to WGS using DNB SEQ-T7 (MGI Tech). Sequence reads were mapped to the human genome reference standard, GRCh38. Large-scale deletion efficiency was analyzed by counting reads mapped to the deleted region between a pair of gRNAs, and counts per million (CPM) was calculated. The percentage of deletion was calculated based on the difference in CPM between the sample and the control.

### Example 2. Construction of nucleic acid editing system for deletion of target gene (dystrophin exon 51)

### Example 2.1. Production of engineered gRNA

The most common type among patients with Duchenne muscular dystrophy (DMD) is a type in which a stop codon occurs in dystrophin exon 51. As shown in FIG. 2, deletion of exons 49 and 50 leads to production of a stop codon in exon 51, which serves as a signal to step protein synthesis, thereby preventing production of dystrophin protein. Here, deletion of exon 51 prevents production of the stop codon, thereby allowing for production of a dystrophin protein that is shorter than normal but has normal function.

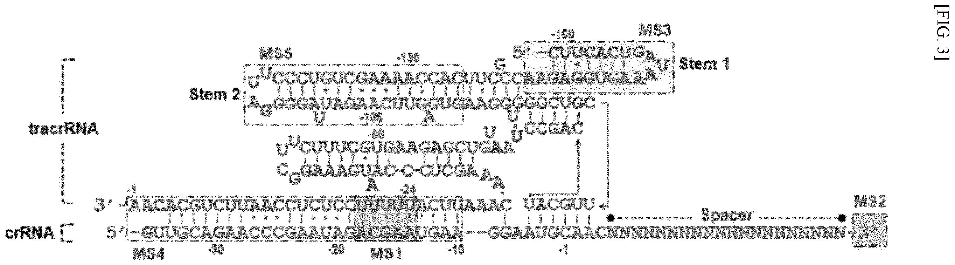

The CRISPR/Cas12f1 system and the TaRGET system were constructed for deletion of dystrophin exon 51. In the systems, for the gRNAs having a guide sequence that hybridizes with a target sequence for deletion of exon 51, engineered gRNAs having at least one of the five modification sites (MS1, MS2, MS3, MS4, and MS5) as shown in FIG. 3 were produced, and the specific sequences thereof are shown in Table 11.

**[Table 11]**

| gRNA | Sequence (5' to 3') | SEQ ID NO |
|---|---|---|
| Canonical sgRNA | CUUCACUGAUAAAGUGGAGAACCGCUUCACCAAAAGCUG UCCCuuagGGGAUUAGAACUUGAGUGAAGGUGGGCUGCUU GCAUCAGCCUAAUGUCGAGAAGUGCUUUCUUCGGAAAG UAACCCUCGAAACAAAUUCAUUUUUCCUCUCCAAUUCUG CACAAgaaaGUUGCAGAACCCGAAUAGacgaaUGAAGGAAUG CAACNNNNNNNNNNNNNNNNNNNN | 13 |
| MS1 | CUUCACUGAUAAAGUGGAGAACCGCUUCACCAAAAGCUG UCCCUUAGGGGAUUAGAACUUGAGUGAAGGUGGGCUGC UUGCAUCAGCCUAAUGUCGAGAAGUGCUUUCUUCGGAA AGUAACCCUCGAAACAAAUUCAGUGCUCCUCUCCAAUUC UGCACAAgaaaGUUGCAGAACCCGAAUAGAGCAAUGAAGG AAUGCAACNNNNNNNNNNNNNNNNNNNN | 149 |
| MS1/MS2 | CUUCACUGAUAAAGUGGAGAACCGCUUCACCAAAAGCUG UCCCUUAGGGGAUUAGAACUUGAGUGAAGGUGGGCUGC UUGCAUCAGCCUAAUGUCGAGAAGUGCUUUCUUCGGAA AGUAACCCUCGAAACAAAUUCAGUGCUCCUCUCCAAUUC UGCACAAgaaaGUUGCAGAACCCGAAUAGAGCAAUGAAGG AAUGCAACNNNNNNNNNNNNNNNNNNNNUUUUAUUUU U | 150 |
| MS1/MS2/MS (ge3.0) | ACCGCUUCACCAAAAGCUGUCCCUUAGGGGAUUAGAACU UGAGUGAAGGUGGGCUGCUUGCAUCAGCCUAAUGUCGA GAAGUGCUUUCUUCGGAAAGUAACCCUCGAAACAAAUUC AGUGCUCCUCUCCAAUUCUGCACAAgaaaGUUGCAGAACC CGAAUAGAGCAAUGAAGGAAUGCAACNNNNNNNNNNNN NNNNNNNNUUUUAUUUUUU | 151 |
| MS2/MS3/MS (ge4.0) | ACCGCUUCACCAAAAGCUGUCCCUUAGGGGAUUAGAACU UGAGUGAAGGUGGGCUGCUUGCAUCAGCCUAAUGUCGA GAAGUGCUUUCUUCGGAAAGUAACCCUCGAAACAAAgaaa | 152 |
| | GGAAUGCAACNNNNNNNNNNNNNNUUUUAUUU UUU | |
| MS2/MS3/MS4 /MS5 (ge4.1) | ACCGCUUCACUUAGAGUGAAGGUGGGCUGCUUGCAUCAG CCUAAUGUCGAGAAGUGCUUUCUUCGGAAAGUAACCCUC GAAACAAAgaaaGGAAUGCAACNNNNNNNNNNNNNNNNNN NNUUUUAUUUUUU | 153 |
| MS1/MS3-1 | GAUAAAGUGGAGAACCGCUUCACCAAAAGCUGUCCCUUA GGGGAUUAGAACUUGAGUGAAGGUGGGCUGCUUGCAUC AGCCUAAUGUCGAGAAGUGCUUUCUUCGGAAAGUAACCC UCGAAACAAAUUCAGUGCUCCUCUCCAAUUCUGCACAAg aaaGUUGCAGAACCCGAAUAGAGCAAUGAAGGAAUGCAAC NNNNNNNNNNNNNNNNNNNN | 154 |
| MS1/MS3-2 | UGGAGAACCGCUUCACCAAAAGCUGUCCCUUAGGGGAUU AGAACUUGAGUGAAGGUGGGCUGCUUGCAUCAGCCUAA UGUCGAGAAGUGCUUUCUUCGGAAAGUAACCCUCGAAAC AAAUUCAGUGCUCCUCUCCAAUUCUGCACAAgaaaGUUGC AGAACCCGAAUAGAGCAAUGAAGGAAUGCAACNNNNNN NNNNNNNNNNNNNN | 155 |
| MS1/MS3-3 | ACCGCUUCACCAAAAGCUGUCCCUUAGGGGAUUAGAACU UGAGUGAAGGUGGGCUGCUUGCAUCAGCCUAAUGUCGA GAAGUGCUUUCUUCGGAAAGUAACCCUCGAAACAAAUUC AGUGCUCCUCUCCAAUUCUGCACAAgaaaGUUGCAGAACC CGAAUAGAGCAAUGAAGGAAUGCAACNNNNNNNNNNNN NNNNNNNN | 156 |
| MS1/MS4*-1 | CUUCACUGAUAAAGUGGAGAACCGCUUCACCAAAAGCUG UCCCUUAGGGGAUUAGAACUUGAGUGAAGGUGGGCUGC UUGCAUCAGCCUAAUGUCGAGAAGUGCUUUCUUCGGAA AGUAACCCUCGAAACAAAUUCAGUGCUCCUCUCCAAUUC gaaaGAACCCGAAUAGAGCAAUGAAGGAAUGCAACNNNNN NNNNNNNNNNNNNNN | 157 |
| MS1/MS4*-2 | CUUCACUGAUAAAGUGGAGAACCGCUUCACCAAAAGCUG UCCCUUAGGGGAUUAGAACUUGAGUGAAGGUGGGCUGC UUGCAUCAGCCUAAUGUCGAGAAGUGCUUUCUUCGGAA AGUAACCCUCGAAACAAAUUCAGUGCUCCUCUCgaaaGAA UAGAGCAAUGAAGGAAUGCAACNNNNNNNNNNNNNNNN NNNN | 158 |
| MS1/MS4*-3 | CUUCACUGAUAAAGUGGAGAACCGCUUCACCAAAAGCUG UCCCUUAGGGGAUUAGAACUUGAGUGAAGGUGGGCUGC UUGCAUCAGCCUAAUGUCGAGAAGUGCUUUCUUCGGAA AGUAACCCUCGAAACAAAUUCAGUGCUgaaaAGCAAUGAA GGAAUGCAACNNNNNNNNNNNNNNNNNNNN | 159 |
| MS1/MS5-1 | CUUCACUGAUAAAGUGGAGAACCGCUUCACCAAAAGCUG UuuagAUUAGAACUUGAGUGAAGGUGGGCUGCUUGCAUCA GCCUAAUGUCGAGAAGUGCUUUCUUCGGAAAGUAACCCU CGAAACAAAUUCAGUGCUCCUCUCCAAUUCUGCACAAgaa aGUUGCAGAACCCGAAUAGAGCAAUGAAGGAAUGCAACN NNNNNNNNNNNNNNNNNN | 160 |
| MS1/MS5-2 | CUUCACUGAUAAAGUGGAGAACCGCUUCACCAAAAGCuua gGAACUUGAGUGAAGGUGGGCUGCUUGCAUCAGCCUAAU GUCGAGAAGUGCUUUCUUCGGAAAGUAACCCUCGAAACA AAUUCAGUGCUCCUCUCCAAUUCUGCACAAgaaaGUUGCA GAACCCGAAUAGAGCAAUGAAGGAAUGCAACNNNNNNN NNNNNNNNNNNNN | 161 |
| MS1/MS5-3 | CUUCACUGAUAAAGUGGAGAACCGCUUCACCAAuuagUUG AGUGAAGGUGGGCUGCUUGCAUCAGCCUAAUGUCGAGA AGUGCUUUCUUCGGAAAGUAACCCUCGAAACAAAUUCAG UGCUCCUCUCCAAUUCUGCACAAgaaaGUUGCAGAACCCG AAUAGAGCAAUGAAGGAAUGCAACNNNNNNNNNNNNNNN NNNNNN | 162 |
| MS1/MS2/MS4 -2 | CUUCACUGAUAAAGUGGAGAACCGCUUCACCAAAAGCUG UCCCUUAGGGGAUUAGAACUUGAGUGAAGGUGGGCUGC UUGCAUCAGCCUAAUGUCGAGAAGUGCUUUCUUCGGAA AGUAACCCUCGAAACAAAUUCAGUGCUCCUCUCgaaaGAA UAGAGCAAUGAAGGAAUGCAACNNNNNNNNNNNNNNNN NNNNUUUUAUUUU | 163 |
| MS1/MS3-3/MS4*-2 | ACCGCUUCACCAAAAGCUGUCCCUUAGGGGAUUAGAACU UGAGUGAAGGUGGGCUGCUUGCAUCAGCCUAAUGUCGA GAAGUGCUUUCUUCGGAAAGUAACCCUCGAAACAAAUUC AGUGCUCCUCUCgaaaGAAUAGAGCAAUGAAGGAAUGCAA CNNNNNNNNNNNNNNNNNNN | 164 |
| MS1/MS2/MS5 -3 | CUUCACUGAUAAAGUGGAGAACCGCUUCACCAAuuagUUG AGUGAAGGUGGGCUGCUUGCAUCAGCCUAAUGUCGAGA AGUGCUUUCUUCGGAAAGUAACCCUCGAAACAAAUUCAG UGCUCCUCUCCAAUUCUGCACAAgaaaGUUGCAGAACCCG AAUAGAGCAAUGAAGGAAUGCAACNNNNNNNNNNNNNNN NNNNNUUUUAUUUU | 165 |
| MS1/MS3-3/MS5-3 | ACCGCUUCACCAAuuagUUGAGUGAAGGUGGGCUGCUUGC AUCAGCCUAAUGUCGAGAAGUGCUUUCUUCGGAAAGUA ACCCUCGAAACAAAUUCAGUGCUCCUCUCCAAUUCUGCA CAAgaaaGUUGCAGAACCCGAAUAGAGCAAUGAAGGAAUG CAACNNNNNNNNNNNNNNNNNNNN | 166 |
| MS1/MS4*- | CUUCACUGAUAAAGUGGAGAACCGCUUCACCAAuuagUUG | 167 |
| 2/MS5-3 | AGUGAAGGUGGGCUGCUUGCAUCAGCCUAAUGUCGAGA AGUGCUUUCUUCGGAAAGUAACCCUCGAAACAAAUUCAG UGCUCCUCUCgaaaGAAUAGAGCAAUGAAGGAAUGCAACN NNNNNNNNNNNNNNNNNNN | |
| MS1/MS2/MS3 -3/MS4*-2 | ACCGCUUCACCAAAAGCUGUCCCuuagGGGAUUAGAACUU GAGUGAAGGUGGGCUGCUUGCAUCAGCCUAAUGUCGAG AAGUGCUUUCUUCGGAAAGUAACCCUCGAAACAAAUUCA GUGCUCCUCUCgaaaGAAUAGAGCAAUGAAGGAAUGCAAC NNNNNNNNNNNNNNNNNNNNUUUUAUUUU | 168 |
| MS1/MS2/MS3 -3/MS5-3 | ACCGCUUCACCAAuuagUUGAGUGAAGGUGGGCUGCUUGC AUCAGCCUAAUGUCGAGAAGUGCUUUCUUCGGAAAGUA ACCCUCGAAACAAAUUCAGUGCUCCUCUCCAAUUCUGCA CAAgaaaGUUGCAGAACCCGAAUAGAGCAAUGAAGGAAUG CAACNNNNNNNNNNNNNNNNNNNNUUUUAUUUU | 169 |
| MS1/MS2/MS4 *-2/MS5-3 | CUUCACUGAUAAAGUGGAGAACCGCUUCACCAAuuagUUG AGUGAAGGUGGGCUGCUUGCAUCAGCCUAAUGUCGAGA AGUGCUUUCUUCGGAAAGUAACCCUCGAAACAAAUUCAG UGCUCCUCUCgaaaGAAUAGAGCAAUGAAGGAAUGCAACN NNNNNNNNNNNNNNNNNNNUUUUAUUUU | 170 |
| MS1/MS3-3/MS4*-2/MS5-3 | ACCGCUUCACCAAuuagUUGAGUGAAGGUGGGCUGCUUGC AUCAGCCUAAUGUCGAGAAGUGCUUUCUUCGGAAAGUA ACCCUCGAAACAAAUUCAGUGCUCCUCUCgaaaGAAUAGA GCAAUGAAGGAAUGCAACNNNNNNNNNNNNNNNNNNNN | 171 |
| MS1/MS2/MS3 -3/MS4*-2/MS5-3 | ACCGCUUCACCAAuuagUUGAGUGAAGGUGGGCUGCUUGC AUCAGCCUAAUGUCGAGAAGUGCUUUCUUCGGAAAGUA ACCCUCGAAACAAAUUCAGUGCUCCUCUCgaaaGAAUAGA GCAAUGAAGGAAUGCAACNNNNNNNNNNNNNNNNNNNN UUUUAUUUU | 172 |

In addition, mature form gRNAs were produced by removing the modification site MS 1 from the canonical gRNA, and the specific sequences thereof are shown in Table 12.

**[Table 12]**

| gRNA | Sequence (5' to 3') | SEQ ID NO |
|---|---|---|
| Mature form gRNA | CUUCACUGAUAAAGUGGAGAACCGCUUCACCAAAAGCUG UCCCuuagGGGAUUAGAACUUGAGUGAAGGUGGGCUGCUU GCAUCAGCCUAAUGUCGAGAAGUGCUUUCUUCGGAAAG UAACCCUCGAAACAAAUUCAUUUgaaaGAAUGAAGGAAUG CAACNNNNNNNNNNNNNNNNNNNN | 173 |
| MS3-1 | GAUAAAGUGGAGAACCGCUUCACCAAAAGCUGUCCCuuag GGGAUUAGAACUUGAGUGAAGGUGGGCUGCUUGCAUCA GCCUAAUGUCGAGAAGUGCUUUCUUCGGAAAGUAACCCU CGAAACAAAUUCAUUUgaaaGAAUGAAGGAAUGCAACNNN NNNNNNNNNNNNNNNNN | 174 |
| MS3-2 | UGGAGAACCGCUUCACCAAAAGCUGUCCCuuagGGGAUUA GAACUUGAGUGAAGGUGGGCUGCUUGCAUCAGCCUAAU GUCGAGAAGUGCUUUCUUCGGAAAGUAACCCUCGAAACA AAUUCAUUUgaaaGAAUGAAGGAAUGCAACNNNNNNNNNN NNNNNNNNNN | 175 |
| MS3-3 | ACCGCUUCACCAAAAGCUGUCCCuuagGGGAUUAGAACUU GAGUGAAGGUGGGCUGCUUGCAUCAGCCUAAUGUCGAG AAGUGCUUUCUUCGGAAAGUAACCCUCGAAACAAAUUCA UUUgaaaGAAUGAAGGAAUGCAACNNNNNNNNNNNNNNN NNNNN | 176 |
| MS4-1 | CUUCACUGAUAAAGUGGAGAACCGCUUCACCAAAAGCUG UCCCuuagGGGAUUAGAACUUGAGUGAAGGUGGGCUGCUU GCAUCAGCCUAAUGUCGAGAAGUGCUUUCUUCGGAAAG UAACCCUCGAAACAAAUUCAUgaaaAUGAAGGAAUGCAAC NNNNNNNNNNNNNNNNNNNN | 177 |
| MS4-2 | CUUCACUGAUAAAGUGGAGAACCGCUUCACCAAAAGCUG UCCCuuagGGGAUUAGAACUUGAGUGAAGGUGGGCUGCUU GCAUCAGCCUAAUGUCGAGAAGUGCUUUCUUCGGAAAG UAACCCUCGAAACAAAUUCgaaaGAAGGAAUGCAACNNNN NNNNNNNNNNNNNNNN | 178 |
| MS4-3 | CUUCACUGAUAAAGUGGAGAACCGCUUCACCAAAAGCUG UCCCuuagGGGAUUAGAACUUGAGUGAAGGUGGGCUGCUU GCAUCAGCCUAAUGUCGAGAAGUGCUUUCUUCGGAAAG UAACCCUCGAAACAAAgaaaGGAAUGCAACNNNNNNNNNN NNNNNNNNNN | 179 |
| MS5-1 | CUUCACUGAUAAAGUGGAGAACCGCUUCACCAAAAGCUG UuuagAUUAGAACUUGAGUGAAGGUGGGCUGCUUGCAUCA GCCUAAUGUCGAGAAGUGCUUUCUUCGGAAAGUAACCCU CGAAACAAAUUCAUUUgaaaGAAUGAAGGAAUGCAACNNN NNNNNNNNNNNNNNNNN | 180 |
| MS5-2 | CUUCACUGAUAAAGUGGAGAACCGCUUCACCAAAAGCUu uagAGAACUUGAGUGAAGGUGGGCUGCUUGCAUCAGCCU AAUGUCGAGAAGUGCUUUCUUCGGAAAGUAACCCUCGA AACAAAUUCAUUUgaaaGAAUGAAGGAAUGCAACNNNNNN NNNNNNNNNNNNNN | 181 |
| MS5-3 | CUUCACUGAUAAAGUGGAGAACCGCUUCACCAAuuagUUG AGUGAAGGUGGGCUGCUUGCAUCAGCCUAAUGUCGAGA AGUGCUUUCUUCGGAAAGUAACCCUCGAAACAAAUUCAU UUgaaaGAAUGAAGGAAUGCAACNNNNNNNNNNNNNNNN NNNN | 182 |
| MS3-3/MS4-3 | ACCGCUUCACCAAAAGCUGUCCCuuagGGGAUUAGAACUU GAGUGAAGGUGGGCUGCUUGCAUCAGCCUAAUGUCGAG AAGUGCUUUCUUCGGAAAGUAACCCUCGAAACAAAgaaaG GAAUGCAACNNNNNNNNNNNNNNNNNNNNN | 183 |
| MS3-3/MS5-3 | ACCGCUUCACCAAuuagUUGAGUGAAGGUGGGCUGCUUGC AUCAGCCUAAUGUCGAGAAGUGCUUUCUUCGGAAAGUA ACCCUCGAAACAAAUUCAUUUgaaaGAAUGAAGGAAUGCA ACNNNNNNNNNNNNNNNNNNNN | 184 |
| MS4-3/MS5-3 | CUUCACUGAUAAAGUGGAGAACCGCUUCACCAAuuagUUG AGUGAAGGUGGGCUGCUUGCAUCAGCCUAAUGUCGAGA AGUGCUUUCUUCGGAAAGUAACCCUCGAAACAAAgaaaGG AAUGCAACNNNNNNNNNNNNNNNNNNNNN | 185 |
| MS3-3/MS4-3/MS5-3 | ACCGCUUCACCAAuuagUUGAGUGAAGGUGGGCUGCUUGC AUCAGCCUAAUGUCGAGAAGUGCUUUCUUCGGAAAGUA ACCCUCGAAACAAAgaaaGGAAUGCAACNNNNNNNNNNNN NNNNNNNN | 186 |

The sequence indicated by NNNNNNNNNNNNNNNNNNNN in Tables 11 and 12 refers to any guide sequence (spacer sequence) that can hybridize with the target sequence. The guide sequence may be appropriately designed by those skilled in the art according to a desired target gene and/or a target sequence, and is not limited to a specific sequence of a particular length.

### Example 2.2. Cas12f1 and TaRGET systems

UnCas12f1 and CWCas21f1 were used together with the guide RNA of Example 2.1. PCR amplification was performed using the human codon-optimized nucleotide sequence (SEQ ID NOS: 10 and 6) of the protein as a template, and cloning was performed, according to the desired cloning sequence, into a vector having a promoter capable of expression in a eukaryotic system and a poly(A) signal sequence using the Gibson assembly method. After cloning, the sequence of the obtained recombinant plasmid vector was finally identified by the Sanger sequencing method. The nucleic acid construct thus produced was cloned into the pMAL-c2 plasmid vector, and transformed into BL21(DE3) *E. coli* cells. The transformed *E*. *coli* colonies were grown in LB broth at 37°C until the optical density reached 0.7. The transformed *E. coli* cells were cultured at 18°C overnight in the presence of 0.1 mM isopropylthio-β-D-galactoside. Thereafter, the cultured cells were collected by centrifugation at 3,500 g for 30 minutes, and the collected cells were resuspended in a buffer containing 20 mM Tris-HCl (pH 7.6), 500 mM NaCl, 5 mM β-mercaptoethanol, and 5% glycerol. The cells were lysed in a lysis buffer and then disrupted by sonication. The sample containing the disrupted cells was centrifuged at 15,000 g for 30 minutes, and the supernatant obtained was filtered through a 0.45 µm syringe filter (Millipore). The filtered supernatant was loaded onto a Ni²⁺-affinity column using an FPLC purification system (KTA Purifier, GE Healthcare). The bound fractions were eluted with a gradient of 80-400 mM imidazole, 20 mM Tris-HCl (pH 7.5).

The eluted proteins were cleaved by treatment with TEV protease for 16 hours. The cleaved proteins were purified on a heparin column with a linear gradient of 0.15-1.6 M NaCl. The recombinant Cas12f1 variant protein purified on the heparin column was dialyzed against a solution of 20 mM Tris (pH 7.6), 150 mM NaCl, 5 mM β-mercaptoethanol, and 5% glycerol. The dialyzed protein was purified by passing it through an MBP column, and then repurified on a monoS column (GE Healthcare) or EnrichS with a linear gradient of 0.5-1.2 M NaCl.

The repurified proteins were collected and dialyzed against a solution of 20 mM Tris (pH 7.6), 150 mM NaCl, 5 mM β-mercaptoethanol, and 5% glycerol to purify the hypercompact gene editing protein (miniature endonuclease) used in the present disclosure. The concentration of the produced hypercompact gene editing protein was quantified by the Bradford quantitative method using bovine serum albumin (BSA) as a standard and measured electrophoretically on a coomassie blue-stained SDS-PAGE gel.

### Comparative Example 1. SaCas9 System

The guide sequence was cloned into plasmids containing U6 promoter and SaCas9 scaffold, respectively. Then, the sequence from each U6 promoter to the guide RNA was inserted into the plasmid encoding SaCas9 to produce a one-vector module. Information on the target sequences is shown in Table 13.

**[Table 13]**

| Target | PAM | Sequence | SEQ ID NO |
|---|---|---|---|
| F68 | CTGAAT | GTGTATTGCTTGTACTACTCA | 471 |
| R84 | GAGAGT | GTGTTATTACTTGCTACTGCA | 472 |

### Example 2.3. Selection of target sequences (E51)

The regions 2000 bp upstream and 2000 bp downstream of exon 51 were set as target regions for deletion of exon 51, and the target regions are referred to as the front region (F region) and the rear region (R region), respectively. Various protospacer sequences were selected from the above regions, guide RNAs were designed based thereon, and analysis of indel and deletion efficiency was performed. As a result, the sequences shown in Table 14 were confirmed to have high indel and deletion efficiency, and were selected as target sequences to perform subsequent experiments.

**[Table 14]**

| Target name | PAM | Target seq | SEQ ID NO |
|---|---|---|---|
| R52 | TTTA | CTCTCCTAGACCATTTCCCA | 218 |
| F142 | TTTG | CTCATTCTCATGCCTGGACA | 213 |

### Example 3. qPCR analysis on shRNA-mediated inhibition of expression of NHEJ component gene

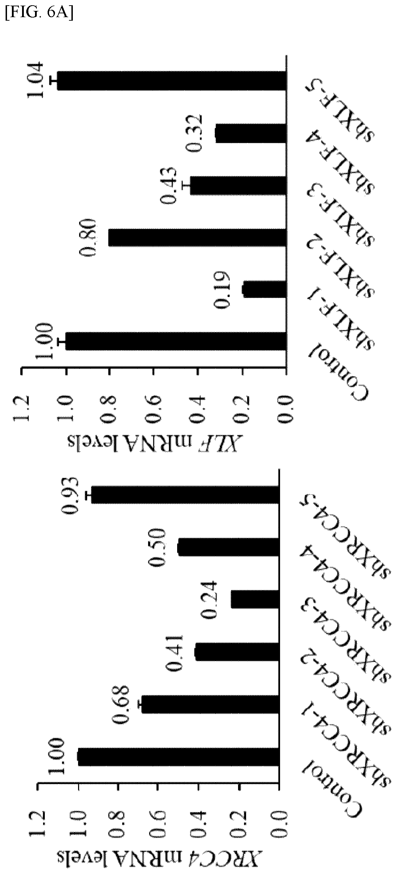

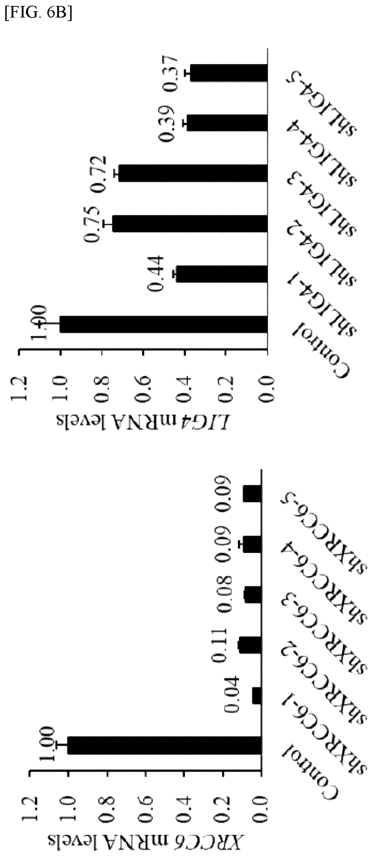

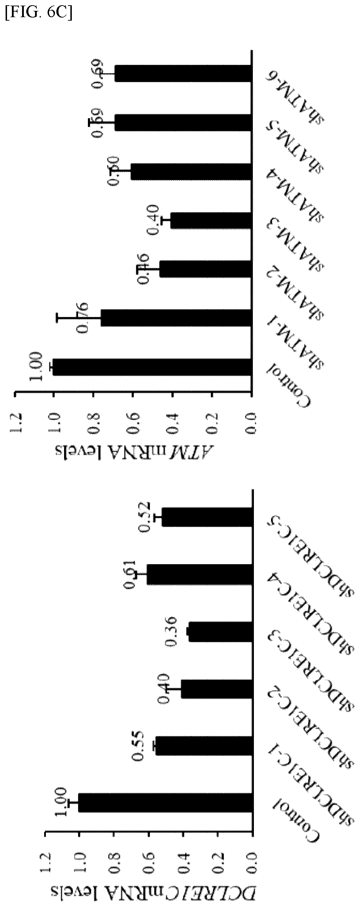

5 µg of plasmid vector encoding shRNA was prepared using All-in-one vector (Biofact), transfected into AC16 cells, and harvested 3 days after transfection. Then, mRNA expression of each NHEJ component gene was measured by qRT-PCR. As a result, XRCC4-3, XLF-1, XRCC6-1, LIG4-5, DCLRE1C-3, and ATM-3 were selected as the most effective shRNAs. The results are shown in FIG. 6. The sequences of sense strands of the shRNAs used are described in Table 9.

### Example 4. Western blot analysis on shRNA-mediated inhibition of expression of NHEJ component gene

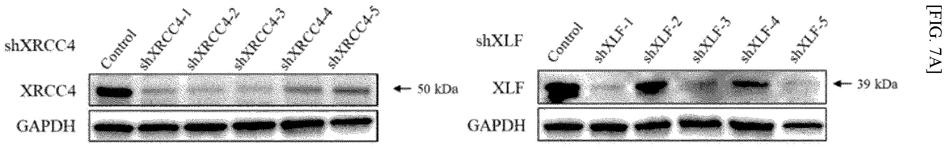

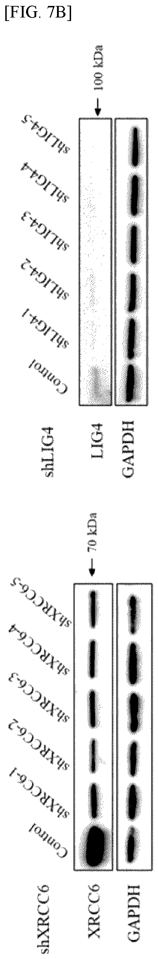

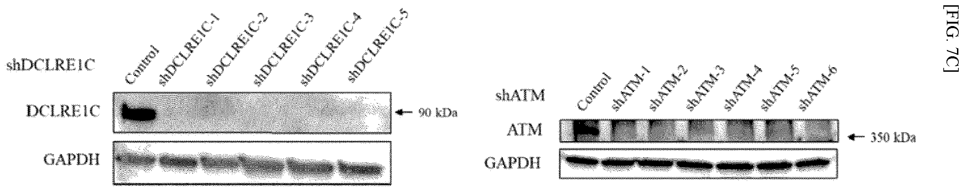

shRNA-mediated inhibition of gene expression was analyzed by Western blot analysis. The experimental conditions were the same as in Example 3. The results are shown in FIG. 7. From the results, it was possible to select the best shRNAs for XRCC4-3, XLF-1, XRCC6-1, LIG4-5, DCLRE1C-3, and ATM-3 and it can be seen that these shRNAs effectively reduced expression of the target genes. As a result, the Western blot results were consistent with the qRT-PCR results.

### Example 5. Confirmation of increased deletion efficiency caused by UnCas12f1 following shRNA treatment (E51)

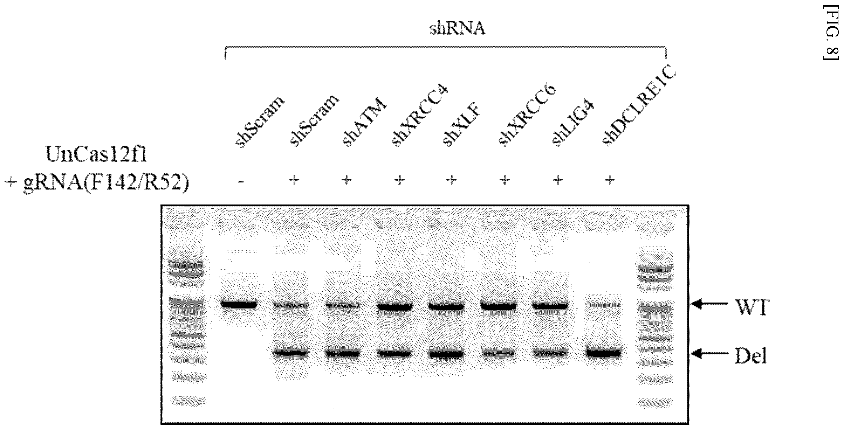

The most effective shRNA module for each target identified in Examples 3 and 4 was cloned into the UnCas12f1 expression vector, and the shRNA was designed to be expressed under the U6 promoter. A pair of guide RNAs for the targets F142 and R52 as shown in Table 14 was used to induce E51 deletion. In order to accurately find the optimal shRNA for a large-scale deletion strategy, comparison of the deletion efficiency altered by the selected shRNAs was conducted in HEK 293T cells. The E51 deletion efficiency was measured on an agarose gel in which the PCR-amplified E51 region was decomposed. The results are shown in FIG. 8. From the results, it can be seen that treatment with shRNA increases the E51 deletion efficiency caused by UnCas12f1.

### Example 6. Quantification of deletion efficiency (E51)

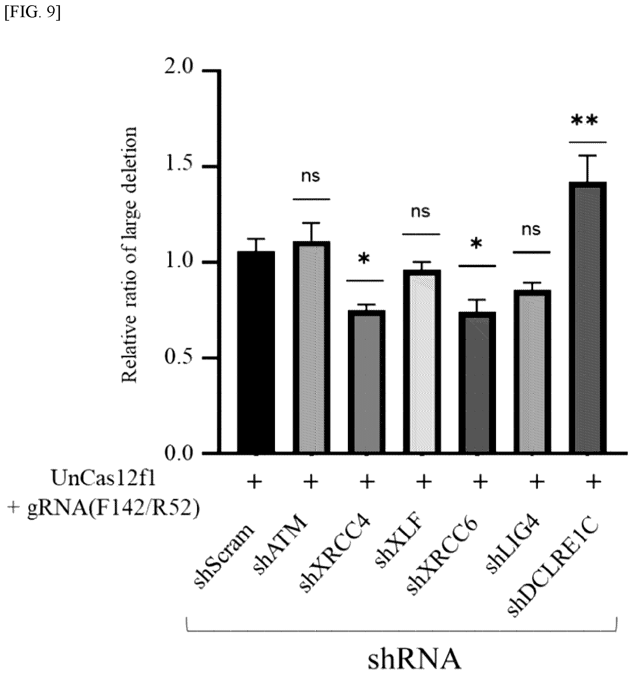

To compare the large-scale deletion efficiency between various treatments with shRNA, intensity analysis was conducted on the deletion band (Del) and the non-deletion band (WT) in FIG. 8, and the relative values were calculated using Image J software. The results are shown in FIG. 9. From the results, it can be seen that shRNA for the DCLRE1C gene is most effective in improving the large-scale deletion efficiency.

### Example 7. Confirmation of optimal shRNA for deletion (E51)

### Example 7.1. Analysis of deletion efficiency following treatment with shRNADCLREIC

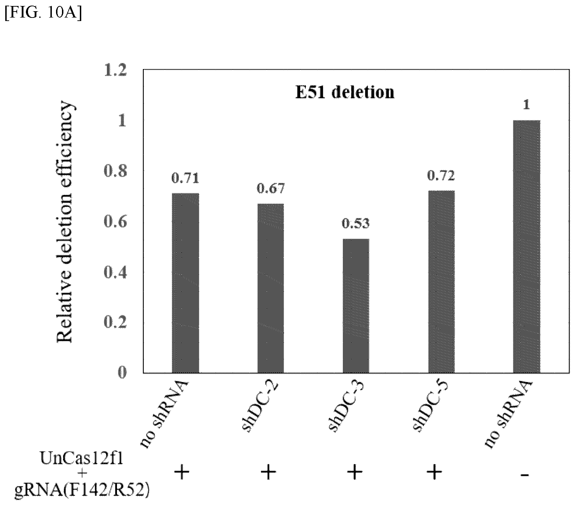

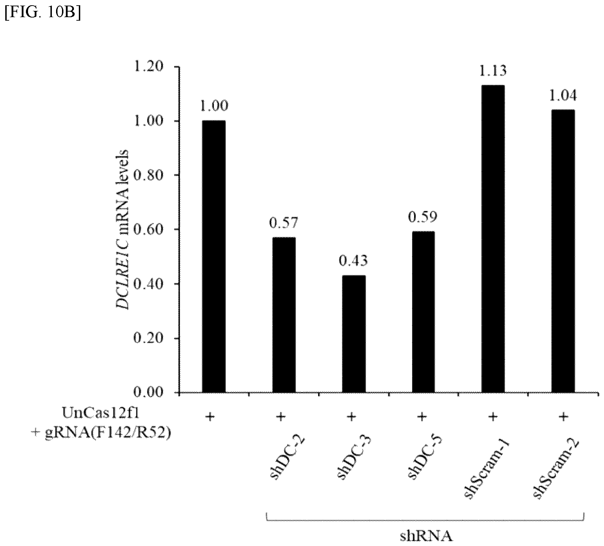

The efficiency of large-scale deletion was analyzed by qPCR 3 days after treatment of HEK293T cells with the selected shRNA (shDCLRE1C-2, -3, or -5) + UnCas12f1 and a pair of gRNA (F142/R52), and the results are shown in FIG. 10A. In addition, the DCLRE1C mRNA levels were measured under the same conditions, and the results are shown in FIG. 10B.

As can be seen from FIG. 10A, the sample treated with shDCLRE1C-3 showed the highest deletion level compared to the remaining shRNAs. These results are thought to stem from the fact that shDCLRE1C-3 most effectively downregulates DCLRE1C expression compared to the remaining shRNAs (see FIG. 10B).

### Example 7.2. Analysis of deletion efficiency folowing treatment with shRNADCLREIC and shXRCC6

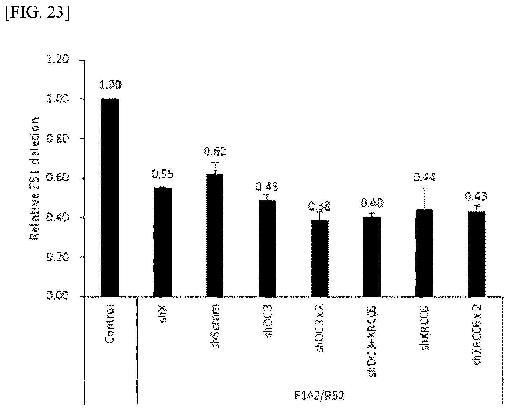

For shDCLRE1C and shXRCC6 among the shRNAs selected in Example 4, experiments were conducted in the same manner as in Example 7.1 using the two shRNAs alone, in combination, or one shRNA thereof in two copies, to analyze the efficiency of large-scale deletion of E51. The results are shown in FIG. 23.

As can be seen from FIG. 23, shDCLRE1C and shXRCC6 resulted in high deletion of E51 when used alone, in combination, or in two copies. In particular, the highest deletion was observed when shDCLRE1C was used in two copies, and very high deletion was also observed when shDCLRE1C and shXRCC6 were used in combination.

### Example 8. Whole genome sequencing (WGS) analysis for measurement of deletion efficiency (E51)

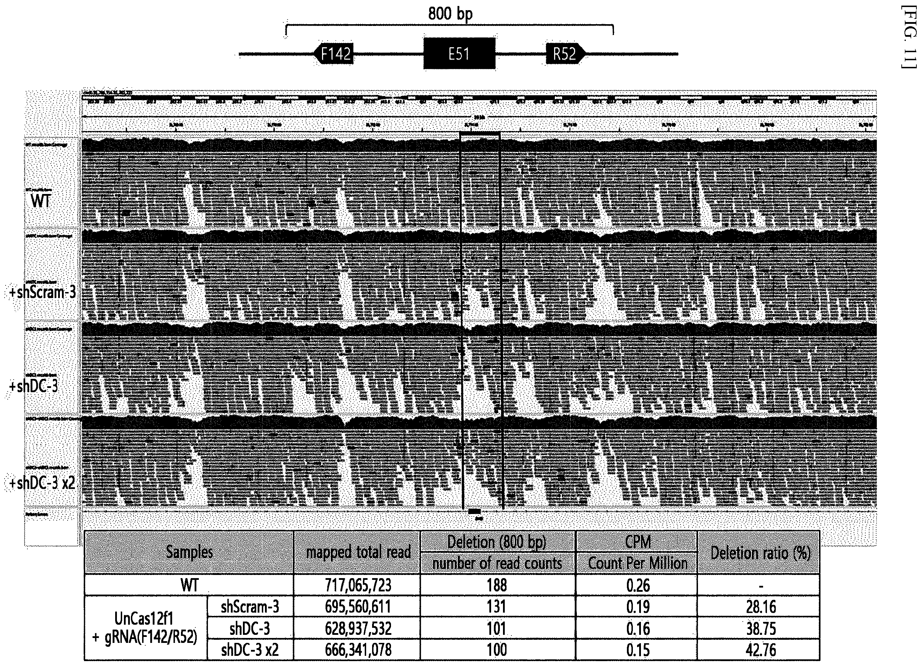

HEK293T cells were transfected with UnCas12f1, a pair of gRNA (F142/R52), and shRNA, and then harvested 5 days after transfection. gDNA was prepared therefrom for WGS that is performed to verify large-scale deletion efficiency. To compare the degree of deletion between the two gRNAs which is induced at both sides of E51, reads were mapped to the reference standard sequence and counting was performed within the deleted region. The data were presented as CPM (Count Per Million) along with each read count. The results are shown in FIG. 11. From the results, it can be seen that in a case of being treated with shDCLRE1C-3 in two copies (shDC-3 ×2), the mapped reads in the deleted region decreased, which indicates increased deletion ofE51.

### Example 9. Confirmation of effect of shDCLREIC on increased deletion efficiency through complementation of DCLRE1C expression (E51)

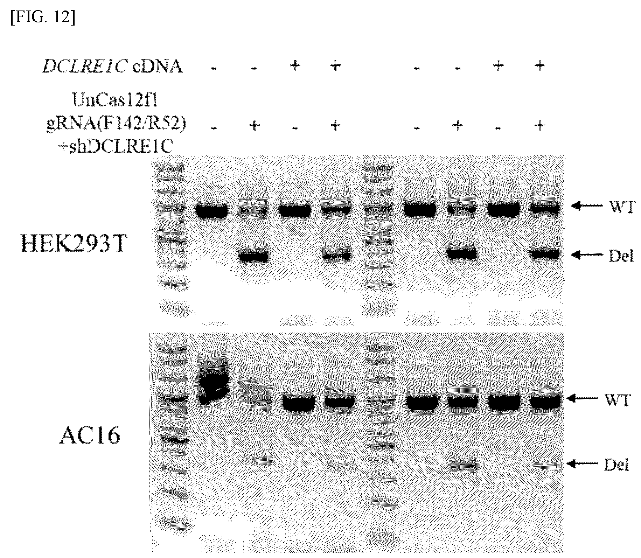

To confirm the effect of functional Artemis protein on large-scale deletion efficiency caused by UnCas12f1, DCLRE1C cDNA was co-transfected with shDCLRE1C-3 into HEK293T and AC16 cells. The results are shown in FIG. 12. From the results, it can be seen that the increased large-scale deletion caused by shDCLRE1C was nullified by restoration of DCLRE1C expression.

### Example 10. Increased deletion efficiency in DCLRE1C-KO cell line (E51)

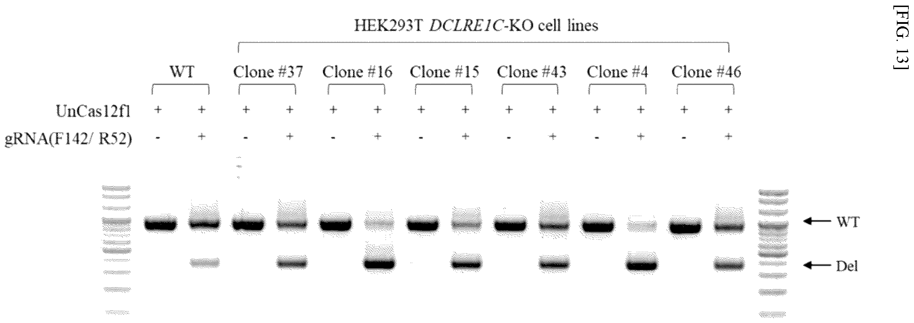

To confirm the effect of DCLRE1C gene on large-scale deletion caused by UnCas12f1, DCLRE1C-KO cell lines of HEK293T cells were generated using a CRISPR-Cas12f1 system. Then, deletion of E51 induced by UnCas12f1 and a pair of gRNA (F142/R52) was analyzed by qPCR at the deleted locus. The results are shown in FIG. 13. From the results, it can be seen that increased large-scale deletion was observed in all KO cell lines compared to the wild type. Therefore, it is thought that the loss of function of the Artemis protein in KO cells resulted in increased large-scale deletion.

### Example 11. Selection of shRNA for inhibited expression of murine Dclrelc

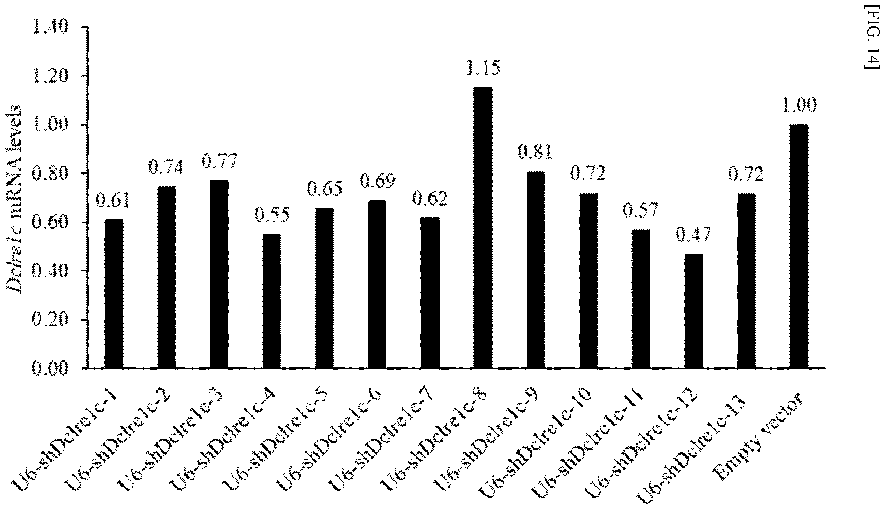

shRNAs for the Dclreic gene encoding mouse Artemis protein were selected in Hepa-1c1c7 cells. The shRNAs, which are expressed under the U6 promoter, were generated, and qRT-PCR was performed to measure of the Dclreic mRNA levels. The sequences of the shRNAs used here are listed in Table 9. The results are shown in FIG. 14. From the results, the greatest decrease in the Dclreic mRNAlevel was observed in cells treated with shDclre1c-12. The thus selected shRNAs can be used for *in vivo* studies using mouse models (for example, DMD mouse model with endogenous murine Dclre1c gene).

### Example 12. Comparison of deletion of exon 51 caused by various gene editing systems comprising shRNA

### Example 12.1. Selection of shRNA

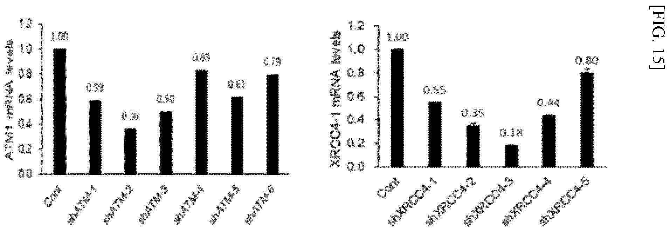

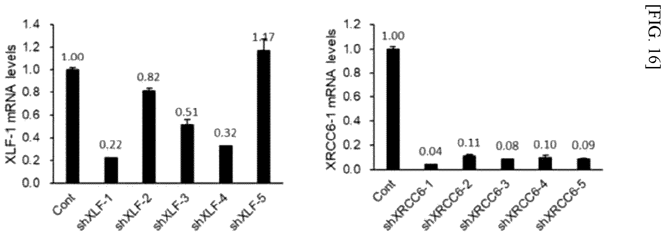

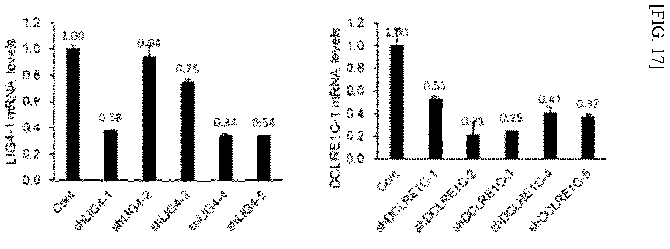

Candidate shRNAs for six genes known to be involved in the NHEJ repair pathway were transfected into previously prepared AC16 cells at a dose of 5 ug, and incubation was performed for 3 days. Then, the cells were harvested and qRT-PCR was used to measure the mRNA expression levels of the genes. The results are shown in FIG. 15 (ATM1 and XRCC4), FIG. 16 (XLF-1 and XRCC6), and FIG. 17 (LIG4 and DCLRE1C). From the results, the optimal shRNAs were selected. The candidate shRNAs used for selection are shown in Table 15. Scrambled indicates the control shRNA.

**[Table 15]**

| Gene | No. | shRNA | SEQ ID NO |
|---|---|---|---|
| ATM1 | 1 | GGAGCCAGAUAGUUUGUAUUUCAAGAGAAUACA AACUAUCUGGCUCC | 360 |
| | 2 | GCAAGCAGCUGAAACAAAUUUCAAGAGAAUUUG UUUCAGCUGCUUGC | 361 |
| | 3 | GGAGCUGAUUGUAGCAACAUUCAAGAGAUGUUG CUACAAUCAGCUCC | 362 |
| | 4 | GCACAGAAGUGCCUCCAAUUUCAAGAGAAUUGGA GGCACUUCUGUGC | 363 |
| | 5 | GGACAUAGUUUCUGGGAGAUUCAAGAGAUCUCCC AGAAACUAUGUCC | 364 |
| | 6 | GAACUUCAGUGGACCUUCAUUCAAGAGAUGAAG GUCCACUGAAGUUC | 473 |
| XRCC4 | 1 | GGAUGACACUGGCACAUUAUUCAAGAGAUAAUG UGCCAGUGUCAUCC | 365 |
| | 2 | GGAGAGUACUGAUGAGGAAUUCAAGAGAUUCCU CAUCAGUACUCUCC | 366 |
| | 3 | GAAUCCACCUUGUUUCUGAUUCAAGAGAUCAGAA ACAAGGUGGAUUC | 367 |
| | 4 | GUACAAGUAUCUUGGGAGAUUCAAGAGAUCUCCC AAGAUACUUGUAC | 368 |
| | 5 | GAAUGCAGCUCAAGAACGAUUCAAGAGAUCGUUC UUGAGCUGCAUUC | 369 |
| XLF-1 | 1 | GCAUGAGUCUGGCAUUACAUUCAAGAGAUGUAA UGCCAGACUCAUGC | 370 |
| | 2 | GAAAGCCCUUUGUCAUGAAUUCAAGAGAUUCAU GACAAAGGGCUUUC | 371 |
| | 3 | GAACAGUGCUUCCCUGCAAUUCAAGAGAUUGCAG GGAAGCACUGUUC | 372 |
| | 4 | GGAAAGACCUAGAGAUCCAUUCAAGAGAUGGAU CUCUAGGUCUUUCC | 373 |
| | 5 | GUAUGGCAGUCACCACACAUUCAAGAGAUGUGUG GUGACUGCCAUAC | 374 |
| XRCC6 | 1 | GCAGCAUUGUGCAGAUACAUUCAAGAGAUGUAU CUGCACAAUGCUGC | 375 |
| | 2 | GCAGGAACAUCCCUCCUUAUUCAAGAGAUAAGGA GGGAUGUUCCUGC | 376 |
| | 3 | GCAGUGCUCUGCUCAUCAAUUCAAGAGAUUGAUG AGCAGAGCACUGC | 377 |
| | 4 | GGAUCAUGCUGUUCACCAAUUCAAGAGAUUGGU GAACAGCAUGAUCC | 378 |
| | 5 | GGAUCUGACUACUCACUCAUUCAAGAGAUGAGUG AGUAGUCAGAUCC | 379 |
| LIG4 | 1 | GCACAAAGAUGGAGAUGUAUUCAAGAGAUACAU CUCCAUCUUUGUGC | 380 |
| | 2 | GCAGACACGUACUGUGUAAUUCAAGAGAUUACAC AGUACGUGUCUGC | 381 |
| | 3 | GGAGCAGACUCCUGAAGAAUUCAAGAGAUUCUUC AGGAGUCUGCUCC | 382 |
| | 4 | GGAGGAUUCUGAUCUGCAAUUCAAGAGAUUGCA GAUCAGAAUCCUCC | 383 |
| | 5 | GCAUGAUCCUUCUGUAGGAUUCAAGAGAUCCUAC AGAAGGAUCAUGC | 384 |
| DCLRE1C | 1 | GGAGACUCCUACCCAGAUAUUCAAGAGAUAUCUG GGUAGGAGUCUCC | 385 |
| | 2 | GGACAAAGCUGACUACAGAUUCAAGAGAUCUGU AGUCAGCUUUGUCC | 386 |
| | 3 | GCAGAGCUCUCGUUUCACAUUCAAGAGAUGUGAA ACGAGAGCUCUGC | 387 |
| | 4 | GGACUCUGAUGGAGAAUCAUUCAAGAGAUGAUU CUCCAUCAGAGUCC | 388 |
| | 5 | GCAGAAUUCUUCCCAGUCAUUCAAGAGAUGACUG GGAAGAAUUCUGC | 389 |
| Scrambled | 3 | CAGAGCUAACUCAGAUAGUACU | 470 |

### Example 12.2. Deletion of exon 51 by system comprising shRNA

### Measurement of deletion efficiency for exon 51 in case of using single shRNA

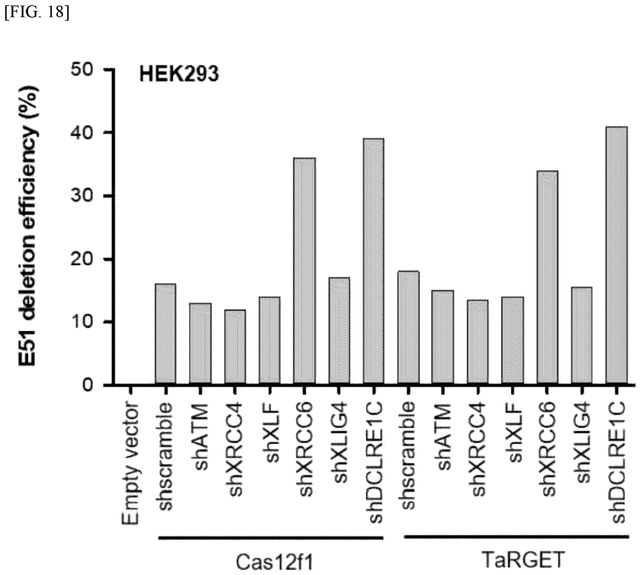

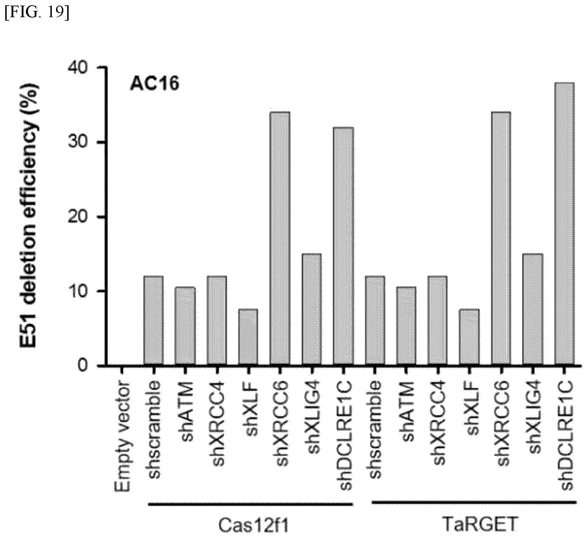

Based on the qRT-PCR results, for each gene, the shRNA that exhibited the highest inhibition efficiency for mRNA expression was selected. Nucleic acids encoding the shRNA, the two guide RNAs, and CWCas12f1 or Cas12f1 were inserted into a single vector, and the vector was transfected into AC16 cells and HEK293 cells. The experiment was independently repeated three times to measure the relative deletion efficiency for exon 51. Here, relative means a relative value under the same conditions, as indel efficiency varies depending on the transfection time, vector type, and concentration. The results are shown in FIG. 18 (HEK293 cells) and FIG. 19 (AC16 cells).

Referring to FIGS. 18 and 19, it was confirmed that exon 51 was effectively deleted through inhibition of expression of NHEJ-related genes in all Cas12f1 systems, and in particular, higher deletion level of exon 51 was observed in a case of using shXRCC6 and shDCLRE1C.

### Measurement of deletion efficiency for exon 51 in case of using two or more shRNAs

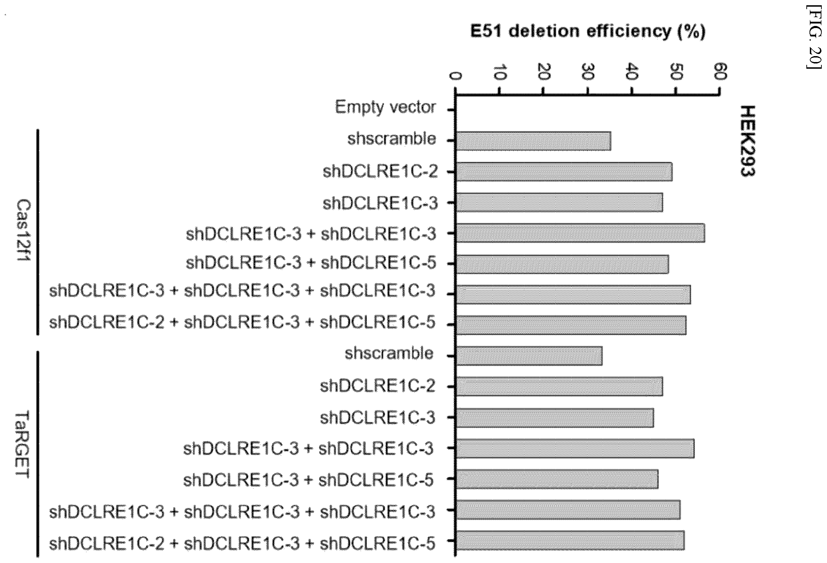

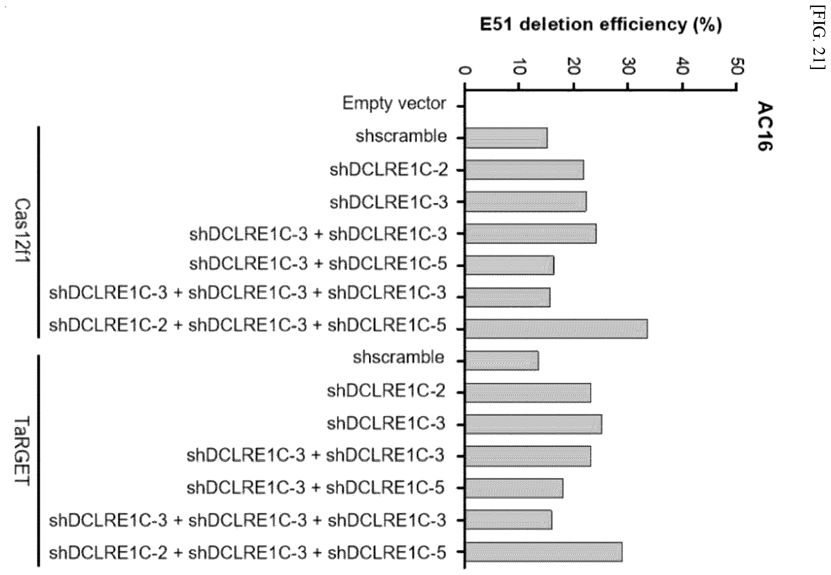

Deletion of exon 51 was induced using two or more identical or different shRNAs. Nucleotide sequences encoding the two guide RNAs, Cas12f1, and shDCLRE1C (one, two, or three selected from shDCLRE1C2, shDCLRE1C3, and shDCLRE1C5) were inserted into a single vector and the vector was transfected into HEK293 cells and AC16 cells using the same method as described above to measure the relative deletion efficiency of exon 51. Here, the shDCLRE1Cs used were three that showed the highest mRNA expression inhibition efficiency among the five shRNAs (right one in FIG. 17). The results are shown in FIG. 20 (HEK293 cells) and FIG. 21 (AC16 cells).

Referring to FIGS. 20 and 21, deletion of exon 51 was successfully achieved in all experimental groups, and the deletion efficiency for exon 51 was particularly excellent in a case where two or more shDCLRE1Cs were introduced.

### Example 12.3. Deletion of exon 51 over time after transfection with system comprising shRNA

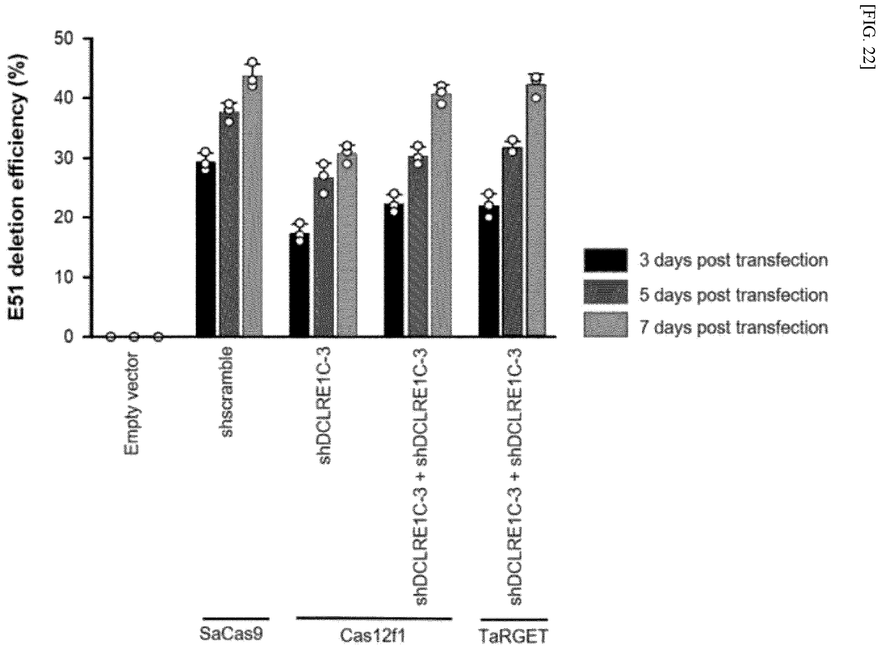

To determine deletion efficiency for exon 51 over different transfection periods (3 days, 5 days, and 7 days post transfection) using one or more shRNAs, nucleic acids encoding the two guide RNAs, TnpB or Cas12f1, and one or more shRNAs were inserted into a single vector, and then the deletion efficiency for exon 51 was determined at various time points starting from the day of transfection. In all experiments, AC16 cells were used, and the empty vector and the SaCas9 system were prepared as controls for comparison. The results are shown in FIG. 22.

] As shown in FIG. 22, the deletion efficiency of exon 51 increased over time from the start of transfection in all experimental groups. In particular, 7 days after transfection, the deletion efficiency for exon 51 in the Cas12f1 and TaRGET systems that use a combination of two shDCLRE1Cs was similar to that of SaCas9.

### Conclusion

As such, it was shown that inhibition of NHEJ pathway factors by shRNA can increase efficiency of large-scale deletion induced by UnCas12f1. It has been found that among the various shRNAs selected, interference of the DCLRE1C gene encoding the Artemis protein most effectively increases the efficiency of large-scale deletion. Large-scale deletion achieved by using Cas nuclease and a pair of gRNA can be utilized as a gene editing approach for treatment of a genetic disease such as DMD, and low editing efficiency *in vivo* is likely to be one of the biggest obstacles in determining whether to proceed to the next stage. Therefore, it is expected that increased efficiency of large-scale deletion achieved by shRNA-mediated inhibition of NHEJ component gene expression will be widely applied in development of deletion-based gene editing therapies.

## Claims

1. A composition for increasing deletion of a nucleic acid segment, comprising a molecule that inhibits expression of a gene involved in non-homologous end joining (NHEJ) pathway, or a nucleic acid construct encoding the molecule.

2. The composition of claim 1, wherein the gene involved in non-homologous end joining pathway comprises at least one selected from the group consisting of ATM1, XRCC4, XLF, XRCC6, LIG4, and DCLRE1C.

3. The composition of claim 2, wherein the gene involved in non-homologous end joining pathway comprises at least one selected from the group consisting of XRCC6 and DCLRE1C.

4. The composition of claim 1, wherein the molecule is shRNA, dsRNA, siRNA, miRNA, or an antisense oligonucleotide.

5. The composition of claim 4, wherein the shRNA molecule comprises at least one selected from the group consisting of shXRCC6 and shDCLRE1C.

6. The composition of claim 4, wherein the shRNA molecule comprises a nucleotide sequence selected from the group consisting of SEQ ID NOS: 360 to 389, SEQ ID NOS: 400 to 430, SEQ ID NOS: 433 to 445, and SEQ ID NO: 473.

7. The composition of claim 5, wherein the shRNA molecule comprises a nucleotide sequence selected from the group consisting of SEQ ID NOS: 375 to 379, SEQ ID NOS: 385 to 389, SEQ ID NOS: 410 to 414, SEQ ID NOS: 420 to 424, and SEQ ID NOS: 433 to 445.

8. The composition of claim 1, wherein the composition comprises at least two of the molecules that inhibit expression of a gene involved in non-homologous end joining (NHEJ) pathway or nucleic acid constructs encoding the molecules.

9. The composition of claim 8, wherein the at least two inhibitory molecules each inhibits expression of the same or different genes.

10. The composition of claim 1, wherein the composition further comprises (i) a Cas endonuclease and (ii) a first guide RNA and a second guide RNA that hybridize to a first target sequence and a second target sequence, respectively;
the nucleic acid segment exists between the first target sequence and the second target sequence, and
the Cas endonuclease forms a complex with the first guide RNA to induce first cleavage, and the Cas endonuclease forms a complex with the second guide RNA to induce second cleavage.

11. The composition of claim 1, wherein the composition further comprises at least one nucleic acid construct comprising (i) a nucleic acid encoding a Cas endonuclease, (ii) a nucleic acid encoding a first guide RNA that hybridizes to a first target sequence, and (iii) a nucleic acid encoding a second guide RNA that hybridizes to a second target sequence,
the nucleic acid segment exists between the first target sequence and the second target sequence, and
the Cas endonuclease forms a first complex with the first guide RNA to induce first cleavage, and the Cas endonuclease forms a second complex with the second guide RNA to induce second cleavage.

12. The composition of claim 10 or 11, wherein the first cleavage and the second cleavage are each independently a single-strand DNA break or a double-strand DNA break.

13. The composition of claim 10 or 11, wherein the composition or nucleic acid construct comprises an additional guide RNA that hybridizes to an additional target sequence or a nucleic acid construct encoding the additional guide RNA.

14. The composition of claim 10 or 11, wherein the Cas endonuclease is an endonuclease comprising a Cas12f1 protein.

15. The composition of claim 14, wherein the Cas12f1 protein has at least 70% sequence identity to the amino acid sequence of SEQ ID NO: 1 or 5.

16. The composition of claim 10 or 11, wherein the first or second guide RNA is an engineered guide RNA.

17. The composition of claim 16, wherein the engineered guide RNA comprises a U-rich tail sequence linked to the 3'-end of its guide sequence, in which the U-rich tail is represented by 5'-(UₘV)ₙUₒ-3', wherein V is each independently A, C, or G, m and o are integers between 1 to 20, and n is an integer between 0 to 5.

18. The composition of claim 16, wherein the engineered guide RNA comprises a nucleotide sequence having at least 50% sequence identity to a scaffold region of a wild-type Cas12f1 guide RNA sequence that sequentially comprises, from the 5'-end, a first stem-loop region, a second stem-loop region, a third stem-loop region, a fourth stem-loop region, and a tracrRNA-crRNA complementarity region, and
the engineered guide RNA comprises at least one modification selected from the group consisting of the following (1) to (5) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region;
(4) replacement of one or more uracil (U) residues with A, G, or C in three or more consecutive U residues when the consecutive U residues are present in the tracrRNA-crRNA complementarity region; and
(5) addition of a U-rich tail to the 3'-end of its crRNA sequence (a sequence of the U-rich tail is represented by 5'-(UₘV)ₙUₒ-3', wherein V is each independently A, C, or G, m and o are integers between 1 to 20, and n is an integer between 0 to 5).

19. The composition of claim 18, wherein the wild-type Cas12f1 guide RNA comprises tracrRNA comprising the nucleotide sequence of SEQ ID NO: 11 and crRNA comprising the nucleotide sequence of SEQ ID NO: 12.

20. The composition of claim 18, wherein the engineered guide RNA consists of a sequence represented by Formula (I) or has at least 80% sequence identity thereto: 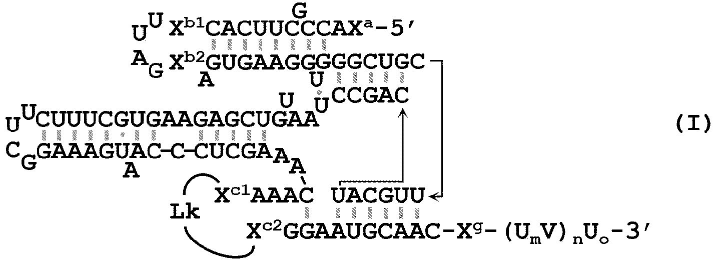
in Formula (I),
X^{a}, X^{b1}, X^{b2}, X^{c1}, and X^{c2} each independently consist of 0 to 35 (poly)nucleotides,
X^{g} is a first or second guide sequence,
Lk is a polynucleotide linker of 2 to 20 nucleotides or is absent, and
(UₘV)ₙUₒ is present as a U-rich tail or absent, and when (UₘV)ₙUₒ is present, U is uridine, V is each independently A, C, or G, m and o are each independently an integer between 1 and 20, and n is an integer between 0 and 5.

21. The composition of claim 20, wherein X^{a} comprises the nucleotide sequence of SEQ ID NO: 14 or a nucleotide sequence having the sequence of SEQ ID NO: 14 from which 1 to 20 nucleotides are deleted.

22. The composition of claim 20, wherein X^{b1} comprises the nucleotide sequence of SEQ ID NO: 25 or a nucleotide sequence having the sequence of SEQ ID NO: 25 from which 1 to 13 nucleotides are deleted.

23. The composition of claim 20, wherein X^{b2} comprises the nucleotide sequence of SEQ ID NO: 29 or a nucleotide sequence having the sequence of SEQ ID NO: 29 from which 1 to 14 nucleotides are deleted.

24. The composition of claim 20, wherein the sequence 5'-X^{b1}UUAGX^{b2}-3' in Formula (I) is a nucleotide sequence selected from the group consisting of SEQ ID NOS: 34 to 38.

25. The composition of claim 20, wherein X^{c1} comprises the nucleotide sequence of SEQ ID NO: 39 or a nucleotide sequence having the sequence of SEQ ID NO: 39 from which 1 to 28 nucleotides are deleted.

26. The composition of claim 25, wherein in a case where three or more consecutive uracil (U) residues are present in a sequence of X^{c1}, the sequence of X^{c1*} comprises a modification in which at least one U residue thereof is replaced with A, G, or C.

27. The composition of claim 20, wherein X^{c2} comprises the nucleotide sequence of SEQ ID NO: 58 or a nucleotide sequence having the sequence of SEQ ID NO: 58 from which 1 to 27 nucleotides are deleted.

28. The composition of claim 27, wherein in a case where the sequence 5'-ACGAA-3' is present in X^{c2}, the sequence is replaced with 5'-NGNNN-3', and N is each independently A, C, G, or U.

29. The composition of claim 20, wherein the sequence 5'-X^{c1}-Lk-X^{c2}-3' in Formula (I) is a nucleotide sequence selected from the group consisting of SEQ ID NOS: 80 to 86.

30. The composition of claim 20, wherein Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

31. The composition of claim 16, wherein the engineered guide RNA comprises an engineered tracrRNA having a nucleotide sequence selected from the group consisting of SEQ ID NOS: 87 to 132.

32. The composition of claim 16, wherein the engineered guide RNA comprises an engineered crRNA sequence having a nucleotide sequence selected from the group consisting of SEQ ID NOS: 133 to 148.

33. The composition of claim 16, wherein the engineered guide RNA is a dual guide RNA or a single guide RNA.

34. The composition of claim 33, wherein the engineered single guide RNA comprises a nucleotide sequence selected from the group consisting of SEQ ID NOS: 149 to 186.

35. The composition of claim 11, wherein the nucleic acid construct is contained in an adeno-associated virus vector.

36. The composition of claim 1, wherein the composition does not comprise a donor sequence required for homology-directed repair (HDR) pathway or a nucleic acid construct encoding the donor sequence.

37. A method for increasing deletion of a nucleic acid segment in a target gene of a cell, comprising bringing, into contact with the cell, the composition of any one of claims 1 to 36.

38. The method of claim 37, wherein the nucleic acid segment comprises a gene fragment that needs to be removed for gene correction.

39. The method of claim 38, wherein the nucleic acid segment comprises a gene fragment that needs to be removed for treatment of a genetic disease.

40. A kit or system for gene editing, comprising:
a composition for increasing deletion of a nucleic acid segment, comprising a molecule that inhibits expression of a gene involved in non-homologous end joining (NHEJ) pathway, or a nucleic acid construct encoding the molecule; and
a gene editing composition for deletion of a nucleic acid segment, comprising (i) a Cas endonuclease and a first guide RNA and a second guide RNA that hybridize to a first target sequence and a second target sequence, respectively, or (ii) at least one nucleic acid construct comprising a nucleic acid encoding a Cas endonuclease, a nucleic acid encoding a first guide RNA that hybridizes to a first target sequence, and a nucleic acid encoding a second guide RNA that hybridizes to a second target sequence,
wherein the nucleic acid segment exists between the first target sequence and the second target sequence, and
the Cas endonuclease forms a complex with the first guide RNA to induce first cleavage, and the Cas endonuclease forms a complex with the second guide RNA to induce second cleavage.
